# EUROPEAN PATENT SPECIFICATION

(11) **EP 3 059 315 B1**
(45) Date of publication and mention of the grant of the patent: **05.06.2019**
(21) Application number: 16156052.9
(22) Date of filing: 17.02.2016
(51) Int. Cl.: C12N 15/82, C12N 9/10, C07K 14/415, C12P 5/00, A01H 5/00

(54) **VECTOR COMPRISING SPECIFIC PROMOTER AND GENE ENCODING SPECIFIC PROTEIN, TRANSGENIC PLANT INTO WHICH THE VECTOR HAS BEEN INTRODUCED, AND METHOD FOR IMPROVING POLYISOPRENOID PRODUCTION BY INTRODUCING THE VECTOR INTO PLANT**
VEKTOR MIT SPEZIFISCHEM PROMOTOR UND GENCODIERENDEM SPEZIFISCHEN PROTEIN, TRANSGENE PFLANZE MIT DARIN EINGEFÜHRTEM VEKTOR UND VERFAHREN ZUR VERBESSERUNG DER POLYISOPRENOIDPRODUKTION DURCH EINFÜHRUNG DES VEKTORS IN DIE PFLANZE
VECTEUR COMPRENANT UN PROMOTEUR SPÉCIFIQUE ET GÈNE CODANT POUR UNE PROTÉINE SPÉCIFIQUE, PLANTE TRANSGÉNIQUE DANS LAQUELLE LE VECTEUR A ÉTÉ INTRODUIT ET PROCÉDÉ PERMETTANT D'AMÉLIORER LA PRODUCTION DE POLYISOPRÉNOÏDE PAR INTRODUCTION DU VECTEUR DANS UNE PLANTE

(30) Priority: 23.02.2015 JP 2015033073
(43) Date of publication of application: 24.08.2016
(73) Proprietor: Sumitomo Rubber Industries, Ltd., Kobe-shi, Hyogo-ken 651-0072 (JP)
(72) Inventor: INOUE, Yukino, Kobe-shi Hyogo 651-0072 (JP); OKADA, Akari, Kobe-shi Hyogo 651-0072 (JP); YAMAGUCHI, Haruhiko, Kobe-shi Hyogo 651-0072 (JP); KURODA, Satoshi, Kobe-shi Hyogo 651-0072 (JP); TAKAGI, Keiji, Kobe-shi Hyogo 651-0072 (JP)
(74) Representative: Henkel, Breuer & Partner

(56) References cited:
- WO-A1-2009/095059
- WO-A1-2013/051925
- WO-A1-2014/007285
- WO-A1-2014/054602
- DATABASE EMBL [Online] 2 July 2012 (2012-07-02), "Hevea brasiliensis MYC1 (MYC1) gene, promoter region and 5' UTR.", XP002758362, retrieved from EBI accession no. EM_STD:HM590649 Database accession no. HM590649
- YUE ZHAO ET AL: "genes with differential responses to tapping, mechanical wounding, ethrel and methyl jasmonate in laticifers of rubber tree (Muell. Arg.)", JOURNAL OF PLANT PHYSIOLOGY, URBAN UND FISCHER VERLAG, DE, vol. 168, no. 14, 23 February 2011 (2011-02-23), pages 1649-1658, XP028238977, ISSN: 0176-1617, DOI: 10.1016/J.JPLPH.2011.02.010 [retrieved on 2011-03-28]
- K.-S. CHOW ET AL: "Metabolic routes affecting rubber biosynthesis in Hevea brasiliensis latex", JOURNAL OF EXPERIMENTAL BOTANY, vol. 63, no. 5, 7 December 2011 (2011-12-07), pages 1863-1871, XP055267404, GB ISSN: 0022-0957, DOI: 10.1093/jxb/err363 & DATABASE EMBL [Online] 2 December 2011 (2011-12-02), "Y95B04 Hevea brasiliensis YLX600 library Hevea brasiliensis cDNA clone Y95B04 5', mRNA sequence.", retrieved from EBI accession no. EM_EST:JG013921 Database accession no. JG013921
- DONG GUO ET AL: "Molecular and functional characterization of the HbSRPP promoter in response to hormones and abiotic stresses", TRANSGENIC RESEARCH, vol. 23, no. 2, 1 April 2014 (2014-04-01), pages 331-340, XP055270006, GB ISSN: 0962-8819, DOI: 10.1007/s11248-013-9753-0

## Description

### TECHNICAL FIELD

The present invention relates to a vector comprising a specific promoter and a gene encoding a specific protein, a transgenic plant into which the vector has been introduced, and a method for improving polyisoprenoid production in a plant by introducing the vector into the plant.

### BACKGROUND ART

Nowadays natural rubber (one example of polyisoprenoids) for use in industrial rubber products can be harvested from isoprenoid-producing plants, such as para rubber tree (*Hevea brasiliensis*) belonging to the family *Euphorbiaceae,* or Indian rubber tree (*Ficus elastica*) belonging to the family *Moraceae.*

At present, para rubber tree is practically the only one source of natural rubber for industrial rubber products. Para rubber tree is a plant that can grow only in limited areas such as in Southeast Asia and South America. Moreover, para rubber tree requires about seven years from planting to mature enough for rubber extraction, and the period during which natural rubber can be extracted is limited to 20 to 30 years. Although more natural rubber is expected to be needed mainly by developing countries in years to come, for the reason mentioned above it is difficult to greatly increase the production of natural rubber using para rubber tree.

Meanwhile, along with the recent development in gene engineering, it is now possible to transform natural plants by introducing desired exogenous genes into the natural plants.

Since natural rubber is obtained from latex produced from the laticifers or latex ducts of specific isoprenoid-producing plants such as para rubber tree and Indian rubber tree, the development of gene recombination techniques has been considered for improving latex productivity to improve natural rubber production, but there is still room for improvement.

For this reason, depletion of natural rubber sources is of concern and there is a need to develop techniques that enables an improvement in natural rubber production.

EMBL AC HM590649 discloses the sequence of the MYC1 promoter.

Zhao et al. describe in J. Plant Physiol. 168, pages 1649 to 1658 that the expression rate of HBlMYC1 shall be up-regulated by tapping and mechanical bounding of the plant.

WO 2014/054602 A1 discloses a method of producing a polyisoprenoid using an isoprenoid-producing plant, wherein the method comprises regulating by a cytokinin-responsive transcription factor the expression of at least one protein being involved in the production of polyisoprenoid, such as small rubber particle protein. Similar methods are known from WO 2014/007285 A1 and WO 2013/051925 A1.

### SUMMARY OF INVENTION

### TECHNICAL PROBLEM

An object of the present invention is to solve the above-described problem and provide a vector that can improve polyisoprenoid production through the introduction of the vector into a plant using gene recombination techniques. Another object is to provide a transgenic plant into which the vector has been introduced and to provide a method for improving polyisoprenoid production in a plant by introducing the vector into the plant.

### SOLUTION TO PROBLEM

The inventors made various studies for improving natural rubber production and therefore focused on gene recombination techniques and conducted research and development to create a transgenic plant that is improved in natural rubber production by enhancing a part of the polyisoprenoid biosynthesis pathway to thereby improve polyisoprenoid production. As a result, they constructed a vector comprising a base sequence in which a gene encoding a protein involved in polyisoprenoid biosynthesis is linked so as to be controlled by a promoter of a gene encoding MYC1 transcription factor. Then, the inventors found that by introducing the constructed vector into a plant, the gene encoding a protein involved in polyisoprenoid biosynthesis in the vector can be expressed specifically in laticifers, thereby improving polyisoprenoid production in the plant.

The present invention relates to a vector, comprising: a MYC1 transcription factor encoding gene promoter; and a gene, the gene being functionally linked to the promoter, wherein the gene is at least one gene selected from the group consisting of a gene encoding farnesyl diphosphate synthase, a gene encoding geranylgeranyl diphosphate synthase, a gene encoding 3-hydroxy-3-methylglutaryl CoA reductase, a gene encoding isopentenyl diphosphate isomerase, a gene encoding cis-prenyltransferase, a gene encoding Small Rubber Particle Protein, and a gene encoding Rubber Elongation Factor, wherein the MYC1 transcription factor encoding gene promoter comprises any one of the following DNAs:
[A1] a DNA comprising the base sequence of base numbers 1 to 1610 represented by SEQ ID NO: 1;
[A2] a DNA hybridizing to a DNA comprising a base sequence complementary to the base sequence of base numbers 1 to 1610 represented by SEQ ID NO: 1 under stringent conditions, and having a promoter activity for laticifer-specific gene expression; and
[A3] a DNA comprising a base sequence having 80% or more sequence identity with the base sequence of base numbers 1 to 1610 represented by SEQ ID NO: 1, and having a promoter activity for laticifer-specific gene expression.

As set out above, the gene encoding a protein involved in polyisoprenoid biosynthesis is at least one gene selected from the group consisting of a gene encoding farnesyl diphosphate synthase, a gene encoding geranylgeranyl diphosphate synthase, a gene encoding 3-hydroxy-3-methylglutaryl CoA reductase, a gene encoding isopentenyl diphosphate isomerase, a gene encoding cis-prenyltransferase, a gene encoding Small Rubber Particle Protein, and a gene encoding Rubber Elongation Factor.

It is preferable that the gene encoding farnesyl diphosphate synthase comprises any one of the following DNAs :
[B1] a DNA comprising the base sequence of base numbers 1 to 1029 represented by SEQ ID NO: 2;
[B2] a DNA hybridizing to a DNA comprising a base sequence complementary to the base sequence of base numbers 1 to 1029 represented by SEQ ID NO: 2 under stringent conditions, and encoding a protein having an enzyme activity that catalyzes a reaction using isopentenyl diphosphate and dimethylallyl diphosphate as substrates or a reaction using isopentenyl diphosphate and geranyl diphosphate as substrates; and
[B3] a DNA comprising a base sequence having 80% or more sequence identity with the base sequence of base numbers 1 to 1029 represented by SEQ ID NO: 2, and encoding a protein having an enzyme activity that catalyzes a reaction using isopentenyl diphosphate and dimethylallyl diphosphate as substrates or a reaction using isopentenyl diphosphate and geranyl diphosphate as substrates.

It is preferable that the gene encoding geranylgeranyl diphosphate synthase comprises any one of the following DNAs:
[C1] a DNA comprising the base sequence of base numbers 1 to 1113 represented by SEQ ID NO: 4;
[C2] a DNA hybridizing to a DNA comprising a base sequence complementary to the base sequence of base numbers 1 to 1113 represented by SEQ ID NO: 4 under stringent conditions, and encoding a protein having an enzyme activity that catalyzes a reaction using isopentenyl diphosphate and dimethylallyl diphosphate as substrates, a reaction using isopentenyl diphosphate and geranyl diphosphate as substrates, or a reaction using isopentenyl diphosphate and farnesyl diphosphate as substrates; and
[C3] a DNA comprising a base sequence having 80% or more sequence identity with the base sequence of base numbers 1 to 1113 represented by SEQ ID NO: 4, and encoding a protein having an enzyme activity that catalyzes a reaction using isopentenyl diphosphate and dimethylallyl diphosphate as substrates, a reaction using isopentenyl diphosphate and geranyl diphosphate as substrates, or a reaction using isopentenyl diphosphate and farnesyl diphosphate as substrates.

It is preferable that the gene encoding 3-hydroxy-3-methylglutaryl CoA reductase comprises any one of the following DNAs:
[D1] a DNA comprising the base sequence of base numbers 1 to 1728 represented by SEQ ID NO: 6;
[D2] a DNA hybridizing to a DNA comprising a base sequence complementary to the base sequence of base numbers 1 to 1728 represented by SEQ ID NO: 6 under stringent conditions, and encoding a protein having an enzyme activity that catalyzes a reaction of reduction of 3-hydroxy-3-methylglutaryl CoA;
[D3] a DNA comprising a base sequence having 80% or more sequence identity with the base sequence of base numbers 1 to 1728 represented by SEQ ID NO: 6, and encoding a protein having an enzyme activity that catalyzes a reaction of reduction of 3-hydroxy-3-methylglutaryl CoA;
[D4] a DNA comprising the base sequence of base numbers 1 to 1761 represented by SEQ ID NO: 7;
[D5] a DNA hybridizing to a DNA comprising a base sequence complementary to the base sequence of base numbers 1 to 1761 represented by SEQ ID NO: 7 under stringent conditions, and encoding a protein having an enzyme activity that catalyzes a reaction of reduction of 3-hydroxy-3-methylglutaryl CoA;
[D6] a DNA comprising a base sequence having 80% or more sequence identity with the base sequence of base numbers 1 to 1761 represented by SEQ ID NO: 7, and encoding a protein having an enzyme activity that catalyzes a reaction of reduction of 3-hydroxy-3-methylglutaryl CoA;
[D7] a DNA comprising the base sequence of base numbers 1 to 1821 represented by SEQ ID NO: 8;
[D8] a DNA hybridizing to a DNA comprising a base sequence complementary to the base sequence of base numbers 1 to 1821 represented by SEQ ID NO: 8 under stringent conditions, and encoding a protein having an enzyme activity that catalyzes a reaction of reduction of 3-hydroxy-3-methylglutaryl CoA;
[D9] a DNA comprising a base sequence having 80% or more sequence identity with the base sequence of base numbers 1 to 1821 represented by SEQ ID NO: 8, and encoding a protein having an enzyme activity that catalyzes a reaction of reduction of 3-hydroxy-3-methylglutaryl CoA;
[D10] a DNA comprising the base sequence of base numbers 1 to 1581 represented by SEQ ID NO: 9;
[D11] a DNA hybridizing to a DNA comprising a base sequence complementary to the base sequence of base numbers 1 to 1581 represented by SEQ ID NO: 9 under stringent conditions, and encoding a protein having an enzyme activity that catalyzes a reaction of reduction of 3-hydroxy-3-methylglutaryl CoA; and [D12] a DNA comprising a base sequence having 80% or more sequence identity with the base sequence of base numbers 1 to 1581 represented by SEQ ID NO: 9, and encoding a protein having an enzyme activity that catalyzes a reaction of reduction of 3-hydroxy-3-methylglutaryl CoA.

It is preferable that the gene encoding isopentenyl diphosphate isomerase comprises any one of the following DNAs :
[E1] a DNA comprising the base sequence of base numbers 1 to 705 represented by SEQ ID NO: 14;
[E2] a DNA hybridizing to a DNA comprising a base sequence complementary to the base sequence of base numbers 1 to 705 represented by SEQ ID NO: 14 under stringent conditions, and encoding a protein having an enzyme activity that catalyzes a reaction of isomerization of isopentenyl diphosphate or dimethylallyl diphosphate; and
[E3] a DNA comprising a base sequence having 80% or more sequence identity with the base sequence of base numbers 1 to 705 represented by SEQ ID NO: 14, and encoding a protein having an enzyme activity that catalyzes a reaction of isomerization of isopentenyl diphosphate or dimethylallyl diphosphate.

It is preferable that the gene encoding cis-prenyltransferase comprises any one of the following DNAs:
[F1] a DNA comprising the base sequence of base numbers 1 to 873 represented by SEQ ID NO: 16;
[F2] a DNA hybridizing to a DNA comprising a base sequence complementary to the base sequence of base numbers 1 to 873 represented by SEQ ID NO: 16 under stringent conditions, and encoding a protein having an enzyme activity that catalyzes a reaction of cis-chain elongation of isoprenoid compounds;
[F3] a DNA comprising a base sequence having 80% or more sequence identity with the base sequence of base numbers 1 to 873 represented by SEQ ID NO: 16, and encoding a protein having an enzyme activity that catalyzes a reaction of cis-chain elongation of isoprenoid compounds;
[F4] a DNA comprising the base sequence of base numbers 1 to 855 represented by SEQ ID NO: 66;
[F5] a DNA hybridizing to a DNA comprising a base sequence complementary to the base sequence of base numbers 1 to 855 represented by SEQ ID NO: 66 under stringent conditions, and encoding a protein having an enzyme activity that catalyzes a reaction of cis-chain elongation of isoprenoid compounds; and
[F6] a DNA comprising a base sequence having 80% or more sequence identity with the base sequence of base numbers 1 to 855 represented by SEQ ID NO: 66, and encoding a protein having an enzyme activity that catalyzes a reaction of cis-chain elongation of isoprenoid compounds.

It is preferable that the gene encoding Small Rubber Particle Protein comprises any one of the following DNAs:
[G1] a DNA comprising the base sequence of base numbers 1 to 615 represented by SEQ ID NO: 18;
[G2] a DNA hybridizing to a DNA comprising a base sequence complementary to the base sequence of base numbers 1 to 615 represented by SEQ ID NO: 18 under stringent conditions, and encoding a rubber particle-associated protein which is associated with rubber particles in latex; and
[G3] a DNA comprising a base sequence having 80% or more sequence identity with the base sequence of base numbers 1 to 615 represented by SEQ ID NO: 18, and encoding a rubber particle-associated protein which is associated with rubber particles in latex.

It is preferable that the gene encoding Rubber Elongation Factor comprises any one of the following DNAs:
[H1] a DNA comprising the base sequence of base numbers 1 to 417 represented by SEQ ID NO: 60;
[H2] a DNA hybridizing to a DNA comprising a base sequence complementary to the base sequence of base numbers 1 to 417 represented by SEQ ID NO: 60 under stringent conditions, and encoding a rubber particle-associated protein which is associated with rubber particles in latex; and
[H3] a DNA comprising a base sequence having 80% or more sequence identity with the base sequence of base numbers 1 to 417 represented by SEQ ID NO: 60, and encoding a rubber particle-associated protein which is associated with rubber particles in latex.

The present invention also relates to a transgenic plant into which any of the vectors described above has been introduced.

The present invention also relates to a method for improving polyisoprenoid production in a plant by introducing any of the vectors described above into the plant.

### ADVANTAGEOUS EFFECTS OF INVENTION

The vector of the present invention comprises a base sequence in which a gene encoding a protein involved in polyisoprenoid biosynthesis is functionally linked to a promoter of a gene encoding MYC1 transcription factor. Further, by introducing such a vector into a plant, the gene encoding a protein involved in polyisoprenoid biosynthesis in the vector can be expressed specifically in laticifers, thereby improving polyisoprenoid production in the plant.

### BRIEF DESCRIPTION OF DRAWINGS

FIG. 1 is a schematic diagram showing a part of the polyisoprenoid biosynthesis pathway;
FIG. 2 is a schematic diagram showing a part of the polyisoprenoid biosynthesis pathway;
FIG. 3 is a schematic diagram showing a part of the mevalonic acid pathway which is upstream of a polyisoprenoid synthesis pathway;
FIG. 4 is a schematic diagram showing a part of the polyisoprenoid biosynthesis pathway;
FIG. 5 is a schematic diagram showing a part of the polyisoprenoid biosynthesis pathway;
FIG. 6 is a schematic diagram showing a part of the polyisoprenoid biosynthesis pathway;
FIG. 7 is a schematic diagram showing a T-DNA region of an expression vector introduced into Agrobacterium used in Example 1;
FIG. 8 is a schematic diagram showing a T-DNA region of an expression vector introduced into Agrobacterium used in Example 2;
FIG. 9 is a schematic diagram showing a T-DNA region of an expression vector introduced into Agrobacterium used in Example 3, 8, 9, or 10;
FIG. 10 is a schematic diagram showing a T-DNA region of an expression vector introduced into Agrobacterium used in Example 4;
FIG. 11 is a schematic diagram showing a T-DNA region of an expression vector introduced into Agrobacterium used in Example 5 or 11;
FIG. 12 is a schematic diagram showing a T-DNA region of an expression vector introduced into Agrobacterium used in Example 6; and
FIG. 13 is a schematic diagram showing a T-DNA region of an expression vector introduced into Agrobacterium used in Example 7.

### DESCRIPTION OF EMBODIMENTS

### (Vector)

The vector of the present invention comprises a base sequence in which a gene encoding a protein involved in polyisoprenoid biosynthesis is functionally linked to a promoter of a gene encoding MYC1 transcription factor (MYC1) having a promoter activity for laticifer-specific gene expression. By introducing such a vector into a plant for transformation, the gene encoding a protein involved in polyisoprenoid biosynthesis in the vector can be expressed specifically in laticifers, thereby improving polyisoprenoid production in the plant. This is presumably because if the expression of an exogenous gene introduced for improvement of latex productivity is promoted in sites other than laticifers, a certain load is imposed on the metabolism or the production of latex of the plant, thereby causing adverse effects.

In the present specification, the term "MYC1 transcription factor (MYC1)" refers to a transcription factor that is highly expressed in the latex of polyisoprenoid-producing plants such as para rubber tree (*Hevea brasiliensis*) and is involved in jasmonic acid signaling. The term "transcription factor" means a protein that has activity for increasing or decreasing, and preferably increasing, gene transcription. In other words, in the present specification, the MYC1 transcription factor is a protein that has activity (transcription factor activity) for increasing or decreasing, and preferably increasing, the transcription of a gene encoding at least one protein among the proteins involved in jasmonic acid signaling.

In the present specification, the "promoter having a promoter activity for laticifer-specific gene expression" means that the promoter has an activity for controlling gene expression to cause the laticifer-specific expression of a desired gene when the desired gene is functionally linked to the promoter and introduced into a plant. The terms "laticifer-specific gene expression" means that the gene is expressed substantially exclusively in laticifers with no or little expression in sites other than laticifers in the plant. Also, "a gene is functionally linked to a promoter" means that the gene sequence is linked downstream of the promoter so that the gene is controlled by the promoter.

The vector of the present invention can be prepared by inserting the base sequence of a promoter of a gene encoding MYC1 and the base sequence of a gene encoding a protein involved in polyisoprenoid biosynthesis into a vector generally known as a plant transformation vector by conventional techniques. Examples of vectors usable for preparing the vector of the present invention include pBI vectors, binary vectors such as pGA482, pGAH, and pBIG, intermediate plasmids such as pLGV23Neo, pNCAT, and pMON200, pH35GS containing GATEWAY cassette, and the like.

The vector of the present invention may contain other base sequences as long as the vector comprises the base sequence of a promoter of a gene encoding MYC1 and the base sequence of a gene encoding a protein involved in polyisoprenoid biosynthesis. Usually, the vector contains sequences derived from the original vector in addition to these base sequences and further contains a restriction enzyme recognition sequence, a spacer sequence, a sequence of a marker gene, a sequence of a reporter gene, and so forth.

Examples of the marker gene include drug-resistant genes such as a kanamycin-resistant gene, hygromycin-resistant gene, and a bleomycin-resistant gene. The reporter gene is introduced to determine the expression site in a plant, and examples include a luciferase gene, a GUS (β-glucuronidase) gene, GFP (green fluorescent protein), RFP (red fluorescent protein), and so forth.

### (MYC1 Encoding Gene Promoter)

The encoding gene promoter, which is also referred to as promoter of the gene encoding MYC1, may preferably be derived from a plant, more preferably from a polyisoprenoid-producing plant, without particular limitation thereto. Among them, the promoter may further preferably be derived from para rubber tree, guayule, Russian dandelion, Canada goldenrod, common sowthistle, lettuce, or sunflower, particularly preferably from para rubber tree.

In the present specification, the polyisoprenoid-producing plant means a plant capable of producing a polyisoprenoid, and specific examples will be described later.

The promoter of the gene encoding MYC1 is according to the present invention
any one of the following DNAs:
[A1] a DNA comprising the base sequence of base numbers 1 to 1610 represented by SEQ ID NO: 1;
[A2] a DNA hybridizing to a DNA comprising a base sequence complementary to the base sequence of base numbers 1 to 1610 represented by SEQ ID NO: 1 under stringent conditions, and having a promoter activity for laticifer-specific gene expression; and
[A3] a DNA comprising a base sequence having 80% or more sequence identity with the base sequence of base numbers 1 to 1610 represented by SEQ ID NO: 1, and having a promoter activity for laticifer-specific gene expression.

As used herein, the term "hybridizing" means a process in which a DNA hybridizes to a DNA having a specific base sequence or a part of the DNA. Accordingly, the DNA having a specific base sequence or part of the DNA may have a base sequence long enough to be usable as a probe in Northern or Southern blot analysis or as an oligonucleotide primer in polymerase chain reaction (PCR) analysis. The DNA used as a probe may have a length of at least 100 bases, preferably at least 200 bases, and more preferably at least 500 bases although it may be a DNA of at least 10 bases, and preferably of at least 15 bases in length.

Techniques to perform DNA hybridization experiments are well known. The hybridization conditions under which experiments are performed may be determined according to, for example, Molecular Cloning, 2nd ed. and 3rd ed. (2001), Methods for General and Molecular Bacteriology, ASM Press (1994), Immunology methods manual, Academic press (Molecular), and many other standard textbooks.

The stringent conditions may include, for example, an overnight incubation at 42°C of a DNA-immobilized filter and a DNA probe in a solution containing 50% formamide, 5× SSC (750 mM sodium chloride, 75 mM sodium citrate), 50 mM sodium phosphate (pH 7.6), 5× Denhardt's solution, 10% dextran sulfate, and 20 µg/l denatured salmon sperm DNA, followed by washing the filter for example in a 0.2× SSC solution at approximately 65°C. Less stringent conditions may also be used. Changes in the stringency may be accomplished through the manipulation of formamide concentration (lower percentages of formamide result in lower stringency), salt concentrations or temperature. For example, low stringent conditions include an overnight incubation at 37°C in a solution containing 6× SSCE (20× SSCE: 3 mol/l sodium chloride, 0.2 mol/l sodium dihydrogen phosphate, 0.02 mol/l EDTA, pH 7.4), 0.5% SDS, 30% formamide, and 100 µg/l denatured salmon sperm DNA, followed by washing in a 1× SSC solution containing 0.1% SDS at 50°C. In addition, to achieve even lower stringency, washes performed following hybridization may be done at higher salt concentrations (e.g. 5× SSC) in the above-mentioned low stringent conditions.

Variations in the above various conditions may be accomplished through the inclusion or substitution of blocking reagents used to suppress background in hybridization experiments. The inclusion of blocking reagents may require modification of the hybridization conditions for compatibility.

Like the DNA capable of hybridization under stringent conditions described above, it is known that some promoters with base sequences having a certain sequence identity with the original base sequence have similar promoter activity. In order to maintain the promoter activity, the sequence identity with the base sequence of base numbers 1 to 1610 represented by SEQ ID NO: 1 is at least 80% or more, preferably 90% or more, more preferably 95% or more, further preferably 98% or more, particularly preferably 99% or more.

The sequence identity between base sequences or amino acid sequences may be determined using the algorithm BLAST [Pro. Natl. Acad. Sci. USA, 90, 5873 (1993)] developed by Karlin and Altschul or FASTA [Methods Enzymol., 183, 63 (1990)].

Whether a DNA hybridizing to the above-described DNA under stringent conditions or a DNA having 80% or more sequence identity with the above-described DNA has a promoter activity for laticifer-specific gene expression may be determined by conventional techniques, such as reporter assays using a reporter gene encoding β-galactosidase, luciferase, green fluorescent protein (GFP), or the like.

Conventional techniques may be employed to identify the base sequence of the promoter of the gene encoding MYC1, and, for example, a genomic DNA may be extracted from a growing plant by the CTAB (Cetyl Trimethyl Ammonium Bromide) method. Next, specific primers and random primers are designed based on the known base sequence of the gene encoding MYC1, and the gene including the MYC1 promoter is amplified by TAIL (Thermal Asymmetric Interlaced) -PCR using the extracted genomic DNA as a template to identify the base sequence.

### (Protein Involved in Polyisoprenoid Biosynthesis)

The gene encoding a protein involved in polyisoprenoid biosynthesis is at least one gene selected from the group consisting of a gene encoding farnesyl diphosphate synthase, a gene encoding geranylgeranyl diphosphate synthase, a gene encoding 3-hydroxy-3-methylglutaryl CoA reductase, a gene encoding isopentenyl diphosphate isomerase, a gene encoding cis-prenyltransferase, a gene encoding Small Rubber Particle Protein, and a gene encoding Rubber Elongation Factor. Among them, for further improved polyisoprenoid production, it may more preferably be at least one gene selected from the group consisting of a gene encoding 3-hydroxy-3-methylglutaryl CoA reductase, a gene encoding cis-prenyltransferase, a gene encoding Small Rubber Particle Protein, and a gene encoding Rubber Elongation Factor, further preferably a gene encoding cis-prenyltransferase, a gene encoding Small Rubber Particle Protein, or a gene encoding Rubber Elongation Factor, particularly preferably a gene encoding cis-prenyltransferase.

The inventors aimed at creation of a transgenic plant that is improved in natural rubber production by enhancing a part of the polyisoprenoid biosynthesis pathway to thereby improve polyisoprenoid production. First of all, the two pathways, mevalonic acid pathway (MVA pathway) and non-mevalonic acid pathway (MEP pathway) are known as pathways for biosynthesis of isoprenyl diphosphate (IPP), which is an important member of the polyisoprenoid biosynthesis pathway. The inventors focused on the MVA pathway and selected, from various proteins involved in the polyisoprenoid biosynthesis pathway, some proteins that are expected to have important roles to enhance the MVA pathway or a downstream part of the pathway.

Specifically, the following seven proteins were selected: farnesyl diphosphate synthase (FPS) and geranylgeranyl diphosphate synthase (GGPS), which are prenyltransferases involved in reactions in which IPP is linked to allylic substrates so that isoprene units are sequentially connected, and synthesize farnesyl diphosphate (FPP) and geranylgeranyl diphosphate (GGPP), respectively, which are considered as starting substrates for natural rubber; 3-hydroxy-3-methylglutaryl CoA reductase (HMGR) which is a rate-limiting factor in the MVA pathway; isopentenyl diphosphate isomerase (IPI) which is involved in isomerization of IPP and dimethylallyl diphosphate (DMAPP); cis-prenyltransferase (CPT) which is thought to be involved in chain elongation of isoprenoid compounds; Small Rubber Particle Protein (SRPP) and Rubber Elongation Factor (REF), which are known to be involved in polyisoprenoid biosynthesis. Then, vectors were constructed, each of which comprises a base sequence in which a gene encoding each of these proteins is linked so as to be under the control of a promoter of a gene encoding MYC1 transcription factor. The constructed vectors could each be introduced into a plant to improve polyisoprenoid production in the plant.

As mentioned above, the reactions in which isopentenyl diphosphate (IPP) is sequentially linked to allylic substrates proceed in the polyisoprenoid biosynthesis pathway. The enzymes catalyzing the reactions are collectively referred to as prenyltransferase in the sense that isoprene units are sequentially connected. In the present specification, the term "prenyltransferase" means a collective term of enzymes that each catalyze a condensation reaction between IPP and an isoprenyl diphosphate (the number of isoprene units is n) (allylic substrate) to synthesize a new isoprenyl diphosphate (the number of isoprene units is n + 1) in which one isoprene unit is added.

The prenyltransferases are a group of enzymes that link isoprene units to synthesize various isoprenyl diphosphates such as geranyl diphosphate (GPP: C10), farnesyl diphosphate (FPP: C15), geranylgeranyl diphosphate (GGPP: C20), geranylfarnesyl diphosphate (GFPP: C25), hexaprenyl diphosphate (HPP: C30), or the like serving as basic precursors of terpenoids, and are positioned in the mainstream of terpenoid biosynthesis. Farnesyl diphosphate synthase (FPS), geranylgeranyl diphosphate synthase (GGPS), and so forth are classified as prenyltransferases.

In the present specification, the term "farnesyl diphosphate synthase (FPS)" refers to an enzyme that catalyzes a farnesyl diphosphate (FPP) biosynthesis reaction using isopentenyl diphosphate (IPP), dimethylallyl diphosphate (DMAPP), and geranyl diphosphate (GPP) as substrates.

Also, in the present specification, the term "geranylgeranyl diphosphate synthase (GGPS)" refers to an enzyme that catalyzes a geranylgeranyl diphosphate (GGPP) biosynthesis reaction using isopentenyl diphosphate (IPP), dimethylallyl diphosphate (DMAPP), geranyl diphosphate (GPP), and farnesyl diphosphate (FPP) as substrates.

Also, in the present specification, the term "3-hydroxy-3-methylglutaryl CoA reductase (HMG-CoA reductase, HMGR)" is one of rate-limiting enzymes in the mevalonic acid pathway (MVA pathway) and includes both 3-hydroxy-3-methylglutaryl CoA reductase (NADPH) (EC 1.1.1.34) and 3-hydroxy-3-methylglutaryl CoA reductase (EC 1.1.1.88).

Also, in the present specification, the term "isopentenyl diphosphate isomerase (IPP isomerase, IPI)" refers to an enzyme that catalyzes an isomerization reaction between isopentenyl diphosphate (IPP) and its isomer, dimethylallyl diphosphate (DMAPP).

Also, in the present specification, the term "cis-prenyltransferase (cis-type prenyltransferase, CPT)" refers to an enzyme that catalyzes a reaction of cis-chain elongation of isoprenoid compounds. As used herein, the term "isoprenoid compound" means a compound containing an isoprene unit (C₅H₈). Also, the term "cis isoprenoid" refers to a compound including an isoprenoid compound in which isoprene units are cis-bonded and examples include cis-farnesyl diphosphate, undecaprenyl diphosphate, natural rubber, and the like.

Also, in the present specification, the term "Small Rubber Particle Protein (SRPP)" refers to a rubber particle-associated protein which is associated with rubber particles in the latex of a polyisoprenoid-producing plant such as para rubber tree (*Hevea brasiliensis*).

Also, in the present specification, the term "Rubber Elongation Factor (REF) " refers to a rubber particle-associated protein which is associated with rubber particles in the latex of a polyisoprenoid-producing plant such as para rubber tree (*Hevea brasiliensis*).

### (Gene)

The gene encoding farnesyl diphosphate synthase, gene encoding geranylgeranyl diphosphate synthase, gene encoding 3-hydroxy-3-methylglutaryl CoA reductase, gene encoding isopentenyl diphosphate isomerase, gene encoding cis-prenyltransferase, gene encoding Small Rubber Particle Protein, and gene encoding Rubber Elongation Factor may each preferably be derived from a plant, more preferably from a polyisoprenoid-producing plant, without particular limitation thereto. Among others, the genes may each further preferably be derived from para rubber tree, guayule, Russian dandelion, Canada goldenrod, common sowthistle, lettuce, or sunflower, particularly preferably from para rubber tree.

The gene encoding farnesyl diphosphate synthase may preferably comprise any one of the following DNAs:
[B1] a DNA comprising the base sequence of base numbers 1 to 1029 represented by SEQ ID NO: 2;
[B2] a DNA hybridizing to a DNA comprising a base sequence complementary to the base sequence of base numbers 1 to 1029 represented by SEQ ID NO: 2 under stringent conditions, and encoding a protein having an enzyme activity that catalyzes a reaction using isopentenyl diphosphate and dimethylallyl diphosphate as substrates or a reaction using isopentenyl diphosphate and geranyl diphosphate as substrates; and
[B3] a DNA comprising a base sequence having 80% or more sequence identity with the base sequence of base numbers 1 to 1029 represented by SEQ ID NO: 2, and encoding a protein having an enzyme activity that catalyzes a reaction using isopentenyl diphosphate and dimethylallyl diphosphate as substrates or a reaction using isopentenyl diphosphate and geranyl diphosphate as substrates.

The gene encoding geranylgeranyl diphosphate synthase may preferably comprise any one of the following DNAs:
[C1] a DNA comprising the base sequence of base numbers 1 to 1113 represented by SEQ ID NO: 4;
[C2] a DNA hybridizing to a DNA comprising a base sequence complementary to the base sequence of base numbers 1 to 1113 represented by SEQ ID NO: 4 under stringent conditions, and encoding a protein having an enzyme activity that catalyzes a reaction using isopentenyl diphosphate and dimethylallyl diphosphate as substrates, a reaction using isopentenyl diphosphate and geranyl diphosphate as substrates, or a reaction using isopentenyl diphosphate and farnesyl diphosphate as substrates; and
[C3] a DNA comprising a base sequence having 80% or more sequence identity with the base sequence of base numbers 1 to 1113 represented by SEQ ID NO: 4, and encoding a protein having an enzyme activity that catalyzes a reaction using isopentenyl diphosphate and dimethylallyl diphosphate as substrates, a reaction using isopentenyl diphosphate and geranyl diphosphate as substrates, or a reaction using isopentenyl diphosphate and farnesyl diphosphate as substrates.

The gene encoding 3-hydroxy-3-methylglutaryl CoA reductase may preferably comprise any one of the following DNAs :
[D1] a DNA comprising the base sequence of base numbers 1 to 1728 represented by SEQ ID NO: 6;
[D2] a DNA hybridizing to a DNA comprising a base sequence complementary to the base sequence of base numbers 1 to 1728 represented by SEQ ID NO: 6 under stringent conditions, and encoding a protein having an enzyme activity that catalyzes a reaction of reduction of 3-hydroxy-3-methylglutaryl CoA;
[D3] a DNA comprising a base sequence having 80% or more sequence identity with the base sequence of base numbers 1 to 1728 represented by SEQ ID NO: 6, and encoding a protein having an enzyme activity that catalyzes a reaction of reduction of 3-hydroxy-3-methylglutaryl CoA;
[D4] a DNA comprising the base sequence of base numbers 1 to 1761 represented by SEQ ID NO: 7;
[D5] a DNA hybridizing to a DNA comprising a base sequence complementary to the base sequence of base numbers 1 to 1761 represented by SEQ ID NO: 7 under stringent conditions, and encoding a protein having an enzyme activity that catalyzes a reaction of reduction of 3-hydroxy-3-methylglutaryl CoA;
[D6] a DNA comprising a base sequence having 80% or more sequence identity with the base sequence of base numbers 1 to 1761 represented by SEQ ID NO: 7, and encoding a protein having an enzyme activity that catalyzes a reaction of reduction of 3-hydroxy-3-methylglutaryl CoA;
[D7] a DNA comprising the base sequence of base numbers 1 to 1821 represented by SEQ ID NO: 8;
[D8] a DNA hybridizing to a DNA comprising a base sequence complementary to the base sequence of base numbers 1 to 1821 represented by SEQ ID NO: 8 under stringent conditions, and encoding a protein having an enzyme activity that catalyzes a reaction of reduction of 3-hydroxy-3-methylglutaryl CoA;
[D9] a DNA comprising a base sequence having 80% or more sequence identity with the base sequence of base numbers 1 to 1821 represented by SEQ ID NO: 8, and encoding a protein having an enzyme activity that catalyzes a reaction of reduction of 3-hydroxy-3-methylglutaryl CoA;
[D10] a DNA comprising the base sequence of base numbers 1 to 1581 represented by SEQ ID NO: 9;
[D11] a DNA hybridizing to a DNA comprising a base sequence complementary to the base sequence of base numbers 1 to 1581 represented by SEQ ID NO: 9 under stringent conditions, and encoding a protein having an enzyme activity that catalyzes a reaction of reduction of 3-hydroxy-3-methylglutaryl CoA; and
[D12] a DNA comprising a base sequence having 80% or more sequence identity with the base sequence of base numbers 1 to 1581 represented by SEQ ID NO: 9, and encoding a protein having an enzyme activity that catalyzes a reaction of reduction of 3-hydroxy-3-methylglutaryl CoA.

The gene encoding isopentenyl diphosphate isomerase may preferably comprise any one of the following DNAs:
[E1] a DNA comprising the base sequence of base numbers 1 to 705 represented by SEQ ID NO: 14;
[E2] a DNA hybridizing to a DNA comprising a base sequence complementary to the base sequence of base numbers 1 to 705 represented by SEQ ID NO: 14 under stringent conditions, and encoding a protein having an enzyme activity that catalyzes a reaction of isomerization of isopentenyl diphosphate or dimethylallyl diphosphate; and
[E3] a DNA comprising a base sequence having 80% or more sequence identity with the base sequence of base numbers 1 to 705 represented by SEQ ID NO: 14, and encoding a protein having an enzyme activity that catalyzes a reaction of isomerization of isopentenyl diphosphate or dimethylallyl diphosphate.

The gene encoding cis-prenyltransferase may preferably comprise any one of the following DNAs:
[F1] a DNA comprising the base sequence of base numbers 1 to 873 represented by SEQ ID NO: 16;
[F2] a DNA hybridizing to a DNA comprising a base sequence complementary to the base sequence of base numbers 1 to 873 represented by SEQ ID NO: 16 under stringent conditions, and encoding a protein having an enzyme activity that catalyzes a reaction of cis-chain elongation of isoprenoid compounds;
[F3] a DNA comprising a base sequence having 80% or more sequence identity with the base sequence of base numbers 1 to 873 represented by SEQ ID NO: 16, and encoding a protein having an enzyme activity that catalyzes a reaction of cis-chain elongation of isoprenoid compounds;
[F4] a DNA comprising the base sequence of base numbers 1 to 855 represented by SEQ ID NO: 66;
[F5] a DNA hybridizing to a DNA comprising a base sequence complementary to the base sequence of base numbers 1 to 855 represented by SEQ ID NO: 66 under stringent conditions, and encoding a protein having an enzyme activity that catalyzes a reaction of cis-chain elongation of isoprenoid compounds; and [F6] a DNA comprising a base sequence having 80% or more sequence identity with the base sequence of base numbers 1 to 855 represented by SEQ ID NO: 66, and encoding a protein having an enzyme activity that catalyzes a reaction of cis-chain elongation of isoprenoid compounds.

The gene encoding Small Rubber Particle Protein may preferably comprise any one of the following DNAs:
[G1] a DNA comprising the base sequence of base numbers 1 to 615 represented by SEQ ID NO: 18;
[G2] a DNA hybridizing to a DNA comprising a base sequence complementary to the base sequence of base numbers 1 to 615 represented by SEQ ID NO: 18 under stringent conditions, and encoding a rubber particle-associated protein which is associated with rubber particles in latex; and
[G3] a DNA comprising a base sequence having 80% or more sequence identity with the base sequence of base numbers 1 to 615 represented by SEQ ID NO: 18, and encoding a rubber particle-associated protein which is associated with rubber particles in latex.

The gene encoding Rubber Elongation Factor may preferably comprise any one of the following DNAs:
[H1] a DNA comprising the base sequence of base numbers 1 to 417 represented by SEQ ID NO: 60;
[H2] a DNA hybridizing to a DNA comprising a base sequence complementary to the base sequence of base numbers 1 to 417 represented by SEQ ID NO: 60 under stringent conditions, and encoding a rubber particle-associated protein which is associated with rubber particles in latex; and
[H3] a DNA comprising a base sequence having 80% or more sequence identity with the base sequence of base numbers 1 to 417 represented by SEQ ID NO: 60, and encoding a rubber particle-associated protein which is associated with rubber particles in latex.

As used herein, the term "hybridizing" is as described above. Also, the stringent conditions are as described above.

Moreover, like the DNA capable of hybridization under stringent conditions described above, it is known that some protein-encoding genes with base sequences having a certain sequence identity with the original base sequence have similar enzyme activity. In order to maintain the enzyme activity, the sequence identity with the base sequence of base numbers 1 to 1029 represented by SEQ ID NO: 2 is at least 80% or more, preferably 90% or more, more preferably 95% or more, further preferably 98% or more, particularly preferably 99% or more. The same range of the sequence identity as described above applies to the base sequences represented by SEQ ID NOs: 4, 6 to 9, 14, 16, 18, 60, and 66.

Whether a DNA hybridizing to the above-described DNA under stringent conditions or a DNA having 80% or more sequence identity with the above-described DNA encodes a protein having a predetermined function such as enzyme activity may be determined by conventional techniques, such as by expressing a target protein in a transformant prepared by introducing a gene encoding the target protein into *Escherichia coli* or the like, and determining the presence or absence of the function of the target protein by the corresponding activity measurement method.

Also, conventional techniques may be employed to identify the base sequence of a gene encoding the protein or the amino acid sequence of the protein. For example, the whole length base sequence or amino acid sequence is identified by extracting total RNA from a growing plant, optionally purifying the mRNA, and synthesizing a cDNA by a reverse transcription reaction; then designing degenerate primers based on the amino acid sequence of a known protein corresponding to the target protein, partially amplifying a DNA fragment by RT-PCR, and partially identifying the sequence; and then performing the RACE method or the like. The RACE method (Rapid Amplification of cDNA Ends method) refers to a method in which, when the base sequence of a cDNA is partially known, PCR is performed based on the base sequence information of such a known region to clone an unknown region extending to the cDNA terminal, and is capable of cloning the whole length cDNA by PCR without preparing a cDNA library.

The degenerate primer may preferably be prepared from a plant-derived sequence having a highly similar sequence part to the target protein.

In the case where the base sequence encoding the protein is known, it is possible to identify the whole length base sequence or amino acid sequence by designing a primer including an initiation codon and a primer including a termination codon using the known base sequence and then performing RT-PCR using a synthesized cDNA as a template.

### (Protein)

A specific example of the farnesyl diphosphate synthase may be [b1] described below. The protein designated by [b1] is a protein encoded by the above-described DNA designated by [B1]:
[b1] a protein comprising the amino acid sequence of amino acid numbers 1 to 342 represented by SEQ ID NO: 3.

A specific example of the geranylgeranyl diphosphate synthase may be [c1] described below. The protein designated by [c1] is a protein encoded by the above-described DNA designated by [C1]:
[c1] a protein comprising the amino acid sequence of amino acid numbers 1 to 370 represented by SEQ ID NO: 5.

A specific example of the 3-hydroxy-3-methylglutaryl CoA reductase may be any one of [d1] to [d4] described below. The proteins designated by [d1] to [d4] are proteins encoded by the above-described DNAs designated by [D1], [D4], [D7], and [D10], respectively:
[d1] a protein comprising the amino acid sequence of amino acid numbers 1 to 575 represented by SEQ ID NO: 10;
[d2] a protein comprising the amino acid sequence of amino acid numbers 1 to 586 represented by SEQ ID NO: 11;
[d3] a protein comprising the amino acid sequence of amino acid numbers 1 to 606 represented by SEQ ID NO: 12; and
[d4] a protein comprising the amino acid sequence of amino acid numbers 1 to 526 represented by SEQ ID NO: 13.

A specific example of the isopentenyl diphosphate isomerase may be [e1] described below. The protein designated by [e1] is a protein encoded by the above-described DNA designated by [E1]:
[e1] a protein comprising the amino acid sequence of amino acid numbers 1 to 234 represented by SEQ ID NO: 15.

A specific example of the cis-prenyltransferase may be [f1] or [f4] described below. The proteins designated by [f1] and [f4] are proteins encoded by the above-described DNAs designated by [F1] and [F4], respectively:
[f1] a protein comprising the amino acid sequence of amino acid numbers 1 to 290 represented by SEQ ID NO: 17; and
[f4] a protein comprising the amino acid sequence of amino acid numbers 1 to 284 represented by SEQ ID NO: 67.

A specific example of the Small Rubber Particle Protein may be [g1] described below. The protein designated by [g1] is a protein encoded by the above-described DNA designated by [G1]:
[g1] a protein comprising the amino acid sequence of amino acid numbers 1 to 204 represented by SEQ ID NO: 19.

A specific example of the Rubber Elongation Factor may be [h1] described below. The protein designated by [h1] is a protein encoded by the above-described DNA designated by [H1] :
[h1] a protein comprising the amino acid sequence of amino acid numbers 1 to 138 represented by SEQ ID NO: 61.

It is known that some proteins having one or more amino acid substitutions, deletions, insertions, or additions relative to the original amino acid sequence have the inherent function. Thus, specific examples of the above-described proteins also include the following [b2], [c2], [d5], [d6], [d7], [d8], [e2], [f2], [f5], [g2], and [h2]:
[b2] a protein comprising an amino acid sequence containing one or more amino acid substitutions, deletions, insertions, and/or additions relative to the amino acid sequence of amino acid numbers 1 to 342 represented by SEQ ID NO: 3, and having an enzyme activity that catalyzes a reaction using isopentenyl diphosphate and dimethylallyl diphosphate as substrates or a reaction using isopentenyl diphosphate and geranyl diphosphate as substrates;
[c2] a protein comprising an amino acid sequence containing one or more amino acid substitutions, deletions, insertions, and/or additions relative to the amino acid sequence of amino acid numbers 1 to 370 represented by SEQ ID NO: 5, and having an enzyme activity that catalyzes a reaction using isopentenyl diphosphate and dimethylallyl diphosphate as substrates, a reaction using isopentenyl diphosphate and geranyl diphosphate as substrates, or a reaction using isopentenyl diphosphate and farnesyl diphosphate as substrates;
[d5] a protein comprising an amino acid sequence containing one or more amino acid substitutions, deletions, insertions, and/or additions relative to the amino acid sequence of amino acid numbers 1 to 575 represented by SEQ ID NO: 10, and having an enzyme activity that catalyzes a reaction of reduction of 3-hydroxy-3-methylglutaryl CoA;
[d6] a protein comprising an amino acid sequence containing one or more amino acid substitutions, deletions, insertions, and/or additions relative to the amino acid sequence of amino acid numbers 1 to 586 represented by SEQ ID NO: 11, and having an enzyme activity that catalyzes a reaction of reduction of 3-hydroxy-3-methylglutaryl CoA;
[d7] a protein comprising an amino acid sequence containing one or more amino acid substitutions, deletions, insertions, and/or additions relative to the amino acid sequence of amino acid numbers 1 to 606 represented by SEQ ID NO: 12, and having an enzyme activity that catalyzes a reaction of reduction of 3-hydroxy-3-methylglutaryl CoA;
[d8] a protein comprising an amino acid sequence containing one or more amino acid substitutions, deletions, insertions, and/or additions relative to the amino acid sequence of amino acid numbers 1 to 526 represented by SEQ ID NO: 13, and having an enzyme activity that catalyzes a reaction of reduction of 3-hydroxy-3-methylglutaryl CoA;
[e2] a protein comprising an amino acid sequence containing one or more amino acid substitutions, deletions, insertions, and/or additions relative to the amino acid sequence of amino acid numbers 1 to 234 represented by SEQ ID NO: 15, and having an enzyme activity that catalyzes a reaction of isomerization of isopentenyl diphosphate or dimethylallyl diphosphate;
[f2] a protein comprising an amino acid sequence containing one or more amino acid substitutions, deletions, insertions, and/or additions relative to the amino acid sequence of amino acid numbers 1 to 290 represented by SEQ ID NO: 17, and having an enzyme activity that catalyzes a reaction of cis-chain elongation of isoprenoid compounds;
[f5] a protein comprising an amino acid sequence containing one or more amino acid substitutions, deletions, insertions, and/or additions relative to the amino acid sequence of amino acid numbers 1 to 284 represented by SEQ ID NO: 67, and having an enzyme activity that catalyzes a reaction of cis-chain elongation of isoprenoid compounds;
[g2] a rubber particle-associated protein comprising an amino acid sequence containing one or more amino acid substitutions, deletions, insertions, and/or additions relative to the amino acid sequence of amino acid numbers 1 to 204 represented by SEQ ID NO: 19, and being associated with rubber particles in latex; and
[h2] a rubber particle-associated protein comprising an amino acid sequence containing one or more amino acid substitutions, deletions, insertions, and/or additions relative to the amino acid sequence of amino acid numbers 1 to 138 represented by SEQ ID NO: 61, and being associated with rubber particles in latex.

In order to maintain the enzyme activity, preferred is an amino acid sequence containing one or more, more preferably 1 to 68, further preferably 1 to 51, still further preferably 1 to 34, particularly preferably 1 to 17, most preferably 1 to 7, yet most preferably 1 to 3 amino acid substitutions, deletions, insertions, and/or additions relative to the amino acid sequence represented by SEQ ID NO: 3.

Also, in order to maintain the enzyme activity, preferred is an amino acid sequence containing one or more, more preferably 1 to 74, further preferably 1 to 56, still further preferably 1 to 37, particularly preferably 1 to 19, most preferably 1 to 7, yet most preferably 1 to 4 amino acid substitutions, deletions, insertions, and/or additions relative to the amino acid sequence represented by SEQ ID NO: 5.

Also, in order to maintain the enzyme activity, preferred is an amino acid sequence containing one or more, more preferably 1 to 115, further preferably 1 to 86, still further preferably 1 to 58, particularly preferably 1 to 29, most preferably 1 to 12, yet most preferably 1 to 6 amino acid substitutions, deletions, insertions, and/or additions relative to the amino acid sequence represented by SEQ ID NO: 10.

Also, in order to maintain the enzyme activity, preferred is an amino acid sequence containing one or more, more preferably 1 to 117, further preferably 1 to 88, still further preferably 1 to 59, particularly preferably 1 to 29, most preferably 1 to 12, yet most preferably 1 to 6 amino acid substitutions, deletions, insertions, and/or additions relative to the amino acid sequence represented by SEQ ID NO: 11.

Also, in order to maintain the enzyme activity, preferred is an amino acid sequence containing one or more, more preferably 1 to 121, further preferably 1 to 91, still further preferably 1 to 61, particularly preferably 1 to 30, most preferably 1 to 12, yet most preferably 1 to 6 amino acid substitutions, deletions, insertions, and/or additions relative to the amino acid sequence represented by SEQ ID NO: 12.

Also, in order to maintain the enzyme activity, preferred is an amino acid sequence containing one or more, more preferably 1 to 105, further preferably 1 to 79, still further preferably 1 to 53, particularly preferably 1 to 26, most preferably 1 to 11, yet most preferably 1 to 5 amino acid substitutions, deletions, insertions, and/or additions relative to the amino acid sequence represented by SEQ ID NO: 13.

Also, in order to maintain the enzyme activity, preferred is an amino acid sequence containing one or more, more preferably 1 to 47, further preferably 1 to 35, still further preferably 1 to 23, particularly preferably 1 to 12, most preferably 1 to 5, yet most preferably 1 to 2 amino acid substitutions, deletions, insertions, and/or additions relative to the amino acid sequence represented by SEQ ID NO: 15.

Also, in order to maintain the enzyme activity, preferred is an amino acid sequence containing one or more, more preferably 1 to 58, further preferably 1 to 44, still further preferably 1 to 29, particularly preferably 1 to 15, most preferably 1 to 6, yet most preferably 1 to 3 amino acid substitutions, deletions, insertions, and/or additions relative to the amino acid sequence represented by SEQ ID NO: 17.

Also, in order to maintain the enzyme activity, preferred is an amino acid sequence containing one or more, more preferably 1 to 57, further preferably 1 to 43, still further preferably 1 to 28, particularly preferably 1 to 14, most preferably 1 to 6, yet most preferably 1 to 3 amino acid substitutions, deletions, insertions, and/or additions relative to the amino acid sequence represented by SEQ ID NO: 67.

Also, in order to maintain the function as SRPP, i.e., the function of being associated with rubber particles in latex, preferred is an amino acid sequence containing one or more, more preferably 1 to 41, further preferably 1 to 31, still further preferably 1 to 20, particularly preferably 1 to 10, most preferably 1 to 4, yet most preferably 1 to 2 amino acid substitutions, deletions, insertions, and/or additions relative to the amino acid sequence represented by SEQ ID NO: 19.

Also, in order to maintain the function as REF, i.e., the function of being associated with rubber particles in latex, preferred is an amino acid sequence containing one or more, more preferably 1 to 28, further preferably 1 to 21, still further preferably 1 to 14, particularly preferably 1 to 7, most preferably 1 to 3, yet most preferably 1 amino acid substitutions, deletions, insertions, and/or additions relative to the amino acid sequence represented by SEQ ID NO: 61.

Among other amino acid substitutions, conservative substitutions are preferred. Specific examples include substitutions within each of the following groups in the parentheses: (glycine, alanine), (valine, isoleucine, leucine), (aspartic acid, glutamic acid), (asparagine, glutamine), (serine, threonine), (lysine, arginine), (phenylalanine, tyrosine), and the like.

It is also known that some proteins with amino acid sequences having high sequence identity with the original amino acid sequence also have similar function. Thus, specific examples of the above-described proteins also include the following [b3], [c3], [d9], [d10], [d11], [d12], [e3], [f3], [f6], [g3], and [h3]:
[b3] a protein comprising an amino acid sequence having 80% or more sequence identity with the amino acid sequence of amino acid numbers 1 to 342 represented by SEQ ID NO: 3, and having an enzyme activity that catalyzes a reaction using isopentenyl diphosphate and dimethylallyl diphosphate as substrates or a reaction using isopentenyl diphosphate and geranyl diphosphate as substrates;
[c3] a protein comprising an amino acid sequence having 80% or more sequence identity with the amino acid sequence of amino acid numbers 1 to 370 represented by SEQ ID NO: 5, and having an enzyme activity that catalyzes a reaction using isopentenyl diphosphate and dimethylallyl diphosphate as substrates, a reaction using isopentenyl diphosphate and geranyl diphosphate as substrates, or a reaction using isopentenyl diphosphate and farnesyl diphosphate as substrates;
[d9] a protein comprising an amino acid sequence having 80% or more sequence identity with the amino acid sequence of amino acid numbers 1 to 575 represented by SEQ ID NO: 10, and having an enzyme activity that catalyzes a reaction of reduction of 3-hydroxy-3-methylglutaryl CoA;
[d10] a protein comprising an amino acid sequence having 80% or more sequence identity with the amino acid sequence of amino acid numbers 1 to 586 represented by SEQ ID NO: 11, and having an enzyme activity that catalyzes a reaction of reduction of 3-hydroxy-3-methylglutaryl CoA;
[d11] a protein comprising an amino acid sequence having 80% or more sequence identity with the amino acid sequence of amino acid numbers 1 to 606 represented by SEQ ID NO: 12, and having an enzyme activity that catalyzes a reaction of reduction of 3-hydroxy-3-methylglutaryl CoA;
[d12] a protein comprising an amino acid sequence having 80% or more sequence identity with the amino acid sequence of amino acid numbers 1 to 526 represented by SEQ ID NO: 13, and having an enzyme activity that catalyzes a reaction of reduction of 3-hydroxy-3-methylglutaryl CoA;
[e3] a protein comprising an amino acid sequence having 80% or more sequence identity with the amino acid sequence of amino acid numbers 1 to 234 represented by SEQ ID NO: 15, and having an enzyme activity that catalyzes a reaction of isomerization of isopentenyl diphosphate or dimethylallyl diphosphate;
[f3] a protein comprising an amino acid sequence having 80% or more sequence identity with the amino acid sequence of amino acid numbers 1 to 290 represented by SEQ ID NO: 17, and having an enzyme activity that catalyzes a reaction of cis-chain elongation of isoprenoid compounds;
[f6] a protein comprising an amino acid sequence having 80% or more sequence identity with the amino acid sequence of amino acid numbers 1 to 284 represented by SEQ ID NO: 67, and having an enzyme activity that catalyzes a reaction of cis-chain elongation of isoprenoid compounds;
[g3] a rubber particle-associated protein comprising an amino acid sequence having 80% or more sequence identity with the amino acid sequence of amino acid numbers 1 to 204 represented by SEQ ID NO: 19, and being associated with rubber particles in latex; and
[h3] a rubber particle-associated protein comprising an amino acid sequence having 80% or more sequence identity with the amino acid sequence of amino acid numbers 1 to 138 represented by SEQ ID NO: 61, and being associated with rubber particles in latex.

In order to maintain the original function of the protein, e.g., the enzyme activity, the sequence identity with the amino acid sequence represented by any one of SEQ ID NOs: 3, 5, 10, 11, 12, 13, 15, 17, 19, 61, and 67 is 80% or more, preferably 85% or more, more preferably 90% or more, further preferably 95% or more, particularly preferably 98% or more, most preferably 99% or more.

Whether it is a protein having a predetermined function such as enzyme activity may be determined by conventional techniques, such as by expressing a target protein in a transformant prepared by introducing a gene encoding the target protein into *Escherichia coli* or the like, and determining the presence or absence of the function of the target protein by the corresponding activity measurement method.

### (Transformant)

By introducing the vector of the present invention (vector comprising a base sequence in which a gene encoding a protein involved in polyisoprenoid biosynthesis is functionally linked to a promoter of a gene encoding MYC1 transcription factor) into a plant, an organism (transformant) which has been transformed to express the predetermined protein involved in polyisoprenoid biosynthesis specifically in laticifers can be obtained. In the transformant, due to the laticifer-specific expression of the predetermined protein involved in polyisoprenoid biosynthesis, the predetermined function, e.g. enzyme activity of the protein is newly enhanced in the laticifers in the plant having the vector of the present invention introduced therein to enhance a part of the polyisoprenoid biosynthesis pathway, resulting in an improved polyisoprenoid production in the plant.

The host to be used for the transformant may be any selected from plants of the genus *Hevea,* such as *Hevea brasiliensis* (para rubber tree); plants of the genus *Sonchus,* such as *Sonchus oleraceus* (common sowthistle), *Sonchus asper,* and *Sonchus brachyotus;* plants of the genus *Solidago,* such as *Solidago altissima* (Canada goldenrod), *Solidago virgaurea* subsp. *asiatica, Solidago virgaurea* subsp. *leipcarpa, Solidago virgaurea* subsp. *leipc arpaf. paludosa, Solidago virgaurea* subsp. *gigantea,* and *Solidago gigantea* Ait. var. *leiophylla* Fernald; plants of the genus *Helianthus,* such as *Helianthus annuus* (sunflower), *Helianthus argophyllus, Helianthus atrorubens, Helianthus debilis, Helianthus decapetalus,* and *Helianthus giganteus;* plants of the genus *Taraxacum,* such as Taraxacum, *Taraxacum venustum* H. Koidz, *Taraxacum hondoense* Nakai, *Taraxacum platycarpum* Dahlst, *Taraxacum japonicum, Taraxacum officinale* Weber, and *Taraxacum kok-saghyz* (Russian dandelion); plants of the genus *Ficus,* such as *Ficus carica, Ficus elastica, Ficus pumila* L., *Ficus erecta* Thunb., *Ficus ampelas* Burm. f., *Ficus benguetensis* Merr., *Ficus irisana* Elm., *Ficus microcarpa* L.f., *Ficus septica* Burm. f., and *Ficus benghalensis;* plants of the genus *Parthenium,* such as *Parthenium argentatum* (guayule), *Parthenium hysterophorus,* and *Parthenium hysterophorus;* and *Lactuca serriola* (lettuce) and Indian banyan. Among them, the plant may more preferably be at least one selected from the group consisting of plants of the genera *Hevea, Sonchus, Taraxacum,* and *Parhenium,* particularly preferably plants of the genus *Hevea,* most preferably *Hevea brasiliensis* (para rubber tree).

The vector of the present invention can be introduced by any method that allows the DNA to be introduced into plant cells . Examples include a method using Agrobacterium (JP S59-140885 A, JP S60-70080 A, WO94/00977, electroporation (JP S60-251887 A, and a method using a particle gun (gene gun) (JP 2606856 B, JP 2517813 B). Among them, the method using Agrobacterium (Agrobacterium method) may preferably be used to introduce the vector of the present invention into a plant to prepare a transformant. In this case, a transformant into which a predetermined gene contained in the vector of the present invention has been introduced can be prepared by introducing the vector of the present invention into a bacterium of the genus *Agrobacterium* and culturing and proliferating the Agrobacterium by an ordinary method (for example, shake culture in YEB medium for 10 to 30 hours at a culture temperature of 20°C to 35°C), followed by infecting a callus, plant tissue slice, or plantlet with the Agrobacterium.

The Agrobacterium containing the vector of the present invention can be prepared by conventional techniques, such as by inserting the base sequence of a promoter of a gene encoding MYC1, the base sequence of a gene encoding a protein involved in polyisoprenoid biosynthesis, and the like into a plasmid capable of homologous recombination with the T-DNA region of the Ti plasmid of Agrobacterium bacteria to prepare a gene recombinant vector as the vector of the present invention and introducing the vector into an Agrobacterium, or by inserting the base sequence of a promoter of a gene encoding MYC1, the base sequence of a gene encoding a protein involved in polyisoprenoid biosynthesis, and the like into the above-described binary vector to prepare a gene recombinant binary vector as the vector of the present invention and introducing the vector into an Agrobacterium.

The Agrobacterium may be *Agrobacterium tumefaciens* (e.g. C58, LBA4404, EHA101, EHA105, C58C1RifR, GV3101).

The transformant (transgenic plant cell) can be obtained by the above-described methods and so forth.

The present invention also provides a transgenic plant into which the vector of the present invention has been introduced. The transgenic plant is not particularly limited as long as the plant has transgenic plant cells. It conceptually includes, for example, not only transgenic plant cells obtained by the above-described methods but also all their progeny or clones and even progeny plants obtained by passaging these cells. Once transgenic plant cells into which the base sequence of a promoter of a gene encoding MYC1 and the base sequence of a gene encoding a protein involved in polyisoprenoid biosynthesis in the vector of the present invention have been introduced in the genome are obtained, progeny or clones can be obtained from the transgenic plant cells by sexual or asexual reproduction, tissue culture, cell culture, cell fusion, or the like. Further, the transgenic plant cells, or progeny or clones thereof may be used to obtain reproductive materials (e.g. seeds, fruits, cuttings, stem tubers, root tubers, shoots, adventitious buds, adventitious embryos, calluses, protoplasts), which can then be used to produce the plant on a large scale.

Techniques to regenerate plants (transgenic plants) from transgenic plant cells are already known; for example, Doi et al. disclose techniques for eucalyptus (JP 2000-316403 A), Fujimura et al. disclose techniques for rice (Fujimura et al., (1995), Plant Tissue Culture Lett., vol. 2: p 74-), Shillito et al. disclose techniques for corn (Shillito et al., (1989), Bio/Technology, vol. 7: p 581-), Visser et al. disclose techniques for potato (Visser et al., (1989), Theor. Appl. Genet., vol. 78: p 589-), and Akama et al. disclose techniques for *Arabidopsis thaliana* (Akama et al., (1992), Plant Cell Rep., vol. 12: p 7-). Those skilled in the art can regenerate plants from transgenic plant cells according to these documents.

An example of the method for preparing the transgenic plant of the present invention will be specifically described below.

An example of the method for preparing the transgenic plant of the present invention may include: an infection step of infecting a callus obtained by culturing a plant-derived tissue under callus-inducing conditions (induction step) for 5 to 9 weeks with an Agrobacterium containing the vector of the present invention; a selective culture step of selectively growing the callus having the vector introduced therein; and a step of inducing an adventitious embryo from the callus (regeneration-inducing step) . With such a preparation method, a transgenic plant can be prepared by preparing transgenic plant cells (transformed callus) by the infection step and the selective culture step, then inducing an adventitious embryo from the callus by the regeneration-inducing step, and culturing the adventitious embryo to regenerate a plant from the callus. More specifically, a plant may be regenerated from the callus by inducing an adventitious embryo from the callus, culturing the adventitious embryo to form a shoot, and culturing the shoot.

More specifically, the method may preferably include: an infection step of infecting a callus obtained by culturing a plant-derived tissue under callus-inducing conditions for 5 to 9 weeks with an Agrobacterium containing the vector of the present invention; a selective culture step of selectively growing the callus having the vector introduced therein; a regeneration-inducing step of culturing the callus in a regeneration-inducing medium to form an adventitious embryo and a shoot; and a rooting step of culturing the shoot in a rooting medium to root it, and more preferably include: an infection step of infecting a callus obtained by culturing a plant-derived tissue under callus-inducing conditions for 5 to 9 weeks with an Agrobacterium containing the vector of the present invention; a selective culture step of selectively growing the callus having the vector introduced therein; a regeneration-inducing step of culturing the callus in a regeneration-inducing medium to form an adventitious embryo and a shoot; an elongation step of culturing the formed shoot in an elongation medium to elongate it; and a rooting step of culturing the elongated shoot in a rooting medium to root it.

Hereinafter, the steps of the method for preparing the transgenic plant of the present invention will be described.

### (Induction Step)

First, a method for preparing a callus (induction step) will be described.

In the induction step, a callus is induced, for example, by culturing a tissue slice (tissue) of a plant in an induction medium containing a plant growth hormone and a carbon source.

The tissue slice may preferably be at least one selected from the group consisting of a leaf, a stem, a root, a bud, a petal, a cotyledon, an anther, and a seed without particular limitation thereto. Among them, it may preferably a leaf or a stem.

In the induction step, the surface of the plant tissue slice is first washed. When an internal tissue of a plant is used as the tissue slice, it may for example be washed using a cleanser or in water containing about 0.1% of a surfactant. When a leaf or the like is used, the surface may be washed using a soft sponge.

Subsequently, the tissue slice is disinfected or sterilized. The disinfection or sterilization may be conducted using a known disinfectant or sterilizer, preferably ethanol, benzalkonium chloride, or aqueous sodium hypochlorite.

Next, callus induction is carried out by culturing the disinfected or sterilized tissue slice in an induction medium containing a plant growth hormone and a carbon source. The induction medium may be either a liquid or a solid, but is preferably a solid medium since callus formation can be facilitated by placing and culturing the tissue slice on the medium. When the induction medium is a liquid medium, static culture or shake culture may be performed.

Examples of the plant growth hormone include auxin plant hormones and/or cytokinin plant hormones.

Examples of auxin plant hormones include 2,4-dichlorophenoxyacetic acid (2,4-D), naphthaleneacetic acid, indolebutyric acid, indoleacetic acid, indolepropionic acid, chlorophenoxyacetic acid, naphthoxyacetic acid, phenylacetic acid, 2,4,5-trichlorophenoxyacetic acid, p-chlorophenoxyacetic acid, 2-methyl-4-chlorophenoxyacetic acid, 4-fluorophenoxyacetic acid, 2-methoxy-3,6-dichlorobenzoic acid, 2-phenyl acid, picloram, and picolinic acid. Among these, 2,4-dichlorophenoxyacetic acid, naphthaleneacetic acid, and indolebutyric acid are preferred, and 2,4-dichlorophenoxyacetic acid is more preferred.

Examples of cytokinin plant hormones include benzyladenine, kinetin (KI), zeatin, benzylaminopurine, isopentenyl aminopurine, thidiazuron, isopentenyl adenine, zeatin riboside, and dihydrozeatin. Among these, benzyladenine, kinetin, and zeatin are preferred, and kinetin is more preferred.

The carbon source is not particularly limited, and examples include sugars such as sucrose, glucose, trehalose, fructose, lactose, galactose, xylose, allose, talose, gulose, altrose, mannose, idose, arabinose, apiose, and maltose. Also, sugar alcohols such as erythritol, xylitol, mannitol, sorbitol, lactitol, and the like may be used. Among them, sucrose is preferred.

The induction medium may be any of the following base media supplemented with the plant growth hormone: basal media such as White's medium (disclosed on pages 20 to 36 of Shokubutsu Saibo Kogaku Nyumon (Introduction to Plant Cell Engineering), Japan Scientific Societies Press), Heller's medium (Heller R., Bot. Biol. Veg. Paris 14, 1-223 (1953)), SH medium (medium of Schenk and Hildebrandt), MS medium (medium of Murashige and Skoog) (disclosed on pages 20 to 36 of Shokubutsu Saibo Kogaku Nyumon (Introduction to Plant Cell Engineering), Japan Scientific Societies Press), LS medium (medium of Linsmaier and Skoog) (disclosed on pages 20 to 36 of Shokubutsu Saibo Kogaku Nyumon (Introduction to Plant Cell Engineering), Japan Scientific Societies Press), Gamborg medium, B5 medium (disclosed on pages 20 to 36 of Shokubutsu Saibo Kogaku Nyumon (Introduction to Plant Cell Engineering), Japan Scientific Societies Press), MB medium, and WP medium (Woody Plant: for woody plants), as well as modified basal media obtained by modifying the composition of the basal media, and the like. Among these, MS medium, B5 medium, or WP medium, supplemented with the plant growth hormone is preferred. Moreover, the medium may preferably contain an auxin plant hormone and a cytokinin plant hormone because such a medium is suitable for maintaining the callus and promoting cell division.

The induction medium may contain at least one selected from the group consisting of jasmonic acid and monoterpene compounds.

Examples of monoterpene compounds include D-limonene, α-pinene, β-pinene, 1-menthol, geraniol, carane, pinane, myrcene, ocimene, cosmene, and so forth. Among them, D-limonene or α-pinene is preferred.

To prepare the induction medium as a solid medium, the medium may be made solid using a solidifying agent. Examples of the solidifying agent include, but are not limited to, agar, gellan gum (e.g. Gelrite), agarose, gelatin, and silica gel.

The suitable composition and culture conditions of the induction medium vary depending on the type of plant, and also vary depending on whether the medium is a liquid medium or a solid medium. Typically (particularly in the case of para rubber tree), the induction medium has the following composition.

The nitrogen concentration in the induction medium may preferably be 0 mM or more, more preferably 1 × 10⁻³ mM or more, further preferably 20 mM or more. The nitrogen concentration may preferably be 100 mM or less, more preferably 70 mM or less.

The concentration of trace inorganic salts in the induction medium may preferably be 0 mM or more, more preferably 1 × 10⁻³ mM or more. The concentration of trace inorganic salts may preferably be 2 mM or less, more preferably 0.23 mM or less .

In the present specification, the "trace inorganic slats" means inorganic salts to be contained in small amounts in the medium, such as boron, manganese, zinc, copper, molybdenum, chlorine, cobalt, titanium, vanadium, aluminum, or silicon. In other words, the trace inorganic salts do not include major inorganic salts (inorganic slats with which culture is not achievable unless they are contained in large amounts in the medium) such as calcium, magnesium, and potassium. Among the trace inorganic salts, boron, manganese, and zinc are preferred, and the combined concentration of boron, manganese, and zinc may preferably be within the above preferred range of the concentration of trace inorganic salts.

The concentration of carbon source in the induction medium may preferably be 0.1 mass% or more, more preferably 1 mass% or more. The concentration of carbon source may preferably be 10 mass% or less, more preferably 6 mass% or less. In the present specification, the concentration of the carbon source means the concentration of sugars.

The calcium ion concentration in the induction medium may preferably be 0 mM or more, more preferably 1 × 10⁻⁵ mM or more, further preferably 0.1 mM or more. The calcium ion concentration may preferably be 10 mM or less, more preferably 5 mM or less.

The concentration of auxin plant hormone in the induction medium may preferably be 0 mg/l or more, more preferably 1 × 10⁻³ mg/l or more, further preferably 1 mg/l or more, particularly preferably 1.5 mg/l or more. The concentration of auxin plant hormone may also preferably be 20 mg/l or less, more preferably 10 mg/l or less, further preferably 3 mg/l or less, particularly preferably 2.5 mg/l or less.

The concentration of cytokinin plant hormone in the induction medium may preferably be 0 mg/l or more, more preferably 1 × 10⁻³ mg/l or more, further preferably 0.5 mg/l or more, particularly preferably 0.8 mg/l or more. The concentration of cytokinin plant hormone may also preferably be 15 mg/l or less, more preferably 10 mg/l or less, further preferably 3 mg/l or less, particularly preferably 1.5 mg/l or less, most preferably 1.2 mg/l or less.

The concentration of jasmonic acid in the induction medium may preferably be 0 mass% or more, more preferably 1 × 10⁻⁶ mass% or more. The concentration of jasmonic acid may preferably be 0.5 mass% or less, more preferably 0.3 mass% or less.

The concentration of monoterpene compound in the induction medium may preferably be 0 mass% or more, more preferably 1 × 10⁻⁶ mass% or more. The concentration of monoterpene compound may preferably be 0.5 mass% or less, more preferably 0.3 mass% or less.

The pH of the induction medium may preferably be 4.0 to 10.0, more preferably 5.0 to 6.5, further preferably 5. 6 to 5.8. The culture temperature may preferably be 0°C to 40°C, more preferably 23°C to 30°C. The culture may be carried out either in a dark place or a bright place, but the illuminance may preferably be 0 to 100000 lx, more preferably 0 to 0.1 lx.

In the present specification, the pH of solid media means the pH of media supplemented with all the components except the solidifying agent. Moreover, in the present specification, the dark place means an illuminance of 0 to 0.1 lx, and the bright place means an illuminance exceeding 0.1 lx.

When the induction medium is a solid medium, the concentration of solidifying agent in the induction medium may preferably be 0.1 mass% or more, more preferably 0.2 mass% or more. The concentration of solidifying agent may also preferably be 2 mass% or less, more preferably 1.1 mass% or less .

Among the above-described conditions, particularly when the plant is para rubber tree, it is particularly preferable that the auxin plant hormone is 2,4-dichlorophenoxyacetic acid at a concentration of 1.5 to 2.5 mg/l, and the cytokinin plant hormone is kinetin at a concentration of 0.8 to 1.2 mg/l.

As described above, callus induction can be carried out by culturing the disinfected or sterilized tissue slice in the induction medium.

In the method for preparing the transgenic plant of the present invention, for example, a callus obtained by culturing a plant-derived tissue under callus-inducing conditions (the above-described induction medium) for 5 to 9 weeks can be used. The culture period from the start of culture of the plant-derived tissue under callus-inducing conditions until the infection of an Agrobacterium is not particularly limited and may appropriately be set in view of the type of plant, the type and amount of plant tissue (tissue slice) used as a material for callus induction, the amount of Agrobacterium bacteria used for infection, and so forth. The callus to be used may preferably be obtained by culturing a plant-derived tissue under callus-inducing conditions for 5 to 9 weeks, more preferably obtained by culturing a plant-derived tissue under callus-inducing conditions for 6 to 8 weeks, particularly preferably obtained by culturing a plant-derived tissue under callus-inducing conditions for 8 weeks.

The callus obtained by culturing a plant-derived tissue under callus-inducing conditions for 8 weeks, for example, means the one that is obtained by culturing a plant-derived tissue for 8 weeks after transfer to the callus induction medium (the above-described induction medium).

### (Infection Step)

In the infection step, the callus obtained by culturing a plant-derived tissue under callus-inducing conditions e.g. for 5 to 9 weeks is infected with an Agrobacterium containing the vector of the present invention.

In the infection step, the callus obtained by culturing a plant-derived tissue under callus-inducing conditions e.g. for 5 to 9 weeks (the callus obtained by the induction step) is infected with an Agrobacterium containing the vector of the present invention (as prepared as described above).

The infection step may be carried out in a manner commonly used in the Agrobacterium method. For example, the infection can be attained by immersing the callus in a suspension obtained by suspending an Agrobacterium containing the vector of the present invention in an infection medium. After the immersion, the suspension and the callus may then be separated from each other using a filter paper or the like. During the immersion, the suspension may be left to stand still or may be shaken, but it is preferable to shake the suspension since shaking facilitates the infection of the callus with the Agrobacterium.

The bacterium concentration in the suspension may appropriately be set in view of the type and proliferation activity of the Agrobacterium, the culture period after the callus induction of the callus to be infected, the immersion period, and so forth. For example, it is preferable to bring 6 g of the callus into contact with the Agrobacterium in an amount that corresponds to 10 to 50 mL, preferably 20 to 40 mL, more preferably 25 to 35 mL of an Agrobacterium suspension having an absorbance measured at 600 nm (O. D. 600) of 0.01 to 0.4, preferably 0.05 to 0.2, more preferably 0.08 to 0.12. In this case, the number of Agrobacterium to infect the callus can be optimized to enable efficient preparation of transgenic plant cells.

The coexistence period of the Agrobacterium and the callus in the infection step, i.e., the period during which the Agrobacterium and the callus are in contact with each other may preferably be 0.5 to 60 minutes, more preferably 1 to 40 minutes, further preferably 25 to 35 minutes. In this case, the number of Agrobacterium to infect the callus can be optimized to enable efficient preparation of transgenic plant cells. The coexistence period means, for example, the immersion period if the callus is immersed in an Agrobacterium suspension.

The infection medium for suspending the Agrobacterium may be any of the above-described base media (e.g., basal media or modified basal media obtained by modifying the composition of the basal media) optionally supplemented with a plant growth hormone. Among them, MS medium, LS medium, B5 medium, or WP medium is preferred, and MS medium is more preferred. As the plant growth hormone and the carbon source, those used for the induction medium may suitably be used, but sucrose is further preferred as the carbon source.

The suitable composition of the infection medium varies depending on the type of plant. Typically (particularly in the case of para rubber tree), the infection medium has the following composition.

The concentration of carbon source in the infection medium may preferably be 0.1 mass% or more, more preferably 1 mass% or more, further preferably 2 mass% or more, particularly preferably 3 mass% or more. The concentration of carbon source may also preferably be 10 mass% or less, more preferably 6 mass% or less, further preferably 5 mass% or less.

For better callus conditions, the infection medium may preferably be an amino acid-containing medium. Examples of the amino acid include aspartic acid, glutamine, glutamic acid, asparagine, proline, and so forth, without particular limitation thereto. Among them, aspartic acid or glutamine is preferred, and it is preferable to use aspartic acid and glutamine in combination.

The concentration of amino acid in the infection medium may preferably be 500 mg/l or more, more preferably 700 mg/l or more, further preferably 1000 mg/l or more. The concentration of amino acid may also preferably be 5000 mg/l or less, more preferably 2000 mg/l or less, further preferably 1300 mg/l or less.

When aspartic acid and glutamine are used in combination in the amino acid-containing medium, the concertation of aspartic acid in the infection medium may preferably be 100 to 700 mg/l, more preferably 200 to 500 mg/l, further preferably 250 to 400 mg/l. On the other hand, the concentration of glutamine in the infection medium may preferably be 100 to 1500 mg/l, more preferably 500 to 1200 mg/l, further preferably 700 to 1100 mg/l.

The infection medium may preferably be an acetosyringone-containing medium because the callus can be more readily infected with Agrobacterium. The concentration of acetosyringone in the infection medium may preferably be 0.1 to 30 mg/l, more preferably 1 to 20 mg/l, further preferably 5 to 15 mg/l.

For better callus conditions and better callus growth, the infection medium may preferably be a casamino acid-containing medium. The concentration of casamino acids in the infection medium may preferably be 50 to 600 mg/l, more preferably 100 to 500 mg/l, further preferably 200 to 400 mg/l.

The pH of the infection medium may preferably be 4.0 to 10.0, more preferably 5.0 to 6.0, without particularly limitation thereto. The temperature for infection (temperature of the infection medium) may preferably be 0°C to 40°C, more preferably 20°C to 36°C, further preferably 22°C to 24°C. The infection step may be carried out either in a dark place or a bright place.

As described above, in the infection step, the callus obtained by the induction step can be infected with an Agrobacterium containing the vector of the present invention, for example, by immersing the callus in a suspension obtained by suspending the Agrobacterium in the infection medium.

### (Coculture Step)

In the coculture step, the callus obtained by the infection step (callus infected with the Agrobacterium), for example, is cultured in a coculture medium. In this case, the gene fragment contained in the vector of the present invention introduced into the callus by infection is incorporated into the genes of the plant cells, whereby stable transgenic plant cells can be obtained.

The coculture medium may be either a liquid or a solid, but solid culture is preferred since stable transgenic plant cells can be obtained by placing the callus on the medium. When the coculture medium is a liquid medium, static culture or shake culture may be performed.

The coculture medium may be any of the above-described base media (e.g., basal media or modified basal media obtained by modifying the composition of the basal media) optionally supplemented with a plant growth hormone. Specifically, those mentioned for the infection medium may suitably be used in the same suitable manner.

When the coculture medium is a solid medium, the medium may be made solid using a solidifying agent in the same manner as in the induction medium.

The culture temperature may preferably be 0°C to 40°C, more preferably 10°C to 36°C, further preferably 20°C to 28°C. The culture may be carried out either in a dark place or a bright place, but the culture may preferably be carried out in a dark place, and the illuminance of the dark place may preferably be 0 to 0.1 lx. The culture period may preferably be 2 to 4 days, without particular limitation thereto.

In the case of a solid medium, the concentration of solidifying agent in the coculture medium may preferably be 0.1 mass% or more, more preferably 0.2 mass% or more. The concentration of solidifying agent may preferably be 2 mass% or less, more preferably 1.1 mass% or less, further preferably 0.6 mass% or less.

As described above, in the coculture step, stable transgenic plant cells can be obtained since the gene fragment contained in the vector of the present invention introduced into the callus by infection is incorporated into the genes of the plant cells by culturing the callus obtained by the infection step (callus infected with the Agrobacterium) in the coculture medium. The calluses (mixture of the transformed callus and the non-transformed callus) obtained by the coculture step are used in the subsequent selective culture step.

### (Selective Culture Step)

The selective culture step may be carried out in a manner commonly used in the Agrobacterium method. With this step, the transformed callus and the non-transformed callus can be separated from each other.

In the selective culture step, the calluses (mixture of the transformed callus and the non-transformed callus) obtained by the coculture step are first washed using any of the above-described base media (e.g., basal media or modified basal media obtained by modifying the composition of the basal media) supplemented with carbenicillin to sterilize Agrobacterium. Before the sterilization, the calluses (mixture of the transformed callus and the non-transformed callus) obtained by the coculture step may previously be washed with the above-described base medium (e.g., basal media or modified basal media obtained by modifying the composition of the basal media).

Subsequently, the calluses sterilized with carbenicillin are cultured in a selective culture medium. The culture conditions in the selective culture step are not particularly limited as long as they allow the transgenic plant cells (callus that has acquired the predetermined promoter and the gene encoding the predetermined protein in the vector of the present invention) to be selectively grown.

The selective culture medium may be a liquid or a solid. When the selective culture medium is a liquid medium, static culture or shake culture may be performed.

The selective culture medium may be any of the above-described base media (e.g., basal media or modified basal media obtained by modifying the composition of the basal media) supplemented with a substance corresponding to the marker gene (the marker gene contained in the vector of the present invention). Among them, MS medium, LS medium, B5 medium, or WP medium supplemented with a substance corresponding to the marker gene is preferred, and MS medium supplemented with a substance corresponding to the marker gene is more preferred. Carbenicillin may optionally be added. Also, a plant growth hormone may optionally be added. As the plant growth hormone and the carbon source, those used in the induction medium may suitably be used.

The substance corresponding to the marker gene is not particularly limited, and those skilled in the art can appropriately select the substance according to the marker gene used. When a hygromycin-resistant gene is used as the marker gene, for example, the calluses (carbenicillin-sterilized calluses (mixture of the transformed callus and the non-transformed callus)) are cultured in the medium supplemented with hygromycin, and then the transformed callus can grow in the medium because the hygromycin-resistant gene is also introduced together with the predetermined promoter and the gene encoding the predetermined protein while the non-transformed callus cannot grow in the medium. As described above, the transformed callus can be selectively grown by culturing a mixture of the transformed callus and the non-transformed callus in the medium supplemented with a substance corresponding to the marker gene.

When the selective culture medium is a solid medium, the medium may be made solid using a solidifying agent in the same manner as in the induction medium.

The pH of the selective culture medium may preferably be 5.0 to 7.0, more preferably 5.6 to 6.5, without particular limitation thereto. The culture temperature may preferably be 0°C to 40°C, more preferably 10°C to 36°C, further preferably 20°C to 28°C. The culture may be carried out either in a dark place or a bright place, but the culture may preferably be carried out in a dark place, and the illuminance of the dark place may preferably be 0 to 0.1 lx. The culture period is not particularly limited, but it is preferable to perform subculture every 1 to 4 weeks.

In the case of a solid medium, the concentration of solidifying agent in the selective culture medium may preferably be 0.1 mass% or more, more preferably 0.2 mass% or more. The concentration of solidifying agent may preferably be 2 mass% or less, more preferably 1.1 mass% or less, further preferably 0.6 mass% or less.

As described above, in the selective culture step, the calluses (mixture of the transformed callus and the non-transformed callus) obtained by the coculture step are washed with the carbenicillin-containing solution to sterilize the Agrobacterium. After that, the calluses sterilized with carbenicillin are cultured in the selective culture medium to selectively grow the transformed callus, whereby the transformed callus and the non-transformed callus can be separated from each other.

With the method described above, transgenic plant cells (transformed callus) can be efficiently prepared. Subsequently, the transformed callus is used in the regeneration-inducing step to stably regenerate a plant.

### (Regeneration-inducing Step)

In the regeneration-inducing step, an adventitious embryo and a shoot are formed by culturing the transformed callus (which may be obtained by proliferating the transformed callus) in a regeneration-inducing medium containing a plant growth hormone and a carbon source. Since it is possible to stably form a shoot by inducing (forming) an adventitious embryo from the callus and culturing the adventitious embryo, the culture conditions in the regeneration-inducing step are not particularly limited as long as they allow an adventitious embryo to be induced from the callus.

In the regeneration-inducing step, an adventitious embryo is induced by culturing the transformed callus in a regeneration-inducing medium. The regeneration-inducing medium may be either a liquid or a solid, but solid culture is preferred since an adventitious embryo can be induced more easily by placing the callus on the medium. When the induction medium is a liquid medium, static culture or shake culture may be performed.

The regeneration-inducing medium may be any of the above-described base media (e.g., basal media or modified basal media obtained by modifying the composition of the basal media) supplemented with a plant growth hormone . Among them, MS medium, LS medium, B5 medium, or WP medium supplemented with a plant growth hormone is preferred, and MS medium supplemented with a plant growth hormone is more preferred. As the plant growth hormone and the carbon source, those used in the induction medium may suitably be used. The medium may preferably contain an auxin plant hormone and a cytokinin plant hormone because such a medium is suitable for inducing an adventitious embryo.

When the regeneration-inducing medium is a solid medium, the medium may be made solid using a solidifying agent in the same manner as in the induction medium.

The suitable composition and culture conditions of the regeneration-inducing medium vary depending on the type of plant, and also vary depending on whether the medium is a liquid medium or a solid medium. Typically (particularly in the case of para rubber tree), the regeneration-inducing medium has the following composition.

The concentration of carbon source in the regeneration-inducing medium may preferably be 0.1 mass% or more, more preferably 1 mass% or more, further preferably 2 mass% or more. The concentration of carbon source may preferably be 10 mass% or less, more preferably 6 mass% or less, further preferably 4 mass% or less.

The concentration of auxin plant hormone in the induction medium may preferably be 0 mg/l or more, more preferably 1 × 10⁻³ mg/l or more, further preferably 5 × 10⁻³ mg/l or more. The concentration of auxin plant hormone may preferably be 5 mg/l or less, more preferably 1 mg/l or less, further preferably 0.5 mg/l or less, particularly preferably 0.1 mg/l or less, most preferably 0.03 mg/l or less, yet most preferably 0.01 mg/l or less.

The concentration of cytokinin plant hormone in the induction medium may preferably be 0 mg/l or more, more preferably 1 × 10⁻³ mg/l or more, further preferably 0.01 mg/l or more, particularly preferably 0.5 mg/l or more, most preferably 0.8 mg/l or more, yet most preferably 1.0 mg/l or more. The concentration of cytokinin plant hormone may preferably be 10 mg/l or less, more preferably 5 mg/l or less, further preferably 2 mg/l or less, particularly preferably 1.5 mg/l or less, most preferably 1.2 mg/l or less. When the concentration of cytokinin plant hormone is within the above-described range, particularly an adventitious embryo can be suitably induced and a shoot can be suitably formed.

The pH of the regeneration-inducing medium may preferably be 4.0 to 10.0, more preferably 5.6 to 6.5, further preferably 5.7 to 5.8, without particular limitation thereto. The culture temperature may preferably be 0°C to 40°C, more preferably 10°C to 36°C, further preferably 20°C to 25°C. The culture may be carried out either in a dark place or a bright place, but the culture may preferably be carried out in a bright place for 10 to 16 hours out of 24 hours, and the illuminance of the bright place may preferably be 2000 to 25000 lx. Regarding the culture period, culture for 1 to 10 months, particularly preferably 3 to 6 months is preferred, without particular limitation thereto. In such a case, it is preferable to perform subculture every 1 to 4 weeks.

In the case of a solid medium, the concentration of solidifying agent in the regeneration-inducing medium may preferably be 0.1 mass% or more, more preferably 0.15 mass% or more. The concentration of solidifying agent may preferably be 2 mass% or less, more preferably 1.1 mass% or less, further preferably 0.6 mass% or less, particularly preferably 0.3 mass% or less.

In the case of para rubber tree, it is preferable that MS medium is used as the base medium for the regeneration-inducing medium and the regeneration-inducing medium has a sucrose concentration of 2 to 4 mass%, a 2, 4-dichlorophenoxyacetic acid concentration of 1 × 10⁻³ to 0.03 mg/l, a kinetin concentration of 0.8 to 1.2 mg/l, and a solidifying agent (gellan gum) concentration of 0.1 to 0.3 mass%.

As described above, in the regeneration-inducing step, an adventitious embryo and a shoot can be formed by culturing the callus in the regeneration-inducing medium. The shoot formed by the regeneration-inducing step is used in the subsequent elongation step. A preferred timing for shifting to the subsequent elongation step is after a shoot has been visually observed and its stable growth has been found. The subsequent elongation step may be omitted to use the shoot formed by the regeneration-inducing step directly in the rooting step.

### (Elongation Step)

In the elongation step, the formed shoot is elongated by culturing the shoot in an elongation medium.

In the elongation step, the shoot formed by the regeneration-inducing step, for example, is elongated by culturing the shoot in an elongation medium. The elongation medium may be either a liquid or a solid, but solid culture is preferred since the shoot can be elongated more easily by placing the shoot on the medium. When the elongation medium is a liquid medium, static culture or shake culture may be performed.

The elongation medium may be any of the above-described basal media or modified basal media obtained by modifying the composition of the basal media, but it may be the same medium as the regeneration-inducing medium because the shoot can be suitably elongated. Among others, the elongation medium may preferably be a medium that does not contain any plant growth hormone, more preferably MS medium that does not contain any plant growth hormone. As the carbon source, those used in the induction medium may suitably be used.

When the elongation medium is a solid medium, the medium may be made solid using solidifying agent in the same manner as in the induction medium.

The suitable culture conditions in the elongation step vary depending on the type of plant and also vary depending on whether the medium is a liquid medium or a solid medium. Typically (particularly in the case of para rubber tree), the following conditions are used.

The pH of the elongation medium may preferably be 4.0 to 10.0, more preferably 5.6 to 6.5, further preferably 5.7 to 5.8, without particular limitation thereto. The culture temperature may preferably be 0°C to 40°C, more preferably 10°C to 36°C, further preferably 20°C to 25°C. The culture may be carried out either in a dark place or a bright place, but the culture may preferably be carried out in a bright place for 10 to 16 hours out of 24 hours, and the illuminance of the bright place may preferably be 2000 to 25000 lx. As for the culture period, culture for 5 to 10 weeks is preferred, without particular limitation thereto.

In the case of a solid medium, the concentration of solidifying agent in the elongation medium may preferably be 0.1 mass% or more, more preferably 0.2 mass% or more. The concentration of solidifying agent may preferably be 2 mass% or less, more preferably 1.1 mass% or less, further preferably 0.6 mass% or less.

As described above, in the elongation step, the formed shoot can be elongated by culturing the short in the elongation medium. Further, not only the elongation of the shoot but also the formation of a new shoot is achieved in the elongation step. The shoot elongated by the elongation step is used in the subsequent rooting step. A preferred timing for shifting to the subsequent rooting step is after the shoot has been elongated to a size of about 2 to 3 cm.

### (Rooting Step)

In the rooting step, the shoot is rooted by culturing in a rooting medium. As the shoot, the shoot elongated by the elongation step may be used, or the shoot formed by the regeneration-inducing step may directly be used.

In the rooting step, the shoot elongated by the elongation step or the shoot formed by the regeneration-inducing step, for example, is rooted by culturing in a rooting medium. The rooting medium may be either a liquid or a solid, but solid culture is preferred since the shoot can be rooted more easily by placing the shoot on the medium. When the rooting medium is a liquid medium, static culture or shake culture may be performed.

The rooting medium may be any of the above-described basal media or modified basal media obtained by modifying the composition of the basal media, but the rooting medium may preferably be a medium that does not contain any plant growth hormone, more preferably 1/2 MS medium that does not contain any plant growth hormone, because the shoot can be suitably rooted. As the carbon source, those used in the induction medium may suitably be used. The composition of the rooting medium may be the same as that of the elongation medium. Moreover, the rooting step may be omitted when rooting has already occurred in the elongation step.

When the rooting medium is a solid medium, the medium may be made solid using a solidifying agent in the same manner as in the induction medium.

The suitable culture conditions in the rooting step vary depending on the type of plant and also vary depending on whether the medium is a liquid medium or a solid medium. Typically (particularly in the case of para rubber tree), the following conditions are used.

The pH of the rooting medium may preferably be 4.0 to 10.0, more preferably 5.6 to 6.5, further preferably 5.7 to 5.8, without particular limitation thereto. The culture temperature may preferably be 0°C to 40°C, more preferably 10°C to 36°C, further preferably 20°C to 25°C. The culture may be carried out either in a dark place or a bright place, but the culture may preferably be carried out in a bright place for 10 to 16 hours out of 24 hours, and the illuminance of the bright place may preferably be 2000 to 25000 lx. As for the culture period, culture for 4 to 10 weeks is preferred, without particular limitation thereto.

In the case of a solid medium, the concentration of solidifying agent in the rooting medium may preferably be 0.1 mass% or more, more preferably 0.2 mass% or more, further preferably 0.3 mass% or more. The concentration of solidifying agent may preferably be 2 mass% or less, more preferably 1.1 mass% or less, further preferably 0.6 mass% or less.

As described above, in the rooting step, the elongated shoot can be rooted by culturing the shoot in the rooting medium, whereby the rooted shoot (plantlet (transgenic plant)) can be obtained. The plantlet may directly be transplanted to soil, but it may preferably be transferred to and acclimatized in an artificial soil such as vermiculite before transplantation to soil.

In the regenerated plant, the expression of the target protein gene can be confirmed by known techniques . For example, the expression of the target protein may be analyzed by Western blot analysis.

In the present invention, by introducing the vector of the present invention into a plant, the gene encoding a protein involved in polyisoprenoid biosynthesis in the vector is expressed specifically in laticifers, thereby improving polyisoprenoid production in the plant. Specifically, a polyisoprenoid can be produced by culturing the transgenic plant cells obtained by the above-described method, the callus obtained from the transgenic plant cells, the cells redifferentiated from the callus, or the like in an appropriate medium or by growing the transgenic plant regenerated from the transgenic plant cells, the plant obtained from the seeds obtained from the transgenic plant, or the like under appropriate cultivation conditions. Since a part of the polyisoprenoid biosynthesis pathway in laticifers is enhanced by the introduced protein in the transgenic plant of the present invention, the protein (enzyme) can function so as to enhance the amount of the compound biosynthesized in the laticifers and therefore to improve polyisoprenoid productivity in the plant.

In the present specification, the term "polyisoprenoid" is a collective term for polymers having isoprene units (C₅H₈). Examples of the polyisoprenoid include monoterpenes (C₁₀), sesquiterpenes (C₁₅), diterpenes (C₂₀), sesterterpenes (C₂₅), triterpenes (C₃₀), tetraterpenes (C₄₀), natural rubber, farnesyl diphosphate, geranylgeranyl diphosphate, and other polymers. Among them, it may preferably be a polymer having isoprene units with a weight average molecular weight of 1000 or more, more preferably a polymer having isoprene units with a weight average molecular weight of 10000 or more, and further preferably a polymer having isoprene units with a weight average molecular weight of 100000 or more.

The weight average molecular weight can be measured by the method described in Examples.

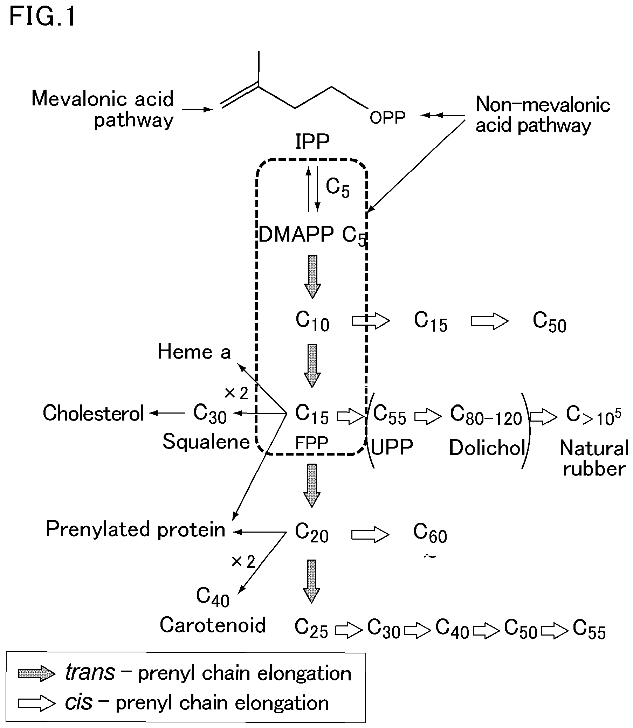

In a polyisoprenoid-producing plant, a polyisoprenoid is biosynthesized via the polyisoprenoid biosynthesis pathway as shown in FIG. 1, in which farnesyl diphosphate synthase is an enzyme that catalyzes the reaction enclosed by the dotted frame in FIG. 1. Accordingly, farnesyl diphosphate synthase activity is increased specifically in laticifers by introducing into a plant a base sequence in which the gene designated by any one of [B1] to [B3] or the gene encoding the protein designated by any one of [b1] to [b3] is functionally linked to a promoter of a gene encoding MYC1 transcription factor. As a result, the amount of farnesyl diphosphate biosynthesized in the laticifers can be enhanced, and therefore polyisoprenoid production in the plant can be improved.

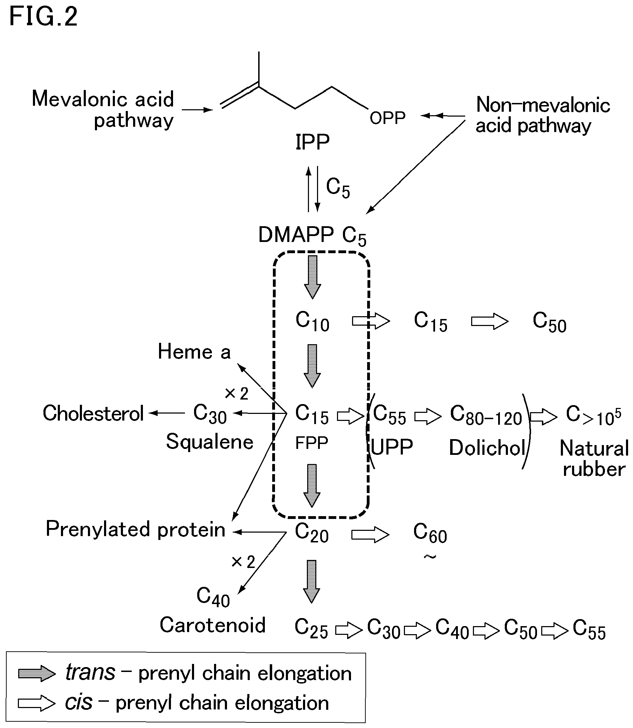

In a polyisoprenoid-producing plant, a polyisoprenoid is biosynthesized via the polyisoprenoid biosynthesis pathway as shown in FIG. 2, in which geranylgeranyl diphosphate synthase is an enzyme that catalyzes the reaction enclosed by the dotted frame in FIG. 2. Accordingly, geranylgeranyl diphosphate synthase activity is increased specifically in laticifers by introducing into a plant a base sequence in which the gene designated by any one of [C1] to [C3] or the gene encoding the protein designated by any one of [c1] to [c3] is functionally linked to a promoter of a gene encoding MYC1 transcription factor. As a result, the amount of geranylgeranyl diphosphate biosynthesized in the laticifers can be enhanced, and therefore polyisoprenoid production can be improved in the plant. Further, it is also possible to increase the weight average molecular weight of polyisoprenoid to be produced. Thus, another aspect of the present invention is a method of increasing the weight average molecular weight of polyisoprenoid to be produced in a plant by introducing into the plant a vector comprising a base sequence in which a gene encoding geranylgeranyl diphosphate synthase is functionally linked to a promoter of a gene encoding MYC1 transcription factor.

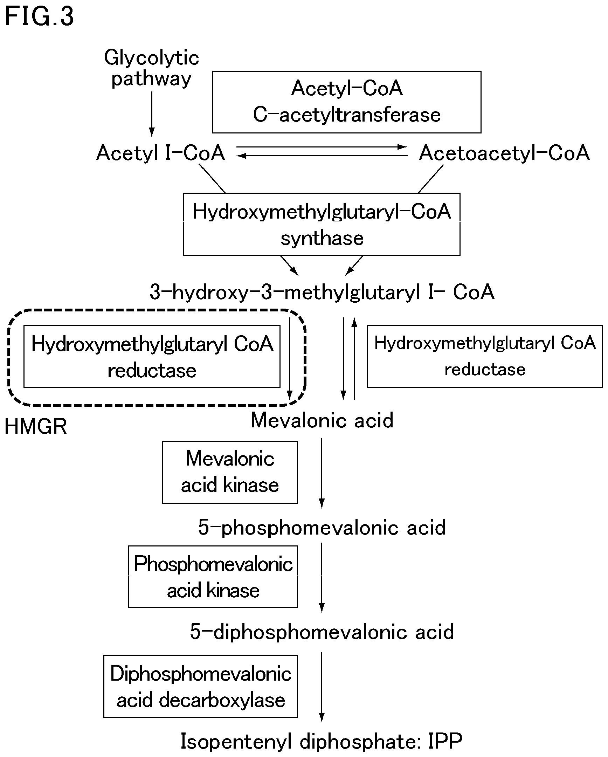

In a polyisoprenoid-producing plant, a polyisoprenoid is biosynthesized via the polyisoprenoid biosynthesis pathway as shown in FIG. 1, and the mevalonic acid pathway located upstream of the pathway is the one as shown in FIG. 3, in which 3-hydroxy-3-methylglutaryl CoA reductase is an enzyme that catalyzes the reaction enclosed by the dotted frame in FIG. 3 and is a rate-limiting enzyme in the mevalonic acid pathway. Accordingly, 3-hydroxy-3-methylglutaryl CoA reductase activity is increased specifically in laticifers by introducing into a plant a base sequence in which the gene designated by any one of [D1] to [D12] or the gene encoding the protein designated by any one of [d1] to [d12] is functionally linked to a promoter of a gene encoding MYC1 transcription factor. As a result, the amount of mevalonic acid biosynthesized in the laticifers can be enhanced, which leads to an improved amount of isopentenyl diphosphate biosynthesized and therefore an improved polyisoprenoid production in the plant. Further, it is also possible to increase the weight average molecular weight of polyisoprenoid to be produced. Thus, another aspect of the present invention is a method of increasing the weight average molecular weight of polyisoprenoid to be produced in a plant by introducing into the plant a vector comprising a base sequence in which a gene encoding 3-hydroxy-3-methylglutaryl CoA reductase is functionally linked to a promoter of a gene encoding MYC1 transcription factor.

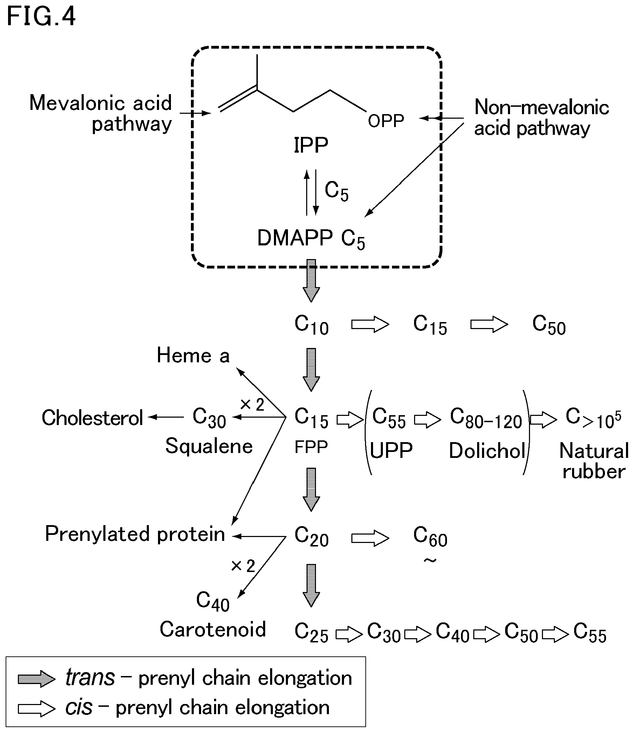

In a polyisoprenoid-producing plant, a polyisoprenoid is biosynthesized via the polyisoprenoid biosynthesis pathway as shown in FIG. 4, in which isopentenyl diphosphate isomerase is an enzyme that catalyzes the reaction enclosed by the dotted frame in FIG. 4. Accordingly, isopentenyl diphosphate isomerase activity is increased specifically in laticifers by introducing into a plant a base sequence in which the gene designated by any one of [E1] to [E3] or the gene encoding the protein designated by any one of [e1] to [e3] is functionally linked to a promoter of a gene encoding MYC1 transcription factor. As a result, the amount of isopentenyl diphosphate or dimethylallyl diphosphate biosynthesized in the laticifers can be enhanced, and therefore polyisoprenoid production can be improved in the plant.

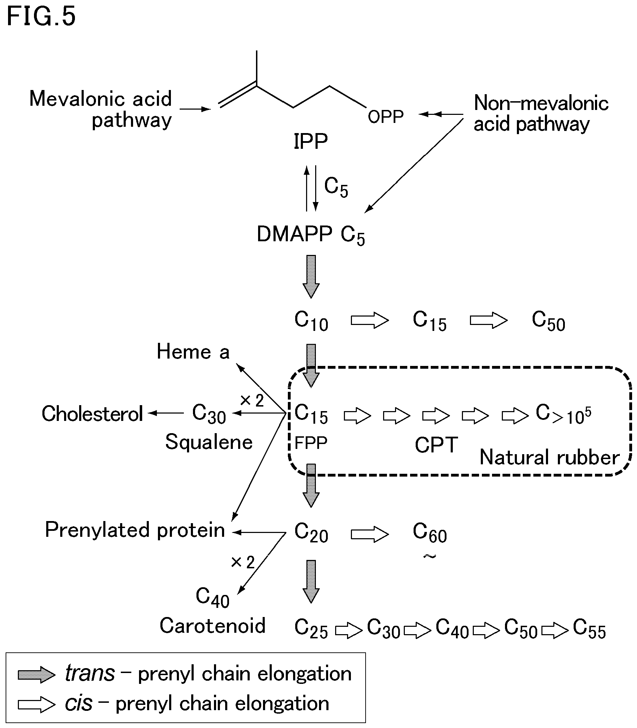

In a polyisoprenoid-producing plant, a polyisoprenoid is biosynthesized via the polyisoprenoid biosynthesis pathway as shown in FIG. 5, in which cis-prenyltransferase is considered to be an enzyme that catalyzes the reaction enclosed by the dotted frame in FIG. 5. Accordingly, cis-prenyltransferase activity is increased specifically in laticifers by introducing into a plant a base sequence in which the gene designated by any one of [F1] to [F6] or the gene encoding the protein designated by any one of [f1] to [f6] is functionally linked to a promoter of a gene encoding MYC1 transcription factor. As a result, the amount of cis isoprenoid biosynthesized in the laticifers can be enhanced, and therefore polyisoprenoid production can be improved in the plant. Further, it is also possible to increase the weight average molecular weight of polyisoprenoid to be produced. Thus, another aspect of the present invention is a method of increasing the weight average molecular weight of polyisoprenoid to be produced in a plant by introducing into the plant a vector comprising a base sequence in which a gene encoding cis-prenyltransferase is functionally linked to a promoter of a gene encoding MYC1 transcription factor.

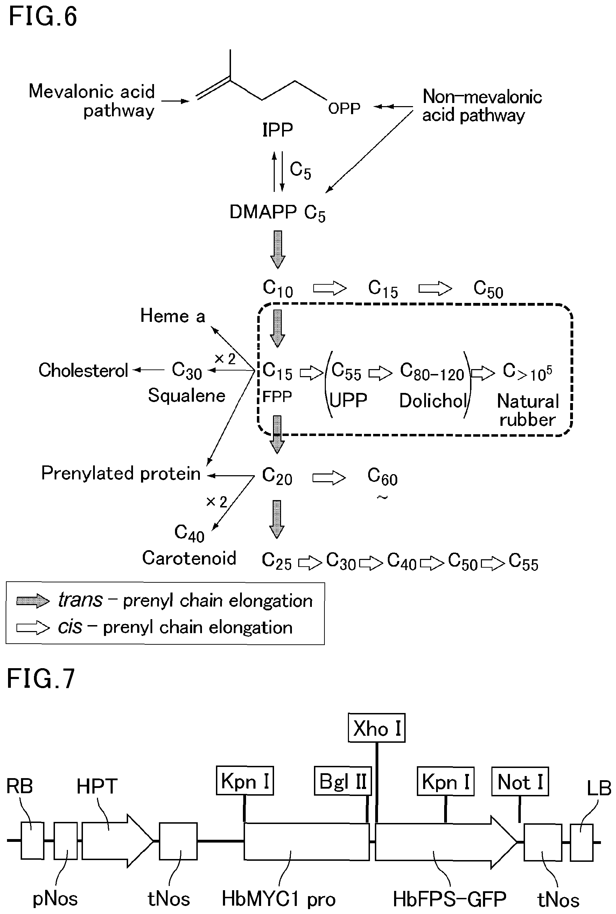

In a polyisoprenoid-producing plant, a polyisoprenoid is biosynthesized via the polyisoprenoid biosynthesis pathway as shown in FIG. 6, in which Small Rubber Particle Protein is considered to be involved in the catalytic reaction of the enzyme catalyzing the reaction enclosed by the dotted frame in FIG. 6. Accordingly, the expression of Small Rubber Particle Protein is increased specifically in laticifers by introducing into a plant a base sequence in which the gene designated by any one of [G1] to [G3] or the gene encoding the protein designated by any one of [g1] to [g3] is functionally linked to a promoter of a gene encoding MYC1 transcription factor. As a result, the amount of cis isoprenoid biosynthesized in the laticifers can be enhanced, and therefore polyisoprenoid production can be improved in the plant. Further, it is also possible to increase the weight average molecular weight of polyisoprenoid to be produced. Thus, another aspect of the present invention is a method of increasing the weight average molecular weight of polyisoprenoid to be produced in a plant by introducing into the plant a vector comprising a base sequence in which a gene encoding Small Rubber Particle Protein is functionally linked to a promoter of a gene encoding MYC1 transcription factor.

Rubber Elongation Factor is also considered to be involved in the catalytic reaction of the enzyme catalyzing the reaction enclosed by the dotted frame in FIG. 6. Accordingly, the expression of Rubber Elongation Factor is increased specifically in laticifers by introducing into a plant a base sequence in which the gene designated by any one of [H1] to [H3] or the gene encoding the protein designated by any one of [h1] to [h3] is functionally linked to a promoter of a gene encoding MYC1 transcription factor. As a result, the amount of cis isoprenoid biosynthesized in the laticifers can be enhanced, and therefore polyisoprenoid production can be improved in the plant.

In the present invention, though polyisoprenoid production in a plant is improved by introducing into the plant at least one of the genes encoding the seven kinds of proteins, polyisoprenoid production in a plant is further improved by introducing into the plant two or more of the genes encoding the seven kinds of proteins, and the effect of the invention is most significantly achieved by introducing all the genes of the seven kinds into the plant.

### EXAMPLES

The present invention will specifically be described with reference to Examples, but the invention is not limited to the Examples.

### (Preparation of Base Sequence of Promoter of Gene Encoding MYC1)

Cloning of a DNA fragment containing a gene encoding MYC1 transcription factor (MYC1) derived from leaves of para rubber tree and its promoter was carried out by the following procedures. First, a genomic DNA was extracted from leaves of para rubber tree. The extraction was carried out by the CTAB method. A gene including the promoter region of the gene encoding MYC1 was amplified by TAIL-PCR using random primers shown as Primers 1 to 2 below and primers corresponding to the gene encoding MYC1.
(Primer 1: SEQ ID NO: 20)
   5'-CTCTACCAGCTATGATTCACC-3'
(Primer 2: SEQ ID NO: 21)
   5'-TGATATCTTCTGCATGCACC-3'

As a result of investigation of the base sequence of a DNA fragment obtained using the above-described primers, it was confirmed that the promoter sequence of the gene encoding MYC1 was obtained. The base sequence of the promoter sequence of the gene encoding MYC1 is shown as SEQ ID NO: 1.

### (Amplification of Promoter Sequence for Introduction into Vector)

Primers 3 and 4 shown below to each of which a restriction enzyme site had been added were designed to be able to be incorporated into a vector for plants, and the above-identified promoter sequence -1 to -1610 bp of the gene encoding MYC1 was amplified by PCR.
(Primer 3: SEQ ID NO: 22)
   5'-GGTACCTACCAGCTATGATTCACCTC-3'
(Primer 4: SEQ ID NO: 23)
   5'-AGATCTAGCACCTTAGTATTTCAATTTTG-3'

### (Identification of Amino Acid Sequences and Base Sequences of Target Proteins of Enzymes Involved in Polyisoprenoid Biosynthesis Pathway in Para Rubber Tree)

Total RNA was extracted from leaves of para rubber tree, and a cDNA was synthesized by reverse transcription reaction using oligo-dT primers.

Next, based on the DNA database of para rubber tree, Primers 5 to 22, 41, 42, 45, and 46 below were designed and the whole length DNA fragments of target proteins were amplified by RT-PCR to identify the base sequences of the DNA fragments and the whole length amino acid sequences of the target proteins. The base sequences of the genes encoding the target proteins are shown as SEQ ID NOs: 2, 4, 6 to 9, 14, 16, 18, 60, and 66, and the amino acid sequences of the target proteins are shown as SEQ ID NOs: 3, 5, 10 to 13, 15, 17, 19, 61, and 67.

The primers used when the target protein was farnesyl diphosphate synthase are shown below.
(Primer 5: SEQ ID NO: 24)
   5'-GAATCCATGGCGGATCTGAAG-3'
(Primer 6: SEQ ID NO: 25)
   5'-GTCCATGTATCTGGATACCC-3'

The primers used when the target protein was geranylgeranyl diphosphate synthase are shown below.
(Primer 7: SEQ ID NO: 26)
   5'-CAAGATGAGTTCAGTGAATTTGGG-3'
(Primer 8: SEQ ID NO: 27)
   5'-TGCATTAGTTTTGCCTGTGAGC-3'

The primers used when the target protein was 3-hydroxy-3-methylglutaryl CoA reductase 1 are shown below.
(Primer 9: SEQ ID NO: 28)
   5'-ATTTTTACATGGACACCACCG-3'
(Primer 10: SEQ ID NO: 29)
   5'-ACCAGATTCCCACTAAGATGC-3'

The primers used when the target protein was 3-hydroxy-3-methylglutaryl CoA reductase 3 are shown below.
(Primer 11: SEQ ID NO: 30)
   5'-TCCATATATGGACGAGGTTCG-3'
(Primer 12: SEQ ID NO: 31)
   5'-GCAGCTGTGTTACCCTTCAG-3'

The primers used when the target protein was 3-hydroxy-3-methylglutaryl CoA reductase 4 are shown below.
(Primer 13: SEQ ID NO: 32)
   5'-CAGTCGCTCCAAAATGGATGTGC-3'
(Primer 14: SEQ ID NO: 33)
   5'-GATTTTCTTAGGAAGAAGGCTTGG-3'

The primers used when the target protein was 3-hydroxy-3-methylglutaryl CoA reductase 5 are shown below.
(Primer 15: SEQ ID NO: 34)
   5'-CTAGCTGGTCTATAATGGATGCC-3'
(Primer 16: SEQ ID NO: 35)
   5'-GAATCAATTTACCTCAATAGAAGGC-3'

The primers used when the target protein was isopentenyl diphosphate isomerase are shown below.
(Primer 17: SEQ ID NO: 36)
   5'-TTCCACCATGGGTGAGGCTCC-3'
(Primer 18: SEQ ID NO: 37)
   5'-TCTCAACTCAACTTGTGAATCG-3'

The primers used when the target protein was cis-prenyltransferase 1 are shown below.
(Primer 19: SEQ ID NO: 38)
   5'-ATGGAATTATACAACGGTGAGAGG-3'
(Primer 20: SEQ ID NO: 39)
   5'-TTTTAAGTATTCCTTATGTTTCTCC-3'

The primers used when the target protein was cis-prenyltransferase 2 are shown below.
(Primer 45: SEQ ID NO: 68)
   5'-ATGGAATTATACAACGGTGAGAGG-3'
(Primer 46: SEQ ID NO: 69)
   5'-TTTTAAGTATTCCTTATGTTTCTCC-3'

The primers used when the target protein was Small Rubber Particle Protein are shown below.
(Primer 21: SEQ ID NO: 40)
   5'-TATGGCTGAAGAGGTGGAGG-3'
(Primer 22: SEQ ID NO: 41)
   5'-TGATGCCTCATCTCCAAACACC-3'

The primers used when the target protein was Rubber Elongation Factor are shown below.
(Primer 41: SEQ ID NO: 62)
   5'-ATGGCTGAAGACGAAGACAACC-3'
(Primer 42: SEQ ID NO: 63)
   5'-ATTCTCTCCATAAAACACCTTAGC-3'

### (Amplification of Base Sequences of Genes Encoding Target Proteins for Introduction into Vector)

Primers 23 to 40, 43, 44, 47, and 48 to each of which a restriction enzyme site had been added were designed to be able to be incorporated into a vector for plants, and the whole length genes encoding the target proteins were amplified by RT-PCR.

The primers used when the target protein was farnesyl diphosphate synthase are shown below.
(Primer 23: SEQ ID NO: 42)
   5'-CTCGAGAACAATGGCGGATCTGAAGTCAAC-3'
(Primer 24: SEQ ID NO: 43)
   5'-GGTACCTGTTTCTGTCTCTTGTAAATTTTGGC-3'

The primers used when the target protein was geranylgeranyl diphosphate synthase are shown below.
(Primer 25: SEQ ID NO: 44)
   5'-CTCGAGAACAATGAGTTCAGTGAATTTGGG-3'
(Primer 26: SEQ ID NO: 45)
   5'-GGATCCTTGTTTTGCCTGTGAGCGATGTAATTAGC-3'

The primers used when the target protein was 3-hydroxy-3-methylglutaryl CoA reductase 1 are shown below.
(Primer 27: SEQ ID NO: 46)
   5'-CTCGAGACAAATGGACACCACCGGCCGGCTCC-3'
(Primer 28: SEQ ID NO: 47)
   5'-GGTACCACAGATGCAGCTTTAGACATATCTTTGC-3'

The primers used when the target protein was 3-hydroxy-3-methylglutaryl CoA reductase 3 are shown below.
(Primer 29: SEQ ID NO: 48)
   5'-CTCGAGACAAATGGACGAGGTTCGCCGGCGACC-3'
(Primer 30: SEQ ID NO: 49)
   5'-GGTACCACGAAAGTTATTTTGGATACATCTTTTGC-3'

The primers used when the target protein was 3-hydroxy-3-methylglutaryl CoA reductase 4 are shown below.
(Primer 31: SEQ ID NO: 50)
   5'-AAGCTTACAAATGGATGTGCGCCGGCGACC-3'
(Primer 32: SEQ ID NO: 51)
   5'-GGTACCACGGAAGAAGGCTTGGAAACAGC-3'

The primers used when the target protein was 3-hydroxy-3-methylglutaryl CoA reductase 5 are shown below.
(Primer 33: SEQ ID NO: 52)
   5'-AAGCTTACAAATGGATGCCCGCCGGCGACC-3'
(Primer 34: SEQ ID NO: 53)
   5'-GGTACCACATTTACCTCAATAGAAGGCATTGTC-3'

The primers used when the target protein was isopentenyl diphosphate isomerase are shown below.
(Primer 35: SEQ ID NO: 54)
   5'-CTCGAGAACAATGGGTGAGGCTCCAGATGTCG-3'
(Primer 36: SEQ ID NO: 55)
   5'-GGTACCTGACTCAACTTGTGAATCGTTTTCATGTC-3'

The primers used when the target protein was cis-prenyltransferase 1 are shown below.
(Primer 37: SEQ ID NO: 56)
   5'-CTCGAGCCAACAATGGAATTATACAACG-3'
(Primer 38: SEQ ID NO: 57)
   5'-GGATCCTCTTTTAAGTATTCCTTATGTTTCTCC-3'

The primers used when the target protein was cis-prenyltransferase 2 are shown below.
(Primer 47: SEQ ID NO: 70)
   5'-CTCGAGCCAACAATGGAATTATACAACG-3'
(Primer 48: SEQ ID NO: 71)
   5'-GGATCCTCTTTTAAGTATTCCTTATGTTTCTCC-3'

The primers used when the target protein was Small Rubber Particle Protein are shown below.
(Primer 39: SEQ ID NO: 58)
   5'-CTCGAGAACAATGGCTGAAGAGGTGGAGG-3'
(Primer 40: SEQ ID NO: 59)
   5'-GGATCCTGTGATGCCTCATCTCCAAACACC-3'

The primers used when the target protein was Rubber Elongation Factor are shown below.
(Primer 43: SEQ ID NO: 64)
   5'-CTCGAGAACAATGGCTGAAGACGAAGACAACC-3'
(Primer 44: SEQ ID NO: 65)
   5'-GGATCCAAATTCTCTCCATAAAACACCTTAGC-3'

### (Construction of Expression Vectors)

A pH35GS vinery vector (Inplanta Innovations Inc.) was digested by restriction enzymes Kpn I and Bgl II, and the promoter sequence of the gene encoding MYC1 amplified in (Amplification of Promoter Sequence for Introduction into Vector) was treated with the restriction enzymes in the same manner and they were ligated using Ligation high (Toyobo Co., Ltd.) .

Also, each of the genes (base sequences) encoding the target proteins amplified in (Amplification of Base Sequences of Genes Encoding Target Proteins for Introduction into Vector) was digested by restriction enzymes, and a Gateway sGFP entry clone vector (Evorogen) was treated with the restriction enzymes in the same manner and they were ligated using Ligation high (Toyobo Co., Ltd.).

The prepared binary vector and entry clone vector were reacted using LR Clonase II enzyme mix (Invitrogen) to insert the base sequence of the GFP (green fluorescent protein) gene and the gene sequence encoding the target protein on the entry clone into pH35GS. In this manner, expression vectors in each of which each of the gene sequences encoding the target proteins and the base sequence of the GFP gene were functionally linked to the promoter sequence (HbMYC1 pro) of the gene encoding MYC1 were constructed. The pH35GS vector had a hygromycin-resistant gene (HPT).

When the target protein was farnesyl diphosphate synthase, the gene encoding farnesyl diphosphate synthase and the Gateway sGFP entry clone vector were digested by restriction enzymes XhoI and KpnI. The thus-constructed expression vector is shown in FIG. 7. In FIG. 7, HbFPS represents the base sequence of the gene encoding farnesyl diphosphate synthase.

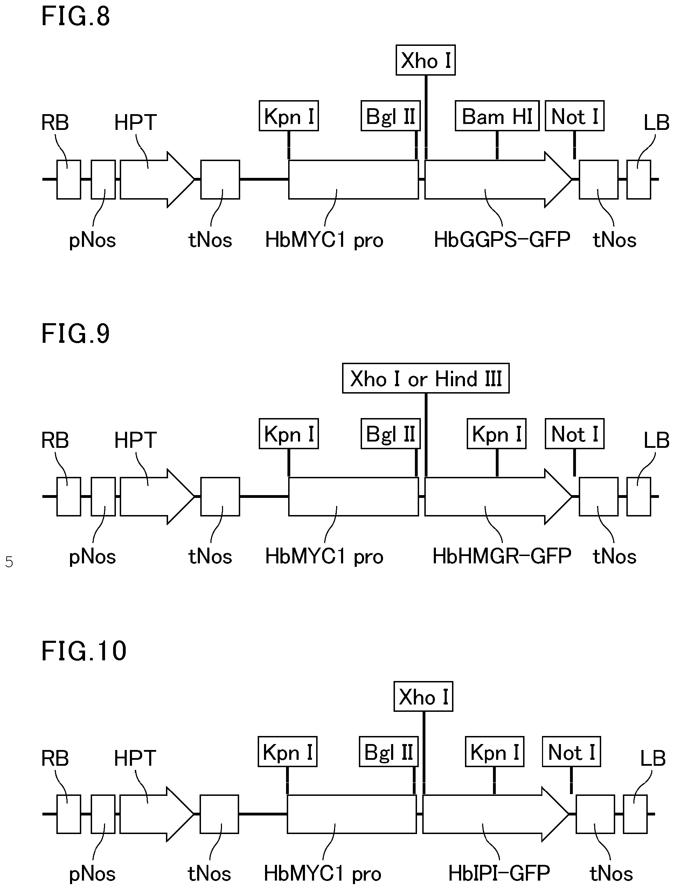

When the target protein was geranylgeranyl diphosphate synthase, the gene encoding geranylgeranyl diphosphate synthase and the Gateway sGFP entry clone vector were digested by restriction enzymes XhoI and BamHI. The thus-constructed expression vector is shown in FIG. 8. In FIG. 8, HbGGPS represents the base sequence of the gene encoding geranylgeranyl diphosphate synthase.

When the target protein was 3-hydroxy-3-methylglutaryl CoA reductase 1, 3, 4, or 5, the gene encoding 3-hydroxy-3-methylglutaryl CoA reductase 1, 3, 4, or 5 and the Gateway sGFP entry clone vector were digested by restriction enzymes XhoI and KpnI, or HindIII and KpnI. The thus-constructed expression vector is shown in FIG. 9. In FIG. 9, HbHMGR represents the base sequence of the gene encoding 3-hydroxy-3-methylglutaryl CoA reductase.

When the target protein was isopentenyl diphosphate isomerase, the gene encoding isopentenyl diphosphate isomerase and the Gateway sGFP entry clone vector were digested by restriction enzymes XhoI and KpnI. The thus-constructed expression vector is shown in FIG. 10. In FIG. 10, HbIPI represents the base sequence of the gene encoding isopentenyl diphosphate isomerase.

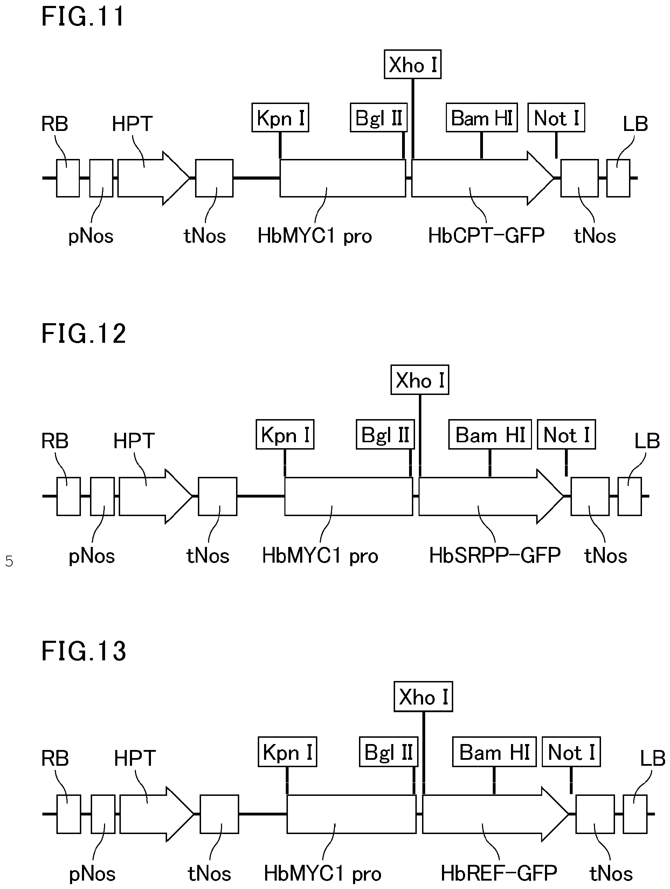

When the target protein was cis-prenyltransferase 1 or 2, the gene encoding cis-prenyltransferase 1 or 2 and the Gateway sGFP entry clone vector were digested by restriction enzymes XhoI and BamHI. The thus-constructed expression vector is shown in FIG. 11. In FIG. 11, HbCPT represents the base sequence of the gene encoding cis-prenyltransferase.

When the target protein was Small Rubber Particle Protein, the gene encoding Small Rubber Particle Protein and the Gateway sGFP entry clone vector were digested by restriction enzymes XhoI and BamHI. The thus-constructed expression vector is shown in FIG. 12. In FIG. 12, HbSRPP represents the base sequence of the gene encoding Small Rubber Particle Protein.

When the target protein was Rubber Elongation Factor, the gene encoding Rubber Elongation Factor and the Gateway sGFP entry clone vector were digested by restriction enzymes XhoI and BamHI. The thus-constructed expression vector is shown in FIG. 13. In FIG. 13, HbREF represents the base sequence of the gene encoding Rubber Elongation Factor.

### (Preparation of Expression Vector-Introduced Agrobacterium)

Each of the expression vectors constructed in (Construction of Expression Vectors) was infected with Agrobacterium to prepare an expression vector-introduced Agrobacterium. Specifically, the expression vectors were each introduced into Agrobacterium by electroporation and subjected to shake culture in YEB medium at 28°C for 24 hours. After that, the culture liquid was centrifuged to recover the Agrobacterium, which was then suspended into a suspension (MS medium) at a concentration corresponding to O. D. 600 = 0.6.

### [Examples 1 to 11]

The chemicals used in Examples are listed below:
NAA: naphthaleneacetic acid;
2,4-D: 2,4-dichlorophenoxyacetic acid;
IBA: indolebutyric acid;
BA: benzyladenine;
KI: kinetin; and
para rubber tree: obtained from the Arboricultural Research Institute of the University of Tokyo Forests, Graduate School of Agricultural and Life Sciences, the University of Tokyo.

### (Callus Induction (Induction Step))

Leaves were collected from the para rubber tree. Then, after the surface of the collected leaves was washed in running water and then with 70% ethanol, they were sterilized with a sodium hypochlorite solution diluted to about 5 to 10% and washed again in running water.

Next, a tissue of the sterilized leaves was inserted into an induction medium (solid medium) and cultured (induction step) . The induction medium was prepared by adding to MS medium (disclosed on pages 20 to 36 of Shokubutsu Saibo Kogaku Nyumon (Introduction to Plant Cell Engineering), Japan Scientific Societies Press) 2,4-dichlorophenoxyacetic acid (2,4-D), kinetin (KI), and sucrose at concentrations of 2.0 mg/l, 1.0 mg/l, and 3 mass%, respectively, adjusting the pH of the medium to 5.7 to 5.8, adding thereto gellan gum at a concentration of 0.2 mass%, sterilizing the medium in an autoclave (121°C, 20 minutes), and cooling it in a clean bench.

A tissue slice of the para rubber tree was inserted into an induction medium (solid medium) and cultured at a culture temperature of 25°C in a dark place (0 to 0.1 lx) for 8 weeks to induce a callus (undifferentiated cells) from the tissue slice of the para rubber tree.

### (Infection Step and Coculture Step)

After the induced callus was contacted with the suspension (MS medium) containing the expression vector-introduced Agrobacterium prepared in (Preparation of Expression Vector-Introduced Agrobacterium) for 30 minutes, the callus was cultured in MS medium under dark place conditions at 28°C for 3 days.

Here, the Agrobacterium into which the expression vector shown in FIG. 7 had been introduced was used in Example 1; the Agrobacterium into which the expression vector shown in FIG. 8 had been introduced was used in Example 2; the Agrobacterium into which the expression vector with HMGR1 shown in FIG. 9 had been introduced was used in Example 3; the Agrobacterium into which the expression vector shown in FIG. 10 had been introduced was used in Example 4; the Agrobacterium into which the expression vector with CPT1 shown in FIG. 11 had been introduced was used in Example 5; the Agrobacterium into which the expression vector shown in FIG. 12 had been introduced was used in Example 6; the Agrobacterium into which the expression vector shown in FIG. 13 had been introduced was used in Example 7; the Agrobacterium into which the expression vector with HMGR3 shown in FIG. 9 had been introduced was used in Example 8; the Agrobacterium into which the expression vector with HMGR4 shown in FIG. 9 had been introduced was used in Example 9; the Agrobacterium into which the expression vector with HMGR5 shown in FIG. 9 had been introduced was used in Example 10; and the Agrobacterium into which the expression vector with CPT2 shown in FIG. 11 had been introduced was used in Example 11.

### (Selective Culture Step)

Next, after the callus was sterilized with carbenicillin, it was transferred to a hygromycin-resistant MS selective medium and cultured at 28°C with a 16 hour daylength for 2 months to prepare a transformant.

### (Formation of Adventitious Embryo and Shoot (Regeneration-inducing Step))

Next, an adventitious embryo and a shoot were formed from the transformant (callus survived through the selective culture step) in MS medium (regeneration-inducing step). Specifically, the transformant was cultured in the medium at a culture temperature of 25°C in 12 hours light (10000 lx) per 24 hour day for 3 to 6 months. During the culture, media were replaced every one month. As a result, it was observed that an adventitious embryo was formed and then a shoot was also formed.

### (Shoot Elongation (Elongation Step))

Next, for shoot elongation, the formed shoot was subcultured in a medium having the same composition as that of the regeneration-inducing medium. Specifically, the shoot was cultured at a culture temperature of 25°C in 12 hours light (10000 lx) per 24 hour day for 8 weeks. As a result of the culture, good shoot elongation was observed.

### (Rooting (Rooting Step))

Next, for rooting, the shoot grown to about 3 cm was transplanted in 1/2 MS medium. The shoot was cultured at a culture temperature of 25°C in 12 hours light (10000 lx) per 24 hour day for 8 weeks . As a result of the culture, good rooting was observed, whereby a transgenic plant was obtained.

### (Calculation of Polyisoprenoid Production and Measurement of Weight Average Molecular Weight of Polyisoprenoid)

A volume of 200 µl of latex exuded by cutting the stem of each of the transgenic plants obtained in Examples 1 to 7 was collected, and 100 µl out of the collected latex was extracted with 99% ethanol to remove unwanted metabolites. After that, it was further extracted with 99% toluene to purify and recover a polyisoprenoid. Then, the amount of the produced polyisoprenoid (recovered toluene extract) (the amount of the polyisoprenoid accumulated in the transgenic plant (intracellular accumulation of polyisoprenoid), i.e., polyisoprenoid production) was calculated.

The polyisoprenoid accumulation was calculated as a ratio (mass%) of the amount of polyisoprenoid purified and recovered from the transgenic plant to the amount of polyisoprenoid accumulated in the wild type (non-recombinant form; also referred to as "control") after the recovered toluene extract was dried. The results are shown in Table 1.

The weight average molecular weight (Mw) of polyisoprenoid was measured by gel permeation chromatography (GPC) under the following conditions (1) to (7). The results are shown in Table 1.
(1) Apparatus: HLC-8020 (Tosoh Corporation)
(2) Separation column: GMH-XL (Tosoh Corporation)
(3) Measurement temperature: 40°C
(4) Carrier: Tetrahydrofuran
(5) Flow rate: 0.6 ml/min.
(6) Detector: Differential refractometer, UV
(7) Molecular weight standards: Polystyrene standards

### [Comparative Examples 1 to 7]

Transgenic plants were obtained in the same manner as in Examples 1 to 7, except for using a cauliflower mosaic virus 35S promoter in place of the promoter of the gene encoding MYC1, i.e. except for not introducing the promoter sequence of the gene encoding MYC1 amplified in (Amplification of Promoter Sequence for Introduction into Vector). Then, polyisoprenoid accumulations were calculated and weight average molecular weights of polyisoprenoids were measured. The results are shown in Table 1.

**[Table 1]**

| | Control | Example 1 | Comparative Example 1 | Example 2 | Comparative Example 2 | Example 3 | Comparative Example 3 | Example 4 | Comparative Example 4 | Example 5 | Comparative Example 5 | Example 6 | Comparative Example 6 | Example 7 | Comparative Example 7 |
|---|---|---|---|---|---|---|---|---|---|---|---|---|---|---|---|
| Promoter | - | MYC1 pro | 35S | MYC1 pro | 35S | MYC1 pro | 35S | MYC1 pro | 35S | MYC1 pro | 35S | MYC1 pro | 35S | MYC1 pro | 35S |
| Protein encoded by gene introduced into transgenic plant | - | FPS | FPS | GGPS | GGPS | HMGR1 | HMGR1 | IPI | IPI | CPT1 | CPT1 | SRPP | SRPP | REF | REF |
| Polyisoprenoid accumulation (% by mass) | 100 | 104 | 101 | 103 | 100 | 105 | 102 | 104 | 102 | 108 | 101 | 106 | 102 | 107 | 102 |
| Weight average molecular weight (Mw) of polyisoprenoid | 8.8 × 10⁵ | 8.8 × 10⁵ | 8.8 × 10⁵ | 9.1 × 10⁵ | 8.8 × 10⁵ | 9.0 × 10⁵ | 8.9 × 10⁵ | 8.8 × 10⁵ | 8.8 × 10⁵ | 1.0 × 10⁶ | 9.0 × 10⁵ | 8.9 × 10⁵ | 8.7 × 10⁵ | 8.8 × 10⁵ | 8.8 × 10⁵ |

The abbreviations in Table 1 stand for the following substances.
MYC1 pro: the promoter of the gene encoding MYC1 (MYC1 transcription factor)
35S: cauliflower mosaic virus 35S promoter
FPS: farnesyl diphosphate synthase
GGPS: geranylgeranyl diphosphate synthase
HMGR1: 3-hydroxy-3-methylglutaryl CoA reductase 1
IPI: isopentenyl diphosphate isomerase
CPT1: cis-prenyltransferase 1
SRPP: Small Rubber Particle Protein
REF: Rubber Elongation Factor

From the results shown in Table 1, it was demonstrated that the amount of polyisoprenoid accumulated in the plant was increased in the transgenic plants into which had been introduced the base sequence in which the gene encoding the protein involved in polyisoprenoid biosynthesis was functionally linked to the promoter of the gene encoding MYC1 transcription factor (Examples 1 to 7), as compared to the wild type (non-recombinant) and the transgenic plants obtained by using the cauliflower mosaic virus 35S promoter in place of the promoter of the gene encoding MYC1.

Further, it was also demonstrated that the weight average molecular weight of polyisoprenoid accumulated in the plant was increased particularly in the transgenic plants into which had been introduced the gene encoding geranylgeranyl diphosphate synthase, 3-hydroxy-3-methylglutaryl CoA reductase, cis-prenyltransferase, or Small Rubber Particle Protein as the protein involved in polyisoprenoid biosynthesis (Examples 2, 3, 5 and 6), as compared to the wild type (non-recombinant) and the transgenic plants obtained by using the cauliflower mosaic virus 35S promoter in place of the promoter of the gene encoding MYC1.

### (SEQUENCE LISTING FREE TEXT)

SEQ ID NO: 1: base sequence of promoter of gene encoding MYC1 transcription factor derived from para rubber tree
SEQ ID NO: 2: base sequence of gene encoding farnesyl diphosphate synthase derived from para rubber tree
SEQ ID NO: 3: amino acid sequence of farnesyl diphosphate synthase derived from para rubber tree
SEQ ID NO: 4: base sequence of gene encoding geranylgeranyl diphosphate synthase derived from para rubber tree
SEQ ID NO: 5: amino acid sequence of geranylgeranyl diphosphate synthase derived from para rubber tree
SEQ ID NO: 6: base sequence of gene encoding 3-hydroxy-3-methylglutaryl CoA reductase 1 derived from para rubber tree
SEQ ID NO: 7: base sequence of gene encoding 3-hydroxy-3-methylglutaryl CoA reductase 3 derived from para rubber tree
SEQ ID NO: 8: base sequence of gene encoding 3-hydroxy-3-methylglutaryl CoA reductase 4 derived from para rubber tree
SEQ ID NO: 9: base sequence of gene encoding 3-hydroxy-3-methylglutaryl CoA reductase 5 derived from para rubber tree
SEQ ID NO: 10: amino acid sequence of 3-hydroxy-3-methylglutaryl CoA reductase 1 derived from para rubber tree
SEQ ID NO: 11: amino acid sequence of 3-hydroxy-3-methylglutaryl CoA reductase 3 derived from para rubber tree
SEQ ID NO: 12: amino acid sequence of 3-hydroxy-3-methylglutaryl CoA reductase 4 derived from para rubber tree
SEQ ID NO: 13: amino acid sequence of 3-hydroxy-3-methylglutaryl CoA reductase 5 derived from para rubber tree
SEQ ID NO: 14: base sequence of gene encoding isopentenyl diphosphate isomerase derived from para rubber tree
SEQ ID NO: 15: amino acid sequence of isopentenyl diphosphate isomerase derived from para rubber tree
SEQ ID NO: 16: base sequence of gene encoding cis-prenyltransferase 1 derived from para rubber tree
SEQ ID NO: 17: amino acid sequence of cis-prenyltransferase 1 derived from para rubber tree
SEQ ID NO: 18: base sequence of gene encoding Small Rubber Particle Protein derived from para rubber tree
SEQ ID NO: 19: amino acid sequence of Small Rubber Particle Protein derived from para rubber tree
SEQ ID NO: 20: Primer 1
SEQ ID NO: 21: Primer 2
SEQ ID NO: 22: Primer 3
SEQ ID NO: 23: Primer 4
SEQ ID NO: 24: Primer 5
SEQ ID NO: 25: Primer 6
SEQ ID NO: 26: Primer 7
SEQ ID NO: 27: Primer 8
SEQ ID NO: 28: Primer 9
SEQ ID NO: 29: Primer 10
SEQ ID NO: 30: Primer 11
SEQ ID NO: 31: Primer 12
SEQ ID NO: 32: Primer 13
SEQ ID NO: 33: Primer 14
SEQ ID NO: 34: Primer 15
SEQ ID NO: 35: Primer 16
SEQ ID NO: 36: Primer 17
SEQ ID NO: 37: Primer 18
SEQ ID NO: 38: Primer 19
SEQ ID NO: 39: Primer 20
SEQ ID NO: 40: Primer 21
SEQ ID NO: 41: Primer 22
SEQ ID NO: 42: Primer 23
SEQ ID NO: 43: Primer 24
SEQ ID NO: 44: Primer 25
SEQ ID NO: 45: Primer 26
SEQ ID NO: 46: Primer 27
SEQ ID NO: 47: Primer 28
SEQ ID NO: 48: Primer 29
SEQ ID NO: 49: Primer 30
SEQ ID NO: 50: Primer 31
SEQ ID NO: 51: Primer 32
SEQ ID NO: 52: Primer 33
SEQ ID NO: 53: Primer 34
SEQ ID NO: 54: Primer 35
SEQ ID NO: 55: Primer 36
SEQ ID NO: 56: Primer 37
SEQ ID NO: 57: Primer 38
SEQ ID NO: 58: Primer 39
SEQ ID NO: 59: Primer 40
SEQ ID NO: 60: base sequence of gene encoding Rubber Elongation Factor derived from para rubber tree
SEQ ID NO: 61: amino acid sequence of Rubber Elongation Factor derived from para rubber tree
SEQ ID NO: 62: Primer 41
SEQ ID NO: 63: Primer 42
SEQ ID NO: 64: Primer 43
SEQ ID NO: 65: Primer 44
SEQ ID NO: 66: base sequence of gene encoding cis-prenyltransferase 2 derived from para rubber tree
SEQ ID NO: 67: amino acid sequence of cis-prenyltransferase 2 derived from para rubber tree
SEQ ID NO: 68: Primer 45
SEQ ID NO: 69: Primer 46
SEQ ID NO: 70: Primer 47
SEQ ID NO: 71: Primer 48

## Claims

1. A vector, comprising:
a MYC1 transcription factor encoding gene promoter; and
a gene, the gene being functionally linked to the promoter, wherein the gene is at least one gene selected from the group consisting of a gene encoding farnesyl diphosphate synthase, a gene encoding geranylgeranyl diphosphate synthase, a gene encoding 3-hydroxy-3-methylglutaryl CoA reductase, a gene encoding isopentenyl diphosphate isomerase, a gene encoding cis-prenyltransferase, a gene encoding Small Rubber Particle Protein, and a gene encoding Rubber Elongation Factor,
wherein the MYC1 transcription factor encoding gene promoter comprises any one of the following DNAs:
[A1] a DNA comprising the base sequence of base numbers 1 to 1610 represented by SEQ ID NO: 1;
[A2] a DNA hybridizing to a DNA comprising a base sequence complementary to the base sequence of base numbers 1 to 1610 represented by SEQ ID NO: 1 under stringent conditions, and having a promoter activity for laticifer-specific gene expression; and
[A3] a DNA comprising a base sequence having 80% or more sequence identity with the base sequence of base numbers 1 to 1610 represented by SEQ ID NO: 1, and having a promoter activity for laticifer-specific gene expression.

2. The vector according to claim 1, wherein the gene encoding farnesyl diphosphate synthase comprises any one of the following DNAs:
[B1] a DNA comprising the base sequence of base numbers 1 to 1029 represented by SEQ ID NO: 2;
[B2] a DNA hybridizing to a DNA comprising a base sequence complementary to the base sequence of base numbers 1 to 1029 represented by SEQ ID NO: 2 under stringent conditions, and encoding a protein having an enzyme activity that catalyzes a reaction using isopentenyl diphosphate and dimethylallyl diphosphate as substrates or a reaction using isopentenyl diphosphate and geranyl diphosphate as substrates; and
[B3] a DNA comprising a base sequence having 80% or more sequence identity with the base sequence of base numbers 1 to 1029 represented by SEQ ID NO: 2, and encoding a protein having an enzyme activity that catalyzes a reaction using isopentenyl diphosphate and dimethylallyl diphosphate as substrates or a reaction using isopentenyl diphosphate and geranyl diphosphate as substrates.

3. The vector according to claim 1, wherein the gene encoding geranylgeranyl diphosphate synthase comprises any one of the following DNAs:
[C1] a DNA comprising the base sequence of base numbers 1 to 1113 represented by SEQ ID NO: 4;
[C2] a DNA hybridizing to a DNA comprising a base sequence complementary to the base sequence of base numbers 1 to 1113 represented by SEQ ID NO: 4 under stringent conditions, and encoding a protein having an enzyme activity that catalyzes a reaction using isopentenyl diphosphate and dimethylallyl diphosphate as substrates, a reaction using isopentenyl diphosphate and geranyl diphosphate as substrates, or a reaction using isopentenyl diphosphate and farnesyl diphosphate as substrates; and
[C3] a DNA comprising a base sequence having 80% or more sequence identity with the base sequence of base numbers 1 to 1113 represented by SEQ ID NO: 4, and encoding a protein having an enzyme activity that catalyzes a reaction using isopentenyl diphosphate and dimethylallyl diphosphate as substrates, a reaction using isopentenyl diphosphate and geranyl diphosphate as substrates, or a reaction using isopentenyl diphosphate and farnesyl diphosphate as substrates.

4. The vector according to claim 1, wherein the gene encoding 3-hydroxy-3-methylglutaryl CoA reductase comprises any one of the following DNAs:
[D1] a DNA comprising the base sequence of base numbers 1 to 1728 represented by SEQ ID NO: 6;
[D2] a DNA hybridizing to a DNA comprising a base sequence complementary to the base sequence of base numbers 1 to 1728 represented by SEQ ID NO: 6 under stringent conditions, and encoding a protein having an enzyme activity that catalyzes a reaction of reduction of 3-hydroxy-3-methylglutaryl CoA;
[D3] a DNA comprising a base sequence having 80% or more sequence identity with the base sequence of base numbers 1 to 1728 represented by SEQ ID NO: 6, and encoding a protein having an enzyme activity that catalyzes a reaction of reduction of 3-hydroxy-3-methylglutaryl CoA;
[D4] a DNA comprising the base sequence of base numbers 1 to 1761 represented by SEQ ID NO: 7;
[D5] a DNA hybridizing to a DNA comprising a base sequence complementary to the base sequence of base numbers 1 to 1761 represented by SEQ ID NO: 7 under stringent conditions, and encoding a protein having an enzyme activity that catalyzes a reaction of reduction of 3-hydroxy-3-methylglutaryl CoA;
[D6] a DNA comprising a base sequence having 80% or more sequence identity with the base sequence of base numbers 1 to 1761 represented by SEQ ID NO: 7, and encoding a protein having an enzyme activity that catalyzes a reaction of reduction of 3-hydroxy-3-methylglutaryl CoA;
[D7] a DNA comprising the base sequence of base numbers 1 to 1821 represented by SEQ ID NO: 8;
[D8] a DNA hybridizing to a DNA comprising a base sequence complementary to the base sequence of base numbers 1 to 1821 represented by SEQ ID NO: 8 under stringent conditions, and encoding a protein having an enzyme activity that catalyzes a reaction of reduction of 3-hydroxy-3-methylglutaryl CoA;
[D9] a DNA comprising a base sequence having 80% or more sequence identity with the base sequence of base numbers 1 to 1821 represented by SEQ ID NO: 8, and encoding a protein having an enzyme activity that catalyzes a reaction of reduction of 3-hydroxy-3-methylglutaryl CoA;
[D10] a DNA comprising the base sequence of base numbers 1 to 1581 represented by SEQ ID NO: 9;
[D11] a DNA hybridizing to a DNA comprising a base sequence complementary to the base sequence of base numbers 1 to 1581 represented by SEQ ID NO: 9 under stringent conditions, and encoding a protein having an enzyme activity that catalyzes a reaction of reduction of 3-hydroxy-3-methylglutaryl CoA; and
[D12] a DNA comprising a base sequence having 80% or more sequence identity with the base sequence of base numbers 1 to 1581 represented by SEQ ID NO: 9, and encoding a protein having an enzyme activity that catalyzes a reaction of reduction of 3-hydroxy-3-methylglutaryl CoA.

5. The vector according to claim 1, wherein the gene encoding isopentenyl diphosphate isomerase comprises any one of the following DNAs:
[E1] a DNA comprising the base sequence of base numbers 1 to 705 represented by SEQ ID NO: 14;
[E2] a DNA hybridizing to a DNA comprising a base sequence complementary to the base sequence of base numbers 1 to 705 represented by SEQ ID NO: 14 under stringent conditions, and encoding a protein having an enzyme activity that catalyzes a reaction of isomerization of isopentenyl diphosphate or dimethylallyl diphosphate; and
[E3] a DNA comprising a base sequence having 80% or more sequence identity with the base sequence of base numbers 1 to 705 represented by SEQ ID NO: 14, and encoding a protein having an enzyme activity that catalyzes a reaction of isomerization of isopentenyl diphosphate or dimethylallyl diphosphate.

6. The vector according to claim 1, wherein the gene encoding cis-prenyltransferase comprises any one of the following DNAs:
[F1] a DNA comprising the base sequence of base numbers 1 to 873 represented by SEQ ID NO: 16;
[F2] a DNA hybridizing to a DNA comprising a base sequence complementary to the base sequence of base numbers 1 to 873 represented by SEQ ID NO: 16 under stringent conditions, and encoding a protein having an enzyme activity that catalyzes a reaction of cis-chain elongation of isoprenoid compounds;
[F3] a DNA comprising a base sequence having 80% or more sequence identity with the base sequence of base numbers 1 to 873 represented by SEQ ID NO: 16, and encoding a protein having an enzyme activity that catalyzes a reaction of cis-chain elongation of isoprenoid compounds;
[F4] a DNA comprising the base sequence of base numbers 1 to 855 represented by SEQ ID NO: 66;
[F5] a DNA hybridizing to a DNA comprising a base sequence complementary to the base sequence of base numbers 1 to 855 represented by SEQ ID NO: 66 under stringent conditions, and encoding a protein having an enzyme activity that catalyzes a reaction of cis-chain elongation of isoprenoid compounds; and
[F6] a DNA comprising a base sequence having 80% or more sequence identity with the base sequence of base numbers 1 to 855 represented by SEQ ID NO: 66, and encoding a protein having an enzyme activity that catalyzes a reaction of cis-chain elongation of isoprenoid compounds.

7. The vector according to claim 1, wherein the gene encoding Small Rubber Particle Protein comprises any one of the following DNAs:
[G1] a DNA comprising the base sequence of base numbers 1 to 615 represented by SEQ ID NO: 18;
[G2] a DNA hybridizing to a DNA comprising a base sequence complementary to the base sequence of base numbers 1 to 615 represented by SEQ ID NO: 18 under stringent conditions, and encoding a rubber particle-associated protein which is associated with rubber particles in latex; and
[G3] a DNA comprising a base sequence having 80% or more sequence identity with the base sequence of base numbers 1 to 615 represented by SEQ ID NO: 18, and encoding a rubber particle-associated protein which is associated with rubber particles in latex.

8. The vector according to claim 1, wherein the gene encoding Rubber Elongation Factor comprises any one of the following DNAs:
[H1] a DNA comprising the base sequence of base numbers 1 to 417 represented by SEQ ID NO: 60;
[H2] a DNA hybridizing to a DNA comprising a base sequence complementary to the base sequence of base numbers 1 to 417 represented by SEQ ID NO: 60 under stringent conditions, and encoding a rubber particle-associated protein which is associated with rubber particles in latex; and
[H3] a DNA comprising a base sequence having 80% or more sequence identity with the base sequence of base numbers 1 to 417 represented by SEQ ID NO: 60, and encoding a rubber particle-associated protein which is associated with rubber particles in latex.

9. A transgenic plant into which the vector according to any one of claims 1 to 8 has been introduced.

10. A method for producing polyisoprenoid in a plant by introducing the vector according to any one of claims 1 to 8 into the plant, wherein the plant is one of the genera *Hevea, Sonchus, Solidago, Helianthus, Taraxacum, Ficus, Parthenium, Lactuca serriola* (lettuce) and Indian banyan.

## Patentansprüche

1. Vektor enthaltend:
einen MYC1-Transkriptionsfaktor kodierenden Genpromoter sowie
ein Gen, wobei das Gen funktionell mit dem Promoter verbunden ist, wobei das Gen wenigstens ein Gen ist, welches aus der Gruppe ausgewählt ist, welche besteht aus einem Gen kodierend Farnesyldiphosphatsynthase, einem Gen kodierend Geranylgernyldiphosphatsynthase, einem Gen kodierend 3-Hydroxy-3-methylglutaryl-CoA-Reduktase, einem Gen kodierend Isopentenyldiphosphatisomerase, einem Gen kodierend cis-Prenyltransferase, einem Gen kodierend Kleines-Kautschuk-Partikel-Protein und einem Gen kodierend Kautschukverlängerungsfaktor,
wobei der MYC1-Transkriptionsfaktor kodierendes Gen-Promoter eine der folgenden DNSen aufweist:
[A1] eine DNS enthaltend eine Basensequenz mit den Basennummern 1 bis 1610 wiedergegeben durch SEQ ID NO: 1,
[A2] eine DNS hybridisierend an eine DNS enthaltend eine Basensequenz komplementär zu der Basensequenz der Basennummern 1 bis 1610 wiedergegeben durch SEQ ID NO. 1 unter stringenten Bedingungen und mit einer Promoteraktivität für Laticifer-spezifische Genexpression und
[A3] eine DNS enthaltend eine Basensequenz mit 80 % oder mehr Sequenzidentität mit einer Basensequenz der Basennummern 1 bis 1610 wiedergegen durch SEQ ID NO: 1 und mit einer Promotoraktivität für Laticifer-spezifische Genexpression.

2. Vektor nach Anspruch 1, wobei das Farnesyldiphosphatsynthase kodierende Gen eine der folgenden DNSen enthält:
[B1] eine DNS enthaltend die Basensequenz mit den Basennummern 1 bis 1029 wiedergegeben durch SEQ ID NO: 2,
[B2] eine DNS hybridisierend an eine DNS enthaltend eine Basensequenz komplementär zu der Basensequenz mit den Basennummern 1 bis 1029 wiedergegen durch SEQ ID NO: 2 unter stringenten Bedingungen und kodierend ein Protein mit einer Enzymaktivität, welche eine Reaktion unter Verwendung von Isopentenyldiphosphat und Dimethylallyldiphosphat als Substrate oder eine Reaktion unter Verwendung von Isopentenyldiphosphat und Geranyldiphosphat als Substrat katalysiert, sowie
[B3] eine DNS enthaltend eine Basensequenz mit 80 % oder mehr Sequenzidentität mit der Basensequenz mit den Basennummern 1 bis 1029 wiedergegeben durch SEQ ID NO: 2 und kodierend ein Protein mit einer Enzymaktivität, welche eine Reaktion unter Verwendung von Isopentenyldiphosphat und Dimethylallyldisphosphat als Substrate oder eine Reaktion unter Verwendung von Isopentenyldisphosphat und Geranyldiphosphat als Substrate analysiert.

3. Vektor nach Anspruch 1, wobei das Geranylgeranyldiphosphatsynthase kodierende Gen eine oder mehrere der folgenden DNSen enthält:
[C1] eine DNS enthaltend eine Basensequenz mit den Basennummern 1 bis 1113 wiedergegeben durch SEQ ID NO: 4,
[C2] eine DNS hybridisierend an eine DNS enthaltend eine Basensequenz komplementär zu der Basensequenz mit den Basennummern 1 bis 1113 wiedergegeben durch SEQ ID NO: 4 unter stringenten Bedingungen und kodierend ein Protein mit einer Enzymaktivität, welche eine Reaktion unter Verwendung von Isopentenyldiphosphat und Dimethylallyldiphosphat als Substrate, eine Reaktion unter Verwendung von Isopentenyldiphosphat und Geranyldiphosphat als Substrate oder eine Reaktion unter Verwendung von Isopentenyldiphosphat und Farnesyldiphosphat als Substrate katalysiert, sowie
[C3] eine DNS enthaltend eine Basensequenz mit 80 % oder mehr Sequenzidentität mit der Basensequenz mit den Basennummern 1 bis 1113 wiedergegeben durch SEQ ID NO: 4 und kodierend ein Protein mit einer Enzymnaktivität, welche eine Reaktion unter Verwendung von Isopentenyldiphosphat und Dimethylallyldiphosphat als Substrate, eine Reaktion unter Verwendung von Isopentenyldiphosphat und Geranyldiphosphat als Substrate oder eine Reaktion unter Verwendung von Isopentenyldiphosphat und Farnesyldiphosphat als Substrate katalysiert.

4. Vektor nach Anspruch 1, wobei das 3-Hydroxy-3-methylglutaryl-CoA-Reduktase kodierende Gen eine oder mehrere der folgenden DNSen enthält:
[D1] eine DNS enthaltend eine Basensequenz mit den Basennummern 1 bis 1728 wiedergegeben durch SEQ ID NO: 6,
[D2] eine DNS hybridisierend an eine DNS enthaltend eine Basensequenz komplementär zu der Basensequenz mit den Basennummern 1 bis 1728 wiedergegeben durch SEQ ID NO. 6 unter stringenten Bedingungen und kodierend ein Protein mit einer Enzymaktivität, die eine Reaktion der Reduktion von 3-Hydroxy-3-methylglutaryl-CoA katalysiert,
[D3] eine DNS enthaltend eine Basensequenz mit 80 % oder mehr Sequenzidentität mit der Basensequenz mit den Basennummern 1 bis 1728 wiedergegeben durch SEQ ID NO: 6 und kodierend ein Protein mit einer Enzymaktivität, welche eine Reaktion der Reduktion von 3-Hydroxy-3-methylglutaryl-CoA katalysiert,
[D4] eine DNS enthaltend eine Basensequenz mit den Basennummern 1 bis 1761 widergegeben durch SEQ ID NO. 7,
[D5] eine DNS hybridisierend an eine DNS enthaltend eine Basensequenz komplementär zu der Basensequenz mit den Basennummern 1 bis 1761 wiedergegeben durch SEQ ID NO:7 unter stringenten Bedingungen und kodierend ein Protein mit einer Enzymaktivität, welche eine Reaktion der Reduktion von 3-Hydroxy-3-methylglutaryl-CoA katalysiert,
[D6] eine DNS enthaltend eine Basensequenz mit 80 % oder mehr Sequenzidentität mit der Basensequenz mit den Basennummern 1 bis 1761 wiedergegeben durch SEQ ID NO. 7 und kodierend ein Protein mit einer Enzymaktivität, welche eine Reaktion der Reduktion von 3-Hydroxy-3-methylglutaryl-CoA katalysiert,
[D7] eine DNS enthaltend eine Basensequenz mit den Basennummern 1 bis 1821 wiedergegeben durch SEQ ID NO: 8,
[D8] eine DNS hybridisierend an eine DNS enthaltend eine Basensequenz komplementär zu der Basensequenz mit den Basennummern 1 bis 1821 wiedergegeben durch SEQ ID NO. 8 unter stringenten Bedingungen und kodierend ein Protein mit einer Enzymaktivität, welche eine Reaktion der Reduktion von 3-Hydroxy-3-methylglutaryl-CoA katalysiert,
[D9] eine DNS enthaltend eine Basensequenz mit 80 % oder mehr Sequenzidentität mit einer Basensequenz mit den Basennummern 1 bis 1821 wiedergegeben durch SEQ ID NO: 8 und kodierend ein Protein mit einer Enzymaktivität, welche eine Reaktion der Reduktion von 3-Hydroxy-3-methylglutaryl-CoA katalysiert,
[D10] eine DNS enthaltend eine Basensequenz mit den Basennummern 1 bis 1581 wiedergegeben durch SEQ ID NO: 9,
[D11] eine DNS hybridisierend an eine DNS enthaltend eine Basensequenz komplementär zu der Basensequenz mit den Basennummern 1 bis 1581 wiedergegeben durch SEQ ID NO: 9 unter stringenten Bedingungen und kodierend ein Protein mit einer Enzymaktivität, welche eine Reaktion der Reduktion von 3-Hydroxy-3-methylglutaryl-CoA katalysiert, sowie
[D12] eine DNS enthaltend eine Basensequenz mit 80 % oder mehr Sequenzidentität mit der Basensequenz mit den Basennummern 1 bis 1581 wiedergegeben durch SEQ ID NO: 9 und kodierend ein Protein mit einer Enzymaktivität, welche eine Reaktion der Reduktion von 3-Hydroxy-3-methylglutaryl-CoA katalysiert.

5. Vektor nach Anspruch 1, wobei das Isopentenyldiphosphatisomerase kodierende Gen eine oder mehrere der folgenden DNSen enthält:
[E1] eine DNS enthaltend eine Basensequenz mit den Basennummern 1 bis 705 wiedergegeben durch SEQ ID NO: 14,
[E2] eine DNS hybridisierend an eine DNS enthaltend eine Basensequenz komplementär zu der Basensequenz mit den Basennummern 1 bis 705 wiedergegeben durch SEQ ID NO: 14 unter stringenten Bedingungen und kodierend ein Protein mit einer Enzymaktivität, welche eine Reaktion der Isomerisierung von Isopentenyldiphosphat oder Dimethylallyldiphosphat katalysiert, und
[E3] eine DNS enthaltend eine Basensequenz mit 80 % oder mehr Sequenzidentität mit der Basensequenz mit den Basennummern 1 bis 705 wiedergegeben durch SEQ ID NO: 14 und kodierend ein Protein mit einer Enzymaktivität, welche eine Reaktion der Isomerisation von Isopentenyldiphosphat oder Dimethylallyldiphosphat katalysiert.

6. Vektor nach Anspruch 1, wobei das cis-Prenyltransferase kodierende Gen eine oder mehrere der folgenden DNSen enthält:
[F1] eine DNS enthaltend eine Basensequenz mit den Basennummern 1 bis 873 wiedergegeben durch SEQ ID NO: 16,
[F2] eine DNS hybridisierend an eine DNS enthaltend eine Basensequenz komplementär zu der Basensequenz mit den Basennummern 1 bis 873 wiedergegeben durch SEQ ID NO: 16 unter stringenten Bedingungen und kodierend ein Protein mit einer Enzymaktivität, welche eine Reaktion von cis-Kettenverlängerung von Isoprenoidverbindungen katalysiert,
[F3] eine DNS enthaltend eine Basensequenz mit 80 % oder mehr Sequenzidentität mit einer Basensequenz mit den Basennummern 1 bis 873 wiedergegeben durch SEQ ID NO: 16 und kodierend ein Protein mit einer Enzymaktivität, welche eine Reaktion von cis-Kettenverlängerung von Isoprenoidverbindungen katalysiert,
[F4] eine DNS enthaltend eine Basensequenz mit den Basennummern 1 bis 855 wiedergegeben durch SEQ ID NO: 66,
[F5] eine DNS hybridisierend an eine DNS enthaltend eine Basensequenz komplementär zu der Basensequenz mit den Basennummern 1 bis 855 wiedergegeben durch SEQ ID NO: 66 unter stringenten Bedingungen und kodierend ein Protein mit einer Enzymaktivität, welche eine Reaktion der cis-Kettenverlängerung von Isoprenoidverbindungen katalysiert, und
[F6] eine DNS enthaltend eine Basensequenz mit 80 % oder mehr Sequenzidentität mit der Basensequenz mit den Basennummern 1 bis 855 widergegeben durch SEQ ID NO: 66 und kodierend ein Protein mit einer Enzymaktivität, welche eine Reaktion von cis-Kettenverlängerung von Isoprenoidverbindungen katalysiert.

7. Vektor nach Anspruch 1, wobei das Kleine-Kautschuk-Partikel-Protein kodierende Gen ein oder mehrere der folgenden DNSen enthält:
[G1] eine DNS enthaltend eine Basensequenz mit den Basennummern 1 bis 615 wiedergegeben durch SEQ ID NO: 18,
[G2] eine DNS hybridisierend an eine DNS enthaltend eine Basensequenz komplementär zu der Basensequenz mit den Basennummern 1 bis 615 wiedergegeben durch SEQ ID NO: 18 unter stringenten Bedingungen und kodierend ein Kautschuk-Partikel assoziiertes Protein, welches mit Kautschukpartikeln in Latex assoziiert ist, sowie
[G3] eine DNS enthaltend eine Basensequenz mit 80 % oder mehr Sequenzidentität mit der Basensequenz mit den Basennummern 1 bis 615 wiedergegeben durch SEQ ID NO: 18 und kodierend ein Kautschuk-Partikel assoziiertes Protein, welches mit Kautschukpartikeln in Latex assoziiert ist.

8. Vektor nach Anspruch 1, wobei das Kautschukverlängerungsfaktor kodierende Gen eine oder mehrere der folgenden DNSen enthält:
[H1] eine DNS enthaltend eine Basensequenz mit den Basennummern 1 bis 417 wiedergegeben durch SEQ ID NO. 60,
[H2] eine DNS hybridisierend an eine DNS enthaltend eine Basensequenz komplementär zu der Basensequenz mit den Basennummern 1 bis 417 wiedergegeben durch SEQ ID NO: 60 unter stringenten Bedingungen und kodierend ein Kautschukpartikel-assoziiertes Protein, welches mit Kautschukpartikeln in Latex assoziiert ist, sowie
[H3] eine DNS enthaltend eine Basensequenz mit 80 % oder mehr Sequenzidentität mit der Basensequenz mit den Basennummern 1 bis 417 wiedergegeben durch SEQ ID NO: 60 und kodierend ein Kautschukpartikel-assoziiertes Protein, welches mit Kautschukpartikeln in Latex assoziiert ist.

9. Transgene Pflanze, in welche ein Vektor nach einem der Ansprüche 1 bis 8 eingeführt ist.

10. Verfahren zum Herstellen von Polyisoprenoid in einer Pflanze durch Einführen eines Vektors nach einem der Ansprüche 1 bis 8 in eine Pflanze, wobei die Pflanze eine der Gattung *Hevea, Sonchus, Solidago, Helianthus, Taraxacum, Ficus, Parthenium, Lactuca serriola* (Salat) und indischer Banyan ist.

## Revendications

1. Vecteur comprenant :
un promoteur de gène codant pour le facteur de transcription MYC1 ; et
un gène, le gène étant fonctionnellement lié au promoteur, le gène étant au moins un gène choisi dans le groupe consistant en un gène codant pour la farnésyl diphosphate synthase, un gène codant pour la géranylgéranyl diphosphate synthase, un gène codant pour la 3-hydroxy-3-méthylglutaryl CoA réductase, un gène codant pour l'isopentényl diphosphate isomérase, un gène codant pour la cis-prényltransférase, un gène codant pour la protéine des petites particules de caoutchouc et un gène codant pour le facteur d'allongement de caoutchouc,
dans lequel le promoteur de gène codant pour le facteur de transcription MYC1 comprend l'un quelconque des ADN suivants :
[A1] un ADN comprenant la séquence de bases de numéros de bases 1 à 1610 représentée par SEQ ID NO:1 ;
[A2] un ADN s'hybridant à un ADN comprenant une séquence de bases complémentaire à la séquence de bases de numéros de bases 1 à 1610 représentée par SEQ ID NO:1 dans des conditions stringentes, et ayant une activité de promoteur pour l'expression d'un gène spécifique des laticifères ; et
[A3] un ADN comprenant une séquence de bases ayant au moins 80 % d'identité de séquence avec la séquence de bases de numéros de bases 1 à 1610 représentée par SEQ ID NO:1, et ayant une activité de promoteur pour l'expression d'un gène spécifique des laticifères.

2. Vecteur selon la revendication 1, dans lequel le gène codant pour la farnésyl diphosphate synthase comprend l'un quelconque des ADN suivants :
[B1] un ADN comprenant la séquence de bases de numéros de bases 1 à 1029 représentée par SEQ ID NO:2 ;
[B2] un ADN s'hybridant à un ADN comprenant une séquence de bases complémentaire à la séquence de bases de numéros de bases 1 à 1029 représentée par SEQ ID NO:2 dans des conditions stringentes, et codant pour une protéine ayant une activité enzymatique qui catalyse une réaction utilisant le diphosphate d'isopentényle et le diphosphate de diméthylallyle comme substrats ou une réaction utilisant le diphosphate d'isopentényle et le diphosphate de géranyle comme substrats ; et
[B3] un ADN comprenant une séquence de bases ayant au moins 80 % d'identité de séquence avec la séquence de bases de numéros de bases 1 à 1029 représentée par SEQ ID NO:2, et codant pour une protéine ayant une activité enzymatique qui catalyse une réaction utilisant le diphosphate d'isopentényle et le diphosphate de diméthylallyle comme substrats ou une réaction utilisant le diphosphate d'isopentényle et le diphosphate de géranyle comme substrats.

3. Vecteur selon la revendication 1, dans lequel le gène codant pour la géranylgéranyl diphosphate synthase comprend l'un quelconque des ADN suivants :
[C1] un ADN comprenant la séquence de bases de numéros de bases 1 à 1113 représentée par SEQ ID NO:4 ;
[C2] un ADN s'hybridant à un ADN comprenant une séquence de bases complémentaire à la séquence de bases de numéros de bases 1 à 1113 représentée par SEQ ID NO:4 dans des conditions stringentes, et codant pour une protéine ayant une activité enzymatique qui catalyse une réaction utilisant le diphosphate d'isopentényle et le diphosphate de diméthylallyle comme substrats, une réaction utilisant le diphosphate d'isopentényle et le diphosphate de géranyle comme substrats, ou une réaction utilisant le diphosphate d'isopentényle et le diphosphate de farnésyle comme substrats ; et
[C3] un ADN comprenant une séquence de bases ayant au moins 80 % d'identité de séquence avec la séquence de bases de numéros de bases 1 à 1113 représentée par SEQ ID NO:4, et codant pour une protéine ayant une activité enzymatique qui catalyse une réaction utilisant le diphosphate d'isopentényle et le diphosphate de diméthylallyle comme substrats, une réaction utilisant le diphosphate d'isopentényle et le diphosphate de géranyle comme substrats ; ou une réaction utilisant le diphosphate d'isopentényle et le le diphosphate de farnésyle comme substrats.

4. Vecteur selon la revendication 1, dans lequel le gène codant pour la 3-hydroxy-3-méthylglutaryl CoA réductase comprend l'un quelconque des ADN suivants :
[D1] un ADN comprenant la séquence de bases de numéros de bases 1 à 1728 représentée par SEQ ID NO:6 ;
[D2] un ADN s'hybridant à un ADN comprenant une séquence de bases complémentaire à la séquence de bases de numéros de bases 1 à 1728 représentée par SEQ ID NO:6 dans des conditions stringentes, et codant pour une protéine ayant une activité enzymatique qui catalyse une réaction de réduction de 3-hydroxy-3-méthylglutaryl CoA ;
[D3] un ADN comprenant une séquence de bases ayant au moins 80 % d'identité de séquence avec la séquence de bases de numéros de bases 1 à 1728 représentée par SEQ ID NO:6, et codant pour une protéine ayant une activité enzymatique qui catalyse une réaction de réduction de la 3-hydroxy-3-méthylglutaryl CoA ;
[D4] un ADN comprenant la séquence de bases de numéros de bases 1 à 1761 représentée par SEQ ID NO:7 ;
[D5] un ADN s'hybridant à un ADN comprenant une séquence de bases complémentaire à la séquence de bases de numéros de bases 1 à 1761 représentée par SEQ ID NO:7 dans des conditions stringentes, et codant pour une protéine ayant une activité enzymatique qui catalyse une réaction de réduction de 3-hydroxy-3-méthylglutaryl CoA ;
[D6] un ADN comprenant une séquence de bases ayant au moins 80 % d'identité de séquence avec la séquence de bases de numéros de bases 1 à 1761 représentée par SEQ ID NO:7, et codant pour une protéine ayant une activité enzymatique qui catalyse une réaction de réduction de 3-hydroxy-3-méthylglutaryl CoA ;
[D7] un ADN comprenant la séquence de bases de numéros de bases 1 à 1821 représentée par SEQ ID NO:8 ;
[D8] un ADN s'hybridant à un ADN comprenant une séquence de bases complémentaire à la séquence à bases de numéros de bases 1 à 1821 représentée par SEQ ID NO:8 dans des conditions stringentes, et codant pour une protéine ayant une activité enzymatique qui catalyse une réaction de réduction de 3-hydroxy-3-méthylglutaryl CoA ;
[D9] un ADN comprenant une séquence de bases ayant au moins 80 % d'identité de séquence avec la séquence de bases de numéros de bases 1 à 1821 représentée par SEQ ID NO:8, et codant pour une protéine ayant une activité enzymatique qui catalyse une réaction de réduction de 3-hydroxy-3-méthylglutaryl CoA ;
[D10] un ADN comprenant la séquence de bases de numéros de bases 1 à 1581 représentée par SEQ ID NO:9 ;
[D11] un ADN s'hybridant à un ADN comprenant une séquence de bases complémentaire à la séquence de bases de numéros de bases 1 à 1581 représentée par SEQ ID NO:9 dans des conditions stringentes, et codant pour une protéine ayant une activité enzymatique qui catalyse une réaction de réduction de 3-hydroxy-3-méthylglutaryl CoA ; et
[D12] un ADN comprenant une séquences de bases ayant au moins 80 % d'identité de séquence avec la séquence de bases de numéros de bases 1 à 1581 représentée par SEQ ID NO:9, et codant pour une protéine ayant une activité enzymatique qui catalyse une réaction de réduction de 3-hydroxy-3-méthylglutaryl CoA.

5. Vecteur selon la revendication 1, dans lequel le gène codant pour l'isopentényl diphosphate isomérase comprend l'un quelconque des ADN suivants :
[E1] un ADN comprenant la séquence de bases de numéros de bases 1 à 705 représentée par SEQ ID NO:14 ;
[E2] un ADN s'hybridant à un ADN comprenant une séquence de bases complémentaire à la séquence de bases de numéros de bases 1 à 705 représentée par SEQ ID NO:14 dans des conditions stringentes, et codant pour une protéine ayant une activité enzymatique qui catalyse une réaction d'isomérisation du diphosphate d'isopentényle ou du diphosphate de diméthylallyle ; et
[E3] un ADN comprenant une séquence de bases ayant au moins 80 % d'identité de séquence avec la séquence de bases de numéros de bases 1 à 705 représentée par SEQ ID NO:14, et codant pour une protéine ayant une activité enzymatique qui catalyse une réaction d'isomérisation du diphosphate d'isopentényle ou du diphosphate de diméthylallyle.

6. Vecteur selon la revendication 1, dans lequel le gène codant pour la cis-prényltransférase comprend l'un quelconque des ADN suivants :
[F1] un ADN comprenant la séquence de bases de numéros de bases 1 à 873 représentée par SEQ ID NO:16 ;
[F2] un ADN s'hybridant à un ADN comprenant une séquence de bases complémentaire à la séquence de bases de numéros de bases 1 à 873 représentée par SEQ ID NO:16 dans des conditions stringentes, et codant pour une protéine ayant une activité enzymatique qui catalyse une réaction d'un allongement de chaîne cis de composés isoprénoïdes ;
[F3] un ADN comprenant une séquence de bases ayant au moins 80 % d'identité de séquence avec la séquence de bases de numéros de bases 1 à 873 représentée par SEQ ID NO:16, et codant pour une protéine ayant une activité enzymatique qui catalyse une réaction d'allongement de chaîne cis de composés isoprénoïdes ;
[F4] un ADN comprenant la séquence de bases de numéros de bases 1 à 855 représentée par SEQ ID NO:66 ;
[F5] un ADN s'hybridant à un ADN comprenant une séquence de bases complémentaire à la séquence de bases de numéros de bases 1 à 855 représentée par SEQ ID NO:66 dans des conditions stringentes, et codant pour une protéine ayant une activité enzymatique qui catalyse une réaction d'allongement de chaîne cis de composés isoprénoïdes ; et
[F6] un ADN comprenant une séquence de bases ayant au moins 80 % d'identité de séquence avec la séquence de bases de numéros de bases 1 à 855 représentée par SEQ ID NO:66, et codant pour une protéine ayant une activité enzymatique qui catalyse une réaction d'allongement de chaîne cis de composés isoprénoïdes.

7. Vecteur selon la revendication 1, dans lequel le gène codant pour la protéine des petites particules de caoutchouc comprend l'un quelconque des ADN suivants :
[G1] un ADN comprenant la séquence de bases de numéros de bases 1 à 615 représentée par SEQ ID NO:18 ;
[G2] un ADN s'hybridant à un ADN comprenant une séquence de bases complémentaire à la séquence de bases de numéros de bases 1 à 615 représentée par SEQ ID NO:18 dans des conditions stringentes, et codant pour une protéine associée aux particules de caoutchouc qui est associée aux particules de caoutchouc dans un latex ; et
[G3] un ADN comprenant une séquence de bases ayant au moins 80 % d'identité de séquence avec la séquence de bases de numéros de bases 1 à 615 représentée par SEQ ID NO:18, et codant pour une protéine associée aux particules de caoutchouc qui est associée aux particules de caoutchouc dans un latex.

8. Vecteur selon la revendication 1, dans lequel le gène codant pour le facteur d'allongement de caoutchouc comprend l'un quelconque des ADN suivants :
[H1] un ADN comprenant la séquence de bases de numéros de bases 1 à 417 représentée par SEQ ID NO:60 ;
[H2] un ADN s'hybridant à un ADN comprenant une séquence de bases complémentaire à la séquence de bases de numéros de bases 1 à 417 représentée par SEQ ID NO:60 dans des conditions stringentes, et codant pour une protéine associée aux particules de caoutchouc qui est associée aux particules de caoutchouc dans un latex ; et
[H3] un ADN comprenant une séquence de bases ayant au moins 80 % d'identité de séquence avec la séquence de bases de numéros de bases 1 à 417 représentée par SEQ ID NO:60, et codant pour une protéine associée aux particules de caoutchouc qui est associée aux particules de caoutchouc dans un latex.

9. Plante transgénique dans laquelle le vecteur selon l'une quelconque des revendications 1 à 8 a été introduit.

10. Procédé de production d'un polyisoprénoïde dans une plante par introduction du vecteur selon l'une quelconque des revendications 1 à 8 dans la plante, la plante étant l'une des genres *Hevea, Sonchus, Solidago, Helianthus, Taraxacum, Ficus, Parthenium, Lactuca serriola* (laitue) et banian de l'Inde.


### SEQUENCE LISTING

<110> SUMITOMO RUBBER INDUSTRIES, LTD.
<120> VECTOR COMPRISING SPECIFIC PROMOTER AND GENE ENCODING SPECIFIC
   PROTEIN, TRANSGENIC PLANT INTO WHICH THE VECTOR HAS BEEN INTRODUCED, AND
   METHOD FOR IMPROVING POLYISOPRENOID PRODUCTION BY INTRODUCING THE VECTOR INTO PLANT
<130> SR1877
<160> 71
<170> PatentIn version 3.5
<210> 1
   <211> 1610
   <212> DNA
   <213> Hevea brasiliensis
<400> 1
   taccagctat gattcacctc tagtgatatt gattttatta aaaattaagg caatgatgaa      60
   tttctttttg aaaaaatcat ttaagattca agagggagat acagtgatgt cgttattttt     120
   taattttatg taagtaaaat tcgtcaatca ttcatcaaag ttaactgcgt gctaaataat     180
   ttaaaaatta ttgtttttta tttttgaaaa ttataataaa attcatttgt tttaataatt     240
   aggttcttag tttataattt ttttatttta aatttaagta tttatgatta attctgattt     300
   aatttctgat aattttaaaa ttaaaattat ttgattctta caaattttta acaatcattg     360
   ttttagacca atctcaaagt aaatgtttca cacgttacaa gggtgcaaaa tgattggacg     420
   gtgacagaaa cagtaaatat tatgaattta attgttgcca tgggctgcta aattcaaagg     480
   gtcatcatta cgtgattctc gatttacgaa aaaaaaaaag tattttcata tatatatgta     540
   tatacacaca cacatcatgc aaaatattta gttaataatt ttccaaaatt aaaacttttt     600
   tttatagcat acaattaaaa tgttaaattc aattaaaaaa gtgaaaataa aaatataatt     660
   ttttacataa aaaatataaa ttttatataa ttattagtga gaatatatat tactagattt     720
   aaaatatact gaataaccac tcgcttttta attggttata gtgattaatt aagaattttt     780
   tttatctaaa tttattaagt gatccaacaa attttgaact attatataag tttataaaat     840
   tttgattctc cattctacat ttttaaattt tcatttttta tattatgaaa atatatacat     900
   aaaaaaatta attaaactag agttaattgt cagaatgaat ctctagtaaa attttctctg     960
   attaaaaaat aaatttcata aattatccca ctaaactttt gtcatgtgat catgtcccca    1020
   ataaaatttg attttattat aattggcaac tcgatgtcta acctgcgagt aattatacac    1080
   catccccatg atacctccat gatttcaagt gtcaaagtat gttttaatga gaaattatta    1140
   ggttaactca atgtatatac attatttttt ataattatgt gaaattaatt ctcataatta    1200
   tataggacac atacttcgtc cacttatttt ttagaaaaaa agcattattt tttagcactt    1260
   tcaatgtaac taataattaa cggtttttaa catgtaagta taattgaata ttataaaacg 1320
   cattaataca tatatatgca tgattcttgt taatttacca ttctacgtag aatattccat 1380
   atagaatcaa tgctttatta tataataatt tctgctacat aataagaggc tttcatttcc 1440
   tttgtcttta aataacccca agtctcactt gtaaaccaac gtcgctcatt tatccctctt 1500
   accctgtccc tctccaactc tcaaactttc tggaattcca tagattgtgg aaactctcta 1560
   gctaaaccaa aaaacagaaa agaacataca aaattgaaat actaaggtgc 1610
<210> 2
   <211> 1029
   <212> DNA
   <213> Hevea brasiliensis
<400> 2
   atggcggatc tgaagtcaac tttcttgaag gtctactctg tcctcaagca ggagctcctt 60
   gaggatccgg ctttcgaatg gacaccagat tcccgtcaat gggttgagcg gatgttggac 120
   tacaatgtgc ctggagggaa gctgaatagg gggctttctg taattgacag ctacaaattg 180
   ttgaaagaag gacaggaatt aacagaagaa gagatctttc ttgcaagtgc tcttggttgg 240
   tgtattgaat ggcttcaagc ctattttctt gtacttgatg acattatgga tagctctcat 300
   acacgacgtg gtcagccttg ctggtttagg gtgcccaagg ttggtctgat tgcagcaaat 360
   gatgggattt tgcttcgcaa tcacattccc aggattctta aaaagcactt ccgagggaag 420
   gcatactatg tagatcttct agatttgttt aatgaggtgg agtttcaaac agcctcagga 480
   cagatgatag atctaattac aacacttgaa ggagaaaagg atttatccaa atacactttg 540
   tcactccacc ggagaattgt tcagtacaaa actgcctact actcatttta ccttcctgtt 600
   gcttgtgcat tgctcatagc gggtgagaat ctggacaatc atattgttgt aaaagacatt 660
   cttgttcaga tgggaatcta cttccaagta caggatgatt atttggattg ctttggtgat 720
   cccgagacaa ttggtaagat aggaacagat atagaagatt ttaagtgttc atggttggtc 780
   gtgaaggctt tagaactttg caatgaagaa caaaagaaag tgttatatga gcactatggg 840
   aaagctgacc cagccagtgt agcaaaggtg aaggtccttt ataatgagct gaagcttcag 900
   ggggtattta cggagtatga gaatgaaagc tataagaaac tagtaacctc tattgaagct 960
   catcctagca agccggtgca agcagtgttg aagtcctttt tggccaaaat ttacaagaga 1020
   cagaaataa 1029
<210> 3
   <211> 342
   <212> PRT
   <213> Hevea brasiliensis
<400> 3
   Met Ala Asp Leu Lys Ser Thr Phe Leu Lys Val Tyr Ser Val Leu Lys
   1               5                   10                  15
   Gln Glu Leu Leu Glu Asp Pro Ala Phe Glu Trp Thr Pro Asp Ser Arg
               20                  25                  30
   Gln Trp Val Glu Arg Met Leu Asp Tyr Asn Val Pro Gly Gly Lys Leu
           35                  40                  45
   Asn Arg Gly Leu Ser Val Ile Asp Ser Tyr Lys Leu Leu Lys Glu Gly
       50                  55                  60
   Gln Glu Leu Thr Glu Glu Glu Ile Phe Leu Ala Ser Ala Leu Gly Trp
   65                  70                  75                  80
   Cys Ile Glu Trp Leu Gln Ala Tyr Phe Leu Val Leu Asp Asp Ile Met
                   85                  90                  95
   Asp Ser Ser His Thr Arg Arg Gly Gln Pro Cys Trp Phe Arg Val Pro
               100                 105                 110
   Lys Val Gly Leu Ile Ala Ala Asn Asp Gly Ile Leu Leu Arg Asn His
           115                 120                 125
   Ile Pro Arg Ile Leu Lys Lys His Phe Arg Gly Lys Ala Tyr Tyr Val
       130                 135                 140
   Asp Leu Leu Asp Leu Phe Asn Glu Val Glu Phe Gln Thr Ala Ser Gly
   145                 150                 155                 160
   Gln Met Ile Asp Leu Ile Thr Thr Leu Glu Gly Glu Lys Asp Leu Ser
                   165                 170                 175
   Lys Tyr Thr Leu Ser Leu His Arg Arg Ile Val Gln Tyr Lys Thr Ala
               180                 185                 190
   Tyr Tyr Ser Phe Tyr Leu Pro Val Ala Cys Ala Leu Leu Ile Ala Gly
           195                 200                 205
   Glu Asn Leu Asp Asn His Ile Val Val Lys Asp Ile Leu Val Gln Met
       210                 215                 220
   Gly Ile Tyr Phe Gln Val Gln Asp Asp Tyr Leu Asp Cys Phe Gly Asp
   225                 230                 235                 240
   Pro Glu Thr Ile Gly Lys Ile Gly Thr Asp Ile Glu Asp Phe Lys Cys
                   245                 250                 255
   Ser Trp Leu Val Val Lys Ala Leu Glu Leu Cys Asn Glu Glu Gln Lys
               260                 265                 270
   Lys Val Leu Tyr Glu His Tyr Gly Lys Ala Asp Pro Ala Ser Val Ala
           275                 280                 285
   Lys Val Lys Val Leu Tyr Asn Glu Leu Lys Leu Gln Gly Val Phe Thr
       290                 295                 300
   Glu Tyr Glu Asn Glu Ser Tyr Lys Lys Leu Val Thr Ser Ile Glu Ala
   305                 310                 315                 320
   His Pro Ser Lys Pro Val Gln Ala Val Leu Lys Ser Phe Leu Ala Lys
                   325                 330                 335
   Ile Tyr Lys Arg Gln Lys
               340
<210> 4
   <211> 1113
   <212> DNA
   <213> Hevea brasiliensis
<400> 4
   atgagttcag tgaatttggg ctcatgggtt cacacctcct acgtcttaaa ccaagctacc      60
   agatccagat ccaaatccaa atccttctct ctacctttca atcctctaaa aagtttagca     120
   atttcctttg cttatagaaa atcagagcga cccatttcat ctgtctctgc gattattacc     180
   aaggaagaag aaactcttca agaagagcag aataatccac caccctcttt tgatttcaaa     240
   tcctacatgc tccaaaaagg caattccatt aaccaagctt tagaagctgc cattccactc     300
   caagaacccg ctaaaattca cgagtctatg cgttattccc tcttggccgg cggcaagagg     360
   gtacgaccgg ccctctgcct cgctgcgtgt gagcttgttg gtgggaatga ctccatggcg     420
   atgcctgctg catgcgctgt ggaaatgatt catactatgt ctcttatcca tgatgacctc     480
   ccttgcatgg ataacgacga tctccgccgt ggcaagccca ccaatcacat cgtgtttgga     540
   gaggacgtgg cggttctcgc cggtgacgca ctcctagcat ttgcttttga acacatcgct     600
   gtttctactt taaatgtttc ttctgctaga attgtccggg cagttgggga attagcgaag     660
   gcgatcgggg cagaagggtt agttgctggc caagtagttg atataaattc tgagggctca     720
   tctgaggtgg atttagagaa gcttgaattt attcacatcc acaagaccgc taagttgttg     780
   gagggggctg tggtgctagg ggctatattg ggcggaggaa ccgatgagga agtggagaaa     840
   ttgaggaaat atgctaggga tattgggttg ttgttccaag ttgttgacga tattcttgat     900
   gtgactaaat catcccaaga attggggaaa actgcaggca aggacttggt ggcggacaag     960
   gttacatatc ccaagctttt ggggattgag aagtcgaggg aatttgcaga gaagctgaat    1020
   aaggaagctc aggagcagct ggctggattt gatcctgaaa aggcagctcc attgattgct    1080
   ttggctaatt acatcgctca caggcaaaac taa                                 1113
<210> 5
   <211> 370
   <212> PRT
   <213> Hevea brasiliensis
<400> 5
   Met Ser Ser Val Asn Leu Gly Ser Trp Val His Thr Ser Tyr Val Leu
   1               5                   10                  15
   Asn Gln Ala Thr Arg Ser Arg Ser Lys Ser Lys Ser Phe Ser Leu Pro
               20                  25                  30
   Phe Asn Pro Leu Lys Ser Leu Ala Ile Ser Phe Ala Tyr Arg Lys Ser
           35                  40                  45
   Glu Arg Pro Ile Ser Ser Val Ser Ala Ile Ile Thr Lys Glu Glu Glu
       50                  55                  60
   Thr Leu Gln Glu Glu Gln Asn Asn Pro Pro Pro Ser Phe Asp Phe Lys
   65                  70                  75                  80
   Ser Tyr Met Leu Gln Lys Gly Asn Ser Ile Asn Gln Ala Leu Glu Ala
                   85                  90                  95
   Ala Ile Pro Leu Gln Glu Pro Ala Lys Ile His Glu Ser Met Arg Tyr
               100                 105                 110
   Ser Leu Leu Ala Gly Gly Lys Arg Val Arg Pro Ala Leu Cys Leu Ala
           115                 120                 125
   Ala Cys Glu Leu Val Gly Gly Asn Asp Ser Met Ala Met Pro Ala Ala
       130                 135                 140
   Cys Ala Val Glu Met Ile His Thr Met Ser Leu Ile His Asp Asp Leu
   145                 150                 155                 160
   Pro Cys Met Asp Asn Asp Asp Leu Arg Arg Gly Lys Pro Thr Asn His
                   165                 170                 175
   Ile Val Phe Gly Glu Asp Val Ala Val Leu Ala Gly Asp Ala Leu Leu
               180                 185                 190
   Ala Phe Ala Phe Glu His Ile Ala Val Ser Thr Leu Asn Val Ser Ser
           195                 200                 205
   Ala Arg Ile Val Arg Ala Val Gly Glu Leu Ala Lys Ala Ile Gly Ala
       210                 215                 220
   Glu Gly Leu Val Ala Gly Gln Val Val Asp Ile Asn Ser Glu Gly Ser
   225                 230                 235                 240
   Ser Glu Val Asp Leu Glu Lys Leu Glu Phe Ile His Ile His Lys Thr
                   245                 250                 255
   Ala Lys Leu Leu Glu Gly Ala Val Val Leu Gly Ala Ile Leu Gly Gly
               260                 265                 270
   Gly Thr Asp Glu Glu Val Glu Lys Leu Arg Lys Tyr Ala Arg Asp Ile
           275                 280                 285
   Gly Leu Leu Phe Gln Val Val Asp Asp Ile Leu Asp Val Thr Lys Ser
       290                 295                 300
   Ser Gln Glu Leu Gly Lys Thr Ala Gly Lys Asp Leu Val Ala Asp Lys
   305                 310                 315                 320
   Val Thr Tyr Pro Lys Leu Leu Gly Ile Glu Lys Ser Arg Glu Phe Ala
                   325                 330                 335
   Glu Lys Leu Asn Lys Glu Ala Gln Glu Gln Leu Ala Gly Phe Asp Pro
               340                 345                 350
   Glu Lys Ala Ala Pro Leu Ile Ala Leu Ala Asn Tyr Ile Ala His Arg
           355                 360                 365
   Gln Asn
       370
<210> 6
   <211> 1728
   <212> DNA
   <213> Hevea brasiliensis
<400> 6
   atggacacca ccggccggct ccaccaccga aagcatgcta cacccgttga ggaccgttct      60
   ccgaccactc cgaaagcgtc ggacgcgctt ccgcttcccc tctacctgac caacgcggtt     120
   ttcttcacgc tgttcttctc ggtggcgtat tacctccttc accggtggcg cgacaagatc     180
   cgcaactcca ctccccttca tatcgttact ctctctgaaa ttgttgctat tgtctccctc     240
   attgcctctt tcatttacct cctaggattc ttcggtatcg attttgtgca gtcattcatt     300
   gcacgcgcct cccatgacgt gtgggacctc gaagatacgg atcccaacta cctcatcgat     360
   gaagatcacc gtctcgttac ttgccctccc gctaatatat ctactaagac taccattatt     420
   gccgcaccta ccaaattgcc tacctcggaa cccttaattg cacccttagt ctcggaggaa     480
   gacgaaatga tcgtcaactc cgtcgtggat gggaagatac cctcctattc tctggagtcg     540
   aagctcgggg actgcaaacg agcggctgcg attcgacgcg aggctttgca gaggatgaca     600
   aggaggtcgc tggaaggctt gccagtagaa gggttcgatt acgagtcgat tttaggacaa     660
   tgctgtgaaa tgccagtggg atacgtgcag attccggtgg ggattgcggg gccgttgttg     720
   ctgaacgggc gggagtactc tgttccaatg gcgaccacgg agggttgttt ggtggcgagc     780
   actaatagag ggtgtaaggc gatttacttg tcaggtgggg ccaccagcgt cttgttgaag     840
   gatggcatga caagagcgcc tgttgtaaga ttcgcgtcgg cgactagagc cgcggagttg     900
   aagttcttct tggaggatcc tgacaatttt gataccttgg ccgtagtttt taacaagtcc     960
   agtagatttg cgaggctcca aggcattaaa tgctcaattg ctggtaagaa tctttatata    1020
   agattcagct acagcactgg cgatgcaatg gggatgaaca tggtttctaa aggggttcaa     1080
   aacgttcttg aatttcttca aagtgatttt tctgatatgg atgtcattgg aatctcagga    1140
   aatttttgtt cggataagaa gcctgctgct gtaaattgga ttgaaggacg tggcaaatca    1200
   gttgtttgtg aggcaattat caaggaagag gtggtgaaga aggtgttgaa aaccaatgtg    1260
   gcctccctag tggagcttaa catgctcaag aatcttgctg gttctgctgt tgctggtgct    1320
   ttgggtggat ttaatgccca tgcaggcaac atcgtatctg caatctttat tgccactggc    1380
   caggatccag cacagaatgt tgagagttct cattgcatta ccatgatgga agctgtcaat    1440
   gatggaaagg atctccatat ctctgtgacc atgccctcca ttgaggtggg tacagtcgga    1500
   ggtggaactc aacttgcatc tcagtctgct tgtctcaatt tgcttggggt gaagggtgca    1560
   aacaaagagt cgccaggatc aaactcaagg ctccttgctg ccatcgtagc tggttcagtt    1620
   ttggctggtg agctctcctt gatgtctgcc attgcagctg ggcagcttgt caagagtcac    1680
   atgaagtaca acagatccag caaagatatg tctaaagctg catcttag                 1728
<210> 7
   <211> 1761
   <212> DNA
   <213> Hevea brasiliensis
<400> 7
   atggacgagg ttcgccggcg accccccaag catatcgtcc ggaaggacca cgacggcgaa 60
   gttctcaact ccttcagcca tggccaccat cttcctcctc tcaagccgtc tgattattct 120
   ctccctctct ccctctacct cgctaatgct ctcgttttct cgctcttctt ctcagttgct 180
   tatttccttc tccacagatg gcgggaaaag atccgcaaat ccactcctct tcacatagtc 240
   accttccctg agattgctgc tttgatttgc ttggtagctt ctgtcatcta tcttcttggt 300
   ttctttggca ttggcttcgt ccactccttt tctagagctt ccaccgattc ctgggatgtc 360
   gaagaatacg atgatgataa cattatcatc aaggaagata ctcgtcctac gggggcttgc 420
   gctgcacctt cccttgactg ctccctctct cttcccacca aaattcatgc acccattgtc 480
   tcgactacaa ccaccagtac tttatctgat gatgacgaac aaattatcaa atcagtagtc 540
   tctggctcca ttccttccta ttcccttgaa tcaaagcttg ggaactgtaa gcgggctgct 600
   ttaattcgcc gggagacgct gcagaggatg tcggggagat ccttggaggg actgccctta 660
   gatgggttcg attacgagtc aatattgggg cagtgctgcg agatggcaat tgggtacgtg 720
   cagattccgg tgggaattgc tgggccattg ttgctggatg gaaaggaata tactgtgcca 780
   atggccacga ccgagggttg ccttgtggcc agtgcaaata gaggatgcaa ggccatttac 840
   gcatcgggag gggctacctc cgtgttgctg agggacggca tgacccgtgc tcccgttgtt 900
   aggtttccca ccgccaagag ggccgctgac ttgaagtttt tcatggagga ccctgacaat 960
   ttcgatacca ttgccgttgt tttcaataag tcaagcagat ttgcgaggct acagagtgtc 1020
   caatgtgcaa tagcaggaaa aaatttatac atgaggttta gctgcagcac aggtgatgcc 1080
   atggggatga atatggtctc caaagcggtc caaaatgtca tcgattatct ccagaatgat 1140
   tttcctgaca tggatgtcat cggtctcact gggaacttct gtgcggacaa gaaggcagca 1200
   gcagtaaact ggatagaagg gcgtgggaag tctgttgtat gtgaagcaat cataaaggaa 1260
   gaggtggtta agaaggtatt gaaaaccaac gtggccgccc tggtggagct taacatgatt 1320
   aaaaacctta ctggctcagc cgtagcaggt tctcttggtg ggtttaacgc ccatgctagc 1380
   aatatggtaa ctgcagtata catagctaca gggcaggatc ctgctcaaaa cgtggagagc 1440
   tctcactgca ttaccatgat ggaagctgtt aacgatggca aggaccttca catctcagtg 1500
   tccatgcctt ccattgagct gggcacagtt ggaggtggta ctcaacttgc atctcaatca 1560
   gcttgtctga acctacttgg ggtaaagggt gcaagcaaag attcccctgg ttcaaactca 1620
   aggcttctgg caactattgt cgctggttct gttctggcag gggagctgtc tcttatgtct 1680
   gctattgcag ctgggcaact cgttaatagc cacatgaaat acaacagatc tgcaaaagat 1740
   gtatccaaaa taactttctg a 1761
<210> 8
   <211> 1821
   <212> DNA
   <213> Hevea brasiliensis
<400> 8
   atggatgtgc gccggcgacc tacctctgga aaaaccatcc actctgtgaa acctaaatcg 60
   gtggaggatg agagtgccca gaagccctct gatgcattac ctctccctct gtacctaatc 120
   aatgctctct gctttaccgt tttcttttat gttgtttatt ttctgctcag ccgctggcgt 180
   gagaagattc gcacatctac ccctctacat gtcgttgctc tctccgaaat tgctgctatt 240
   gttgctttcg ttgcttcctt catttaccta cttggcttct ttggtattga ctttgttcag 300
   tccctgatat tacgccctcc tacagatatg tgggctgtgg atgatgatga ggaggagacg 360
   gaggaaggga ttgtactcag ggaagatacc cggaaattgc catgtgggca agctctcgat 420
   tgctctcttt ctgcaccacc actgtcacgg gcagttgttt cttctccgaa agcaatggac 480
   cctatcgtgc ttccttctcc gaagccgaaa gtgttcgacg aaatcccatt tccgacaacc 540
   accaccatcc ctattttggg cgatgaagat gaggagatca ttaaatctgt tgtggctgga 600
   actatccctt cgtattccct cgagtctaaa ttgggggatt gtaagcgtgc tgctgcaatc 660
   aggcgcgagg ccttgcagag gataactggc aagtctctct ctgggttacc actggagggc 720
   tttgattacg agtcaatctt ggggcagtgt tgcgagatgc cagttgggta tgtccaaatt 780
   cccgttggaa ttgctggacc tttgttgcta gacggcaagg aatactcggt tcccatggct 840
   accactgaag ggtgcttggt agccagcacc aataggggtt gcaaggccat tcacttatct 900
   ggtggagcca caagtgtcct gttgagagat gggatgacca gagcgccggt cgttaggttt 960
   gggacagcaa aaagagcggc ccagttgaag ttgtacttgg aggatcctgc caattttgag 1020
   accctgtcca cttcgtttaa caaatccagc agatttggca ggcttcaaag catcaaatgc 1080
   gctattgccg gaaagaacct atacatgaga ttctgttgca gcactggtga cgctatgggt 1140
   atgaacatgg tctctaaagg tgtccagaat gtattgaatt tcctccagaa tgatttccct 1200
   gacatggatg ttattggcct ttctggtaac ttctgctcgg ataagaagcc tgcagctgtg 1260
   aactggattg aaggaagggg caagtcagtg gtgtgcgagg ccataatcaa gggagatgtg 1320
   gtgaagaagg tgttgaagac gaatgtggaa gccttagtgg agcttaacat gctcaagaac 1380
   ctcactggtt cagccatggc tggagcttta ggagggttca atgctcatgc cagtaacata 1440
   gtgactgcaa tttacatagc aaccggccaa gatccagcac agaacgtaga gagttctaac 1500
   tgcatcacca tgatggaagc tgttaatgat ggacaggatc ttcatgtctc tgtgactatg 1560
   ccttctatcg aggttggtac tgttggagga ggtactcagc ttgcatctca atcagcatgc 1620
   ctgaacctgc ttggggtgaa gggagcaagc aaagagacgc caggagctaa ctccagagtt 1680
   ctagcctcaa tagttgctgg ttctgttctt gctgctgagc tatctctcat gtctgcaatt 1740
   gctgctggac aactagtgaa cagccacatg aaatacaaca gagccaacaa agaagctgct 1800
   gtttccaagc cttcttccta a 1821
<210> 9
   <211> 1581
   <212> DNA
   <213> Hevea brasiliensis
<400> 9
   atggatgccc gccggcgacc cacctccggg aatcccatcc attcccgcaa agtaaaagca      60
   ttggaggatg agaataccca gaagccctcc gatgcactgc ctctccatat ctacctaacc     120
   aatgctctct gcttcaccgt cttcttttgg gttgtttatt ttcttcttag ccgctggcgt     180
   gagaagattc gcacctccgc ccctctgcat gttgtcactc tctctgaaat tgctgctatt     240
   gttgctttgt ttgcttcctt catttacctt cttggcttct ttggtattga ttttgttcag     300
   tcccttatct tacgccctcc aaccgatatg tgggctgtgg atgatgaaga ggaggagccg     360
   gcggaacaaa ttctgctcaa ggaagatgcc cggaaatcgc catgtgggca agctctcgat     420
   tgctctctta ctgcaccacc actatcacgg ccaattgttt cttcttcaaa agcagtgggc     480
   ccaattgtgc ttccttctcc gaagccgaaa gtggtcgagg aaatctcatt tccagccatc     540
   actaccaccg ccactttggg cgaggaagat gaggagatca tcaaatctgt tgtggctgga     600
   actacccctt cgtattccct cgagtctaaa ttgggggatt gcaagcgtgc tgctgcaatc     660
   aggcgggagg cgttgcagag gataactggc aaatcgcttt ctgggttgcc tctggagggc     720
   tttgattatg agtcaatatt gggacagtgc tgcgagatgc ccgttgggta tgtccagatt     780
   cccgttggca ttgctggacc tttgttgcta gacggtaagg aagtttcggt tcccatggct     840
   accactgaag ggtgcttggt agccagtacc aataggggct gcaaggccat tcacatatct    900
   ggtggagcca caagcgtcgt gttgagggat gggatgacca gggcacctgt tgttaggttc     960
   gggacggcaa aaagagcagc ccaattgaag ttttacttgg aggatagtgc caattttgag    1020
   actttgtcta ctgtgtttaa caaatccagc agatttggca ggcttcaaag tattaggtgc    1080
   gctattgctg gaaagaacct gtacattaga ttctgttgcg gcactggtga cgctatgggc    1140
   atgaacatgg tgtctaaagg tgtccagaat gtattggatt tcctccagaa tgatttccct    1200
   gacatggatg ttattggcgt ctctggtaac ttctgctcgg ataagaagcc tgcagctgtg    1260
   aactggattg aaggaagggg aaagtcagta gcgtgcgaag ccataatcaa gggtgatgtg    1320
   gtgaagaagg tgttgaagac gaatgtggaa gccttggtgg agcttaatat gctgaagaat    1380
   ctaactggtt ctgccctggc tggagctcta ggtgggttca atgctcatgc cagtaacata    1440
   gtgactgcta tctacatagc gacaggccaa gatcctgctc agaacgttga gagttctcac    1500
   tgtatcacca tgatggaagc tgttaatgat gggcaggatc ttcacgtctc tgtgacaatg    1560
   ccttctattg aggtaaattg a                                              1581
<210> 10
   <211> 575
   <212> PRT
   <213> Hevea brasiliensis
<400> 10
   Met Asp Thr Thr Gly Arg Leu His His Arg Lys His Ala Thr Pro Val
   1               5                   10                  15
   Glu Asp Arg Ser Pro Thr Thr Pro Lys Ala Ser Asp Ala Leu Pro Leu
               20                  25                  30
   Pro Leu Tyr Leu Thr Asn Ala Val Phe Phe Thr Leu Phe Phe Ser Val
           35                  40                  45
   Ala Tyr Tyr Leu Leu His Arg Trp Arg Asp Lys Ile Arg Asn Ser Thr
       50                  55                  60
   Pro Leu His Ile Val Thr Leu Ser Glu Ile Val Ala Ile Val Ser Leu
   65                  70                  75                  80
   Ile Ala Ser Phe Ile Tyr Leu Leu Gly Phe Phe Gly Ile Asp Phe Val
                   85                  90                  95
   Gln Ser Phe Ile Ala Arg Ala Ser His Asp Val Trp Asp Leu Glu Asp
               100                 105                 110
   Thr Asp Pro Asn Tyr Leu Ile Asp Glu Asp His Arg Leu Val Thr Cys
           115                 120                 125
   Pro Pro Ala Asn Ile Ser Thr Lys Thr Thr Ile Ile Ala Ala Pro Thr
       130                 135                 140
   Lys Leu Pro Thr Ser Glu Pro Leu Ile Ala Pro Leu Val Ser Glu Glu
   145                 150                 155                 160
   Asp Glu Met Ile Val Asn Ser Val Val Asp Gly Lys Ile Pro Ser Tyr
                   165                 170                 175
   Ser Leu Glu Ser Lys Leu Gly Asp Cys Lys Arg Ala Ala Ala Ile Arg
               180                 185                 190
   Arg Glu Ala Leu Gln Arg Met Thr Arg Arg Ser Leu Glu Gly Leu Pro
           195                 200                 205
   Val Glu Gly Phe Asp Tyr Glu Ser Ile Leu Gly Gln Cys Cys Glu Met
       210                 215                 220
   Pro Val Gly Tyr Val Gln Ile Pro Val Gly Ile Ala Gly Pro Leu Leu
   225                 230                 235                 240
   Leu Asn Gly Arg Glu Tyr Ser Val Pro Met Ala Thr Thr Glu Gly Cys
                   245                 250                 255
   Leu Val Ala Ser Thr Asn Arg Gly Cys Lys Ala Ile Tyr Leu Ser Gly
   260 265 270
   Gly Ala Thr Ser Val Leu Leu Lys Asp Gly Met Thr Arg Ala Pro Val
   275 280 285
   Val Arg Phe Ala Ser Ala Thr Arg Ala Ala Glu Leu Lys Phe Phe Leu
   290 295 300
   Glu Asp Pro Asp Asn Phe Asp Thr Leu Ala Val Val Phe Asn Lys Ser
   305 310 315 320
   Ser Arg Phe Ala Arg Leu Gln Gly Ile Lys Cys Ser Ile Ala Gly Lys
   325 330 335
   Asn Leu Tyr Ile Arg Phe Ser Tyr Ser Thr Gly Asp Ala Met Gly Met
   340 345 350
   Asn Met Val Ser Lys Gly Val Gln Asn Val Leu Glu Phe Leu Gln Ser
   355 360 365
   Asp Phe Ser Asp Met Asp Val Ile Gly Ile Ser Gly Asn Phe Cys Ser
   370 375 380
   Asp Lys Lys Pro Ala Ala Val Asn Trp Ile Glu Gly Arg Gly Lys Ser
   385 390 395 400
   Val Val Cys Glu Ala Ile Ile Lys Glu Glu Val Val Lys Lys Val Leu
   405 410 415
   Lys Thr Asn Val Ala Ser Leu Val Glu Leu Asn Met Leu Lys Asn Leu
   420 425 430
   Ala Gly Ser Ala Val Ala Gly Ala Leu Gly Gly Phe Asn Ala His Ala
   435 440 445
   Gly Asn Ile Val Ser Ala Ile Phe Ile Ala Thr Gly Gln Asp Pro Ala
   450 455 460
   Gln Asn Val Glu Ser Ser His Cys Ile Thr Met Met Glu Ala Val Asn
   465 470 475 480
   Asp Gly Lys Asp Leu His Ile Ser Val Thr Met Pro Ser Ile Glu Val
   485 490 495
   Gly Thr Val Gly Gly Gly Thr Gln Leu Ala Ser Gln Ser Ala Cys Leu
   500 505 510
   Asn Leu Leu Gly Val Lys Gly Ala Asn Lys Glu Ser Pro Gly Ser Asn
   515 520 525
   Ser Arg Leu Leu Ala Ala Ile Val Ala Gly Ser Val Leu Ala Gly Glu
   530 535 540
   Leu Ser Leu Met Ser Ala Ile Ala Ala Gly Gln Leu Val Lys Ser His
   545 550 555 560
   Met Lys Tyr Asn Arg Ser Ser Lys Asp Met Ser Lys Ala Ala Ser
   565 570 575
<210> 11
   <211> 586
   <212> PRT
   <213> Hevea brasiliensis
<400> 11
   Met Asp Glu Val Arg Arg Arg Pro Pro Lys His Ile Val Arg Lys Asp
   1               5                   10                  15
   His Asp Gly Glu Val Leu Asn Ser Phe Ser His Gly His His Leu Pro
               20                  25                  30
   Pro Leu Lys Pro Ser Asp Tyr Ser Leu Pro Leu Ser Leu Tyr Leu Ala
           35                  40                  45
   Asn Ala Leu Val Phe Ser Leu Phe Phe Ser Val Ala Tyr Phe Leu Leu
       50                  55                  60
   His Arg Trp Arg Glu Lys Ile Arg Lys Ser Thr Pro Leu His Ile Val
   65                  70                  75                  80
   Thr Phe Pro Glu Ile Ala Ala Leu Ile Cys Leu Val Ala Ser Val Ile
                   85                  90                  95
   Tyr Leu Leu Gly Phe Phe Gly Ile Gly Phe Val His Ser Phe Ser Arg
               100                 105                 110
   Ala Ser Thr Asp Ser Trp Asp Val Glu Glu Tyr Asp Asp Asp Asn Ile
           115                 120                 125
   Ile Ile Lys Glu Asp Thr Arg Pro Thr Gly Ala Cys Ala Ala Pro Ser
       130                 135                 140
   Leu Asp Cys Ser Leu Ser Leu Pro Thr Lys Ile His Ala Pro Ile Val
   145                 150                 155                 160
   Ser Thr Thr Thr Thr Ser Thr Leu Ser Asp Asp Asp Glu Gln Ile Ile
   165 170 175
   Lys Ser Val Val Ser Gly Ser Ile Pro Ser Tyr Ser Leu Glu Ser Lys
   180 185 190
   Leu Gly Asn Cys Lys Arg Ala Ala Leu Ile Arg Arg Glu Thr Leu Gln
   195 200 205
   Arg Met Ser Gly Arg Ser Leu Glu Gly Leu Pro Leu Asp Gly Phe Asp
   210 215 220
   Tyr Glu Ser Ile Leu Gly Gln Cys Cys Glu Met Ala Ile Gly Tyr Val
   225 230 235 240
   Gln Ile Pro Val Gly Ile Ala Gly Pro Leu Leu Leu Asp Gly Lys Glu
   245 250 255
   Tyr Thr Val Pro Met Ala Thr Thr Glu Gly Cys Leu Val Ala Ser Ala
   260 265 270
   Asn Arg Gly Cys Lys Ala Ile Tyr Ala Ser Gly Gly Ala Thr Ser Val
   275 280 285
   Leu Leu Arg Asp Gly Met Thr Arg Ala Pro Val Val Arg Phe Pro Thr
   290 295 300
   Ala Lys Arg Ala Ala Asp Leu Lys Phe Phe Met Glu Asp Pro Asp Asn
   305 310 315 320
   Phe Asp Thr Ile Ala Val Val Phe Asn Lys Ser Ser Arg Phe Ala Arg
   325 330 335
   Leu Gln Ser Val Gln Cys Ala Ile Ala Gly Lys Asn Leu Tyr Met Arg
   340 345 350
   Phe Ser Cys Ser Thr Gly Asp Ala Met Gly Met Asn Met Val Ser Lys
   355 360 365
   Ala Val Gln Asn Val Ile Asp Tyr Leu Gln Asn Asp Phe Pro Asp Met
   370 375 380
   Asp Val Ile Gly Leu Thr Gly Asn Phe Cys Ala Asp Lys Lys Ala Ala
   385 390 395 400
   Ala Val Asn Trp Ile Glu Gly Arg Gly Lys Ser Val Val Cys Glu Ala
   405 410 415
   Ile Ile Lys Glu Glu Val Val Lys Lys Val Leu Lys Thr Asn Val Ala
   420 425 430
   Ala Leu Val Glu Leu Asn Met Ile Lys Asn Leu Thr Gly Ser Ala Val
   435 440 445
   Ala Gly Ser Leu Gly Gly Phe Asn Ala His Ala Ser Asn Met Val Thr
   450 455 460
   Ala Val Tyr Ile Ala Thr Gly Gln Asp Pro Ala Gln Asn Val Glu Ser
   465 470 475 480
   Ser His Cys Ile Thr Met Met Glu Ala Val Asn Asp Gly Lys Asp Leu
   485 490 495
   His Ile Ser Val Ser Met Pro Ser Ile Glu Leu Gly Thr Val Gly Gly
   500 505 510
   Gly Thr Gln Leu Ala Ser Gln Ser Ala Cys Leu Asn Leu Leu Gly Val
   515 520 525
   Lys Gly Ala Ser Lys Asp Ser Pro Gly Ser Asn Ser Arg Leu Leu Ala
   530 535 540
   Thr Ile Val Ala Gly Ser Val Leu Ala Gly Glu Leu Ser Leu Met Ser
   545 550 555 560
   Ala Ile Ala Ala Gly Gln Leu Val Asn Ser His Met Lys Tyr Asn Arg
   565 570 575
   Ser Ala Lys Asp Val Ser Lys Ile Thr Phe
   580 585
<210> 12
   <211> 606
   <212> PRT
   <213> Hevea brasiliensis
<400> 12
   Met Asp Val Arg Arg Arg Pro Thr Ser Gly Lys Thr Ile His Ser Val
   1               5                   10                  15
   Lys Pro Lys Ser Val Glu Asp Glu Ser Ala Gln Lys Pro Ser Asp Ala
               20                  25                  30
   Leu Pro Leu Pro Leu Tyr Leu Ile Asn Ala Leu Cys Phe Thr Val Phe
           35                  40                  45
   Phe Tyr Val Val Tyr Phe Leu Leu Ser Arg Trp Arg Glu Lys Ile Arg
       50                  55                  60
   Thr Ser Thr Pro Leu His Val Val Ala Leu Ser Glu Ile Ala Ala Ile
   65                  70                  75                  80
   Val Ala Phe Val Ala Ser Phe Ile Tyr Leu Leu Gly Phe Phe Gly Ile
                   85                  90                  95
   Asp Phe Val Gln Ser Leu Ile Leu Arg Pro Pro Thr Asp Met Trp Ala
               100                 105                 110
   Val Asp Asp Asp Glu Glu Glu Thr Glu Glu Gly Ile Val Leu Arg Glu
           115                 120                 125
   Asp Thr Arg Lys Leu Pro Cys Gly Gln Ala Leu Asp Cys Ser Leu Ser
       130                 135                 140
   Ala Pro Pro Leu Ser Arg Ala Val Val Ser Ser Pro Lys Ala Met Asp
   145                 150                 155                 160
   Pro Ile Val Leu Pro Ser Pro Lys Pro Lys Val Phe Asp Glu Ile Pro
                   165                 170                 175
   Phe Pro Thr Thr Thr Thr Ile Pro Ile Leu Gly Asp Glu Asp Glu Glu
               180                 185                 190
   Ile Ile Lys Ser Val Val Ala Gly Thr Ile Pro Ser Tyr Ser Leu Glu
           195                 200                 205
   Ser Lys Leu Gly Asp Cys Lys Arg Ala Ala Ala Ile Arg Arg Glu Ala
       210                 215                 220
   Leu Gln Arg Ile Thr Gly Lys Ser Leu Ser Gly Leu Pro Leu Glu Gly
   225                 230                 235                 240
   Phe Asp Tyr Glu Ser Ile Leu Gly Gln Cys Cys Glu Met Pro Val Gly
                   245                 250                 255
   Tyr Val Gln Ile Pro Val Gly Ile Ala Gly Pro Leu Leu Leu Asp Gly
               260                 265                 270
   Lys Glu Tyr Ser Val Pro Met Ala Thr Thr Glu Gly Cys Leu Val Ala
           275                 280                 285
   Ser Thr Asn Arg Gly Cys Lys Ala Ile His Leu Ser Gly Gly Ala Thr
       290                 295                 300
   Ser Val Leu Leu Arg Asp Gly Met Thr Arg Ala Pro Val Val Arg Phe
   305                 310                 315                 320
   Gly Thr Ala Lys Arg Ala Ala Gln Leu Lys Leu Tyr Leu Glu Asp Pro
                   325                 330                 335
   Ala Asn Phe Glu Thr Leu Ser Thr Ser Phe Asn Lys Ser Ser Arg Phe
               340                 345                 350
   Gly Arg Leu Gln Ser Ile Lys Cys Ala Ile Ala Gly Lys Asn Leu Tyr
           355                 360                 365
   Met Arg Phe Cys Cys Ser Thr Gly Asp Ala Met Gly Met Asn Met Val
       370                 375                 380
   Ser Lys Gly Val Gln Asn Val Leu Asn Phe Leu Gln Asn Asp Phe Pro
   385                 390                 395                 400
   Asp Met Asp Val Ile Gly Leu Ser Gly Asn Phe Cys Ser Asp Lys Lys
                   405                 410                 415
   Pro Ala Ala Val Asn Trp Ile Glu Gly Arg Gly Lys Ser Val Val Cys
               420                 425                 430
   Glu Ala Ile Ile Lys Gly Asp Val Val Lys Lys Val Leu Lys Thr Asn
           435                 440                 445
   Val Glu Ala Leu Val Glu Leu Asn Met Leu Lys Asn Leu Thr Gly Ser
       450                 455                 460
   Ala Met Ala Gly Ala Leu Gly Gly Phe Asn Ala His Ala Ser Asn Ile
   465                 470                 475                 480
   Val Thr Ala Ile Tyr Ile Ala Thr Gly Gln Asp Pro Ala Gln Asn Val
                   485                 490                 495
   Glu Ser Ser Asn Cys Ile Thr Met Met Glu Ala Val Asn Asp Gly Gln
               500                 505                 510
   Asp Leu His Val Ser Val Thr Met Pro Ser Ile Glu Val Gly Thr Val
           515                 520                 525
   Gly Gly Gly Thr Gln Leu Ala Ser Gln Ser Ala Cys Leu Asn Leu Leu
       530                 535                 540
   Gly Val Lys Gly Ala Ser Lys Glu Thr Pro Gly Ala Asn Ser Arg Val
   545                 550                 555                 560
   Leu Ala Ser Ile Val Ala Gly Ser Val Leu Ala Ala Glu Leu Ser Leu
   565 570 575
   Met Ser Ala Ile Ala Ala Gly Gln Leu Val Asn Ser His Met Lys Tyr
   580 585 590
   Asn Arg Ala Asn Lys Glu Ala Ala Val Ser Lys Pro Ser Ser
   595 600 605
<210> 13
   <211> 526
   <212> PRT
   <213> Hevea brasiliensis
<400> 13
   Met Asp Ala Arg Arg Arg Pro Thr Ser Gly Asn Pro Ile His Ser Arg
   1 5 10 15
   Lys Val Lys Ala Leu Glu Asp Glu Asn Thr Gln Lys Pro Ser Asp Ala
   20 25 30
   Leu Pro Leu His Ile Tyr Leu Thr Asn Ala Leu Cys Phe Thr Val Phe
   35 40 45
   Phe Trp Val Val Tyr Phe Leu Leu Ser Arg Trp Arg Glu Lys Ile Arg
   50 55 60
   Thr Ser Ala Pro Leu His Val Val Thr Leu Ser Glu Ile Ala Ala Ile
   65 70 75 80
   Val Ala Leu Phe Ala Ser Phe Ile Tyr Leu Leu Gly Phe Phe Gly Ile
   85 90 95
   Asp Phe Val Gln Ser Leu Ile Leu Arg Pro Pro Thr Asp Met Trp Ala
   100 105 110
   Val Asp Asp Glu Glu Glu Glu Pro Ala Glu Gln Ile Leu Leu Lys Glu
   115 120 125
   Asp Ala Arg Lys Ser Pro Cys Gly Gln Ala Leu Asp Cys Ser Leu Thr
   130 135 140
   Ala Pro Pro Leu Ser Arg Pro Ile Val Ser Ser Ser Lys Ala Val Gly
   145 150 155 160
   Pro Ile Val Leu Pro Ser Pro Lys Pro Lys Val Val Glu Glu Ile Ser
   165 170 175
   Phe Pro Ala Ile Thr Thr Thr Ala Thr Leu Gly Glu Glu Asp Glu Glu
               180                 185                 190
   Ile Ile Lys Ser Val Val Ala Gly Thr Thr Pro Ser Tyr Ser Leu Glu
           195                 200                 205
   Ser Lys Leu Gly Asp Cys Lys Arg Ala Ala Ala Ile Arg Arg Glu Ala
       210                 215                 220
   Leu Gln Arg Ile Thr Gly Lys Ser Leu Ser Gly Leu Pro Leu Glu Gly
   225                 230                 235                 240
   Phe Asp Tyr Glu Ser Ile Leu Gly Gln Cys Cys Glu Met Pro Val Gly
                   245                 250                 255
   Tyr Val Gln Ile Pro Val Gly Ile Ala Gly Pro Leu Leu Leu Asp Gly
               260                 265                 270
   Lys Glu Val Ser Val Pro Met Ala Thr Thr Glu Gly Cys Leu Val Ala
           275                 280                 285
   Ser Thr Asn Arg Gly Cys Lys Ala Ile His Ile Ser Gly Gly Ala Thr
       290                 295                 300
   Ser Val Val Leu Arg Asp Gly Met Thr Arg Ala Pro Val Val Arg Phe
   305                 310                 315                 320
   Gly Thr Ala Lys Arg Ala Ala Gln Leu Lys Phe Tyr Leu Glu Asp Ser
                   325                 330                 335
   Ala Asn Phe Glu Thr Leu Ser Thr Val Phe Asn Lys Ser Ser Arg Phe
               340                 345                 350
   Gly Arg Leu Gln Ser Ile Arg Cys Ala Ile Ala Gly Lys Asn Leu Tyr
           355                 360                 365
   Ile Arg Phe Cys Cys Gly Thr Gly Asp Ala Met Gly Met Asn Met Val
       370                 375                 380
   Ser Lys Gly Val Gln Asn Val Leu Asp Phe Leu Gln Asn Asp Phe Pro
   385                 390                 395                 400
   Asp Met Asp Val Ile Gly Val Ser Gly Asn Phe Cys Ser Asp Lys Lys
                   405                 410                 415
   Pro Ala Ala Val Asn Trp Ile Glu Gly Arg Gly Lys Ser Val Ala Cys
               420                 425                 430
   Glu Ala Ile Ile Lys Gly Asp Val Val Lys Lys Val Leu Lys Thr Asn
   435 440 445
   Val Glu Ala Leu Val Glu Leu Asn Met Leu Lys Asn Leu Thr Gly Ser
   450 455 460
   Ala Leu Ala Gly Ala Leu Gly Gly Phe Asn Ala His Ala Ser Asn Ile
   465 470 475 480
   Val Thr Ala Ile Tyr Ile Ala Thr Gly Gln Asp Pro Ala Gln Asn Val
   485 490 495
   Glu Ser Ser His Cys Ile Thr Met Met Glu Ala Val Asn Asp Gly Gln
   500 505 510
   Asp Leu His Val Ser Val Thr Met Pro Ser Ile Glu Val Asn
   515 520 525
<210> 14
   <211> 705
   <212> DNA
   <213> Hevea brasiliensis
<400> 14
   atgggtgagg ctccagatgt cggcatggat gctgtccaga aacgcctcat gttcgacgat 60
   gaatgcattt tagtagatga gaacgatggt gttgttggtc atgcttccaa atataattgt 120
   catttgtggg aaaatatttt gaaggggaac gcattacata gagcttttag cgtatttctc 180
   ttcaactcaa aatatgagct actccttcag caacgctctg ggacaaaggt gacattcccg 240
   cttgtatgga caaacacttg ctgtagtcat cctctgtacc gtgaatctga gcttattgat 300
   gaggatgctc ttggtgtgag aaatgctgca caaaggaagc ttttcgatga gcttggtatc 360
   cctgctgaag atgttccagt tgatcagttt actccactag gacgtatact atataaggcg 420
   tcctccgatg gaaagtgggg agagcatgaa cttgattatc tgctctttat agtccgtgat 480
   gttaatgtaa atccaaaccc tgatgaggta gctgatgtaa agtatgttaa ccgggatcag 540
   ttgaaggagc tcttgaggaa ggcggattct ggcgaggaag gtataaattt gtcaccttgg 600
   tttagactag ttgtggacaa cttcttgttg aaatggtggg aaaatgtcga aaatgggaca 660
   ctcaaggaag cagttgacat gaaaacgatt cacaagttga gttga 705
<210> 15
   <211> 234
   <212> PRT
   <213> Hevea brasiliensis
<400> 15
   Met Gly Glu Ala Pro Asp Val Gly Met Asp Ala Val Gln Lys Arg Leu
   1               5                   10                  15
   Met Phe Asp Asp Glu Cys Ile Leu Val Asp Glu Asn Asp Gly Val Val
               20                  25                  30
   Gly His Ala Ser Lys Tyr Asn Cys His Leu Trp Glu Asn Ile Leu Lys
           35                  40                  45
   Gly Asn Ala Leu His Arg Ala Phe Ser Val Phe Leu Phe Asn Ser Lys
       50                  55                  60
   Tyr Glu Leu Leu Leu Gln Gln Arg Ser Gly Thr Lys Val Thr Phe Pro
   65                  70                  75                  80
   Leu Val Trp Thr Asn Thr Cys Cys Ser His Pro Leu Tyr Arg Glu Ser
                   85                  90                  95
   Glu Leu Ile Asp Glu Asp Ala Leu Gly Val Arg Asn Ala Ala Gln Arg
               100                 105                 110
   Lys Leu Phe Asp Glu Leu Gly Ile Pro Ala Glu Asp Val Pro Val Asp
           115                 120                 125
   Gln Phe Thr Pro Leu Gly Arg Ile Leu Tyr Lys Ala Ser Ser Asp Gly
       130                 135                 140
   Lys Trp Gly Glu His Glu Leu Asp Tyr Leu Leu Phe Ile Val Arg Asp
   145                 150                 155                 160
   Val Asn Val Asn Pro Asn Pro Asp Glu Val Ala Asp Val Lys Tyr Val
                   165                 170                 175
   Asn Arg Asp Gln Leu Lys Glu Leu Leu Arg Lys Ala Asp Ser Gly Glu
               180                 185                 190
   Glu Gly Ile Asn Leu Ser Pro Trp Phe Arg Leu Val Val Asp Asn Phe
           195                 200                 205
   Leu Leu Lys Trp Trp Glu Asn Val Glu Asn Gly Thr Leu Lys Glu Ala
       210                 215                 220
   Val Asp Met Lys Thr Ile His Lys Leu Ser
   225                 230
<210> 16
   <211> 873
   <212> DNA
   <213> Hevea brasiliensis
<400> 16
   atgaaattat acaccggtga gaggccaagt gtgttcagac ttttagggaa gtatatgaga       60
   aaagggttat atggcatcct aacccagggt cccatcccta ctcatcttgc cttcatattg      120
   gatggaaaca ggaggtttgc taagaagcat aaactgccag aaggaggtgg tcataaggct      180
   ggatttttag ctcttctgaa cgtgctaact tattgctatg agttaggagt gaaatatgcg      240
   actatctatg cctttagcat cgataatttt cgaaggaaac ctcatgaggt tcagtacgta      300
   atgaatctaa tgctggagaa gattgaaggg atgatcatgg aagaaagtat catcaatgca      360
   tatgatattt gcgtacgttt tgtgggtaac ctgaagcttt taagtgagcc agtcaagacc      420
   gcagcagata agattatgag ggctactgcc aacaattcca aatgtgtgct tctccttgct      480
   gtatgctata cttcaactga tgagatcgtg catgctgttg aagaatcctc tgaattgaac      540
   tccaatgaag tttgtaacaa tcaagaattg gaggaggcaa atgcaactgg aagcggtact      600
   gtgattcaaa ctgagaacat ggagtcgtat tctggaataa aacttgtaga ccttgagaaa      660
   aacacctaca taaatcctta tcctgatgtt ctgattcgaa cttctgggga gacccgtctg      720
   agcaactact tactttggca gactactaat tgcatactgt attctcctta tgcactgtgg      780
   ccagagattg gtcttcgaca cgtggtgtgg tcagtaatta acttccaacg tcattattct      840
   tacttggaga aacataagga atacttaaaa taa                                   873
<210> 17
   <211> 290
   <212> PRT
   <213> Hevea brasiliensis
<400> 17
   Met Lys Leu Tyr Thr Gly Glu Arg Pro Ser Val Phe Arg Leu Leu Gly
   1               5                   10                  15
   Lys Tyr Met Arg Lys Gly Leu Tyr Gly Ile Leu Thr Gln Gly Pro Ile
               20                  25                  30
   Pro Thr His Leu Ala Phe Ile Leu Asp Gly Asn Arg Arg Phe Ala Lys
           35                  40                  45
   Lys His Lys Leu Pro Glu Gly Gly Gly His Lys Ala Gly Phe Leu Ala
       50                  55                  60
   Leu Leu Asn Val Leu Thr Tyr Cys Tyr Glu Leu Gly Val Lys Tyr Ala
   65                  70                  75                  80
   Thr Ile Tyr Ala Phe Ser Ile Asp Asn Phe Arg Arg Lys Pro His Glu
                   85                  90                  95
   Val Gln Tyr Val Met Asn Leu Met Leu Glu Lys Ile Glu Gly Met Ile
               100                 105                 110
   Met Glu Glu Ser Ile Ile Asn Ala Tyr Asp Ile Cys Val Arg Phe Val
           115                 120                 125
   Gly Asn Leu Lys Leu Leu Ser Glu Pro Val Lys Thr Ala Ala Asp Lys
       130                 135                 140
   Ile Met Arg Ala Thr Ala Asn Asn Ser Lys Cys Val Leu Leu Leu Ala
   145                 150                 155                 160
   Val Cys Tyr Thr Ser Thr Asp Glu Ile Val His Ala Val Glu Glu Ser
                   165                 170                 175
   Ser Glu Leu Asn Ser Asn Glu Val Cys Asn Asn Gln Glu Leu Glu Glu
               180                 185                 190
   Ala Asn Ala Thr Gly Ser Gly Thr Val Ile Gln Thr Glu Asn Met Glu
           195                 200                 205
   Ser Tyr Ser Gly Ile Lys Leu Val Asp Leu Glu Lys Asn Thr Tyr Ile
       210                 215                 220
   Asn Pro Tyr Pro Asp Val Leu Ile Arg Thr Ser Gly Glu Thr Arg Leu
   225                 230                 235                 240
   Ser Asn Tyr Leu Leu Trp Gln Thr Thr Asn Cys Ile Leu Tyr Ser Pro
                   245                 250                 255
   Tyr Ala Leu Trp Pro Glu Ile Gly Leu Arg His Val Val Trp Ser Val
               260                 265                 270
   Ile Asn Phe Gln Arg His Tyr Ser Tyr Leu Glu Lys His Lys Glu Tyr
           275                 280                 285
   Leu Lys
       290
<210> 18
   <211> 615
   <212> DNA
   <213> Hevea brasiliensis
<400> 18
   atggctgaag aggtggagga agagaggcta aagtatttgg attttgtgcg agcggctgga        60
   gtttatgctg tagattcttt ctcaactctc tacctttatg ccaaggacat atctggtcca       120
   ttaaaacctg gtgtcgatac tattgagaat gtggtgaaga ccgtggttac tcctgtttat       180
   tatattcccc ttgaggctgt caagtttgta gacaaaacgg tggatgtatc ggtcactagc       240
   ctagatggcg ttgttccccc agttatcaag caggtgtctg cccaaactta ctcggtagct       300
   caagatgctc caagaattgt tcttgatgtg gcttcttcag ttttcaacac tggtgtgcag       360
   gaaggcgcaa aagctctgta cgctaatctt gaaccaaaag ctgagcaata tgcggtcatt       420
   acctggcgtg ccctcaataa gctgccacta gttcctcaag tggcaaatgt agttgtgcca       480
   accgctgttt atttctctga aaagtacaac gatgttgttc gtggcactac tgagcaggga       540
   tatagagtgt cctcttattt gcctttgttg cccactgaga aaattactaa ggtgtttgga       600
   gatgaggcat cataa                                                        615
<210> 19
   <211> 204
   <212> PRT
   <213> Hevea brasiliensis
<400> 19
   Met Ala Glu Glu Val Glu Glu Glu Arg Leu Lys Tyr Leu Asp Phe Val
   1               5                   10                  15
   Arg Ala Ala Gly Val Tyr Ala Val Asp Ser Phe Ser Thr Leu Tyr Leu
               20                  25                  30
   Tyr Ala Lys Asp Ile Ser Gly Pro Leu Lys Pro Gly Val Asp Thr Ile
           35                  40                  45
   Glu Asn Val Val Lys Thr Val Val Thr Pro Val Tyr Tyr Ile Pro Leu
       50                  55                  60
   Glu Ala Val Lys Phe Val Asp Lys Thr Val Asp Val Ser Val Thr Ser
   65                  70                  75                  80
   Leu Asp Gly Val Val Pro Pro Val Ile Lys Gln Val Ser Ala Gln Thr
                   85                  90                  95
   Tyr Ser Val Ala Gln Asp Ala Pro Arg Ile Val Leu Asp Val Ala Ser
               100                 105                 110
   Ser Val Phe Asn Thr Gly Val Gln Glu Gly Ala Lys Ala Leu Tyr Ala
           115                 120                 125
   Asn Leu Glu Pro Lys Ala Glu Gln Tyr Ala Val Ile Thr Trp Arg Ala
       130                 135                 140
   Leu Asn Lys Leu Pro Leu Val Pro Gln Val Ala Asn Val Val Val Pro
   145                 150                 155                 160
   Thr Ala Val Tyr Phe Ser Glu Lys Tyr Asn Asp Val Val Arg Gly Thr
                   165                 170                 175
   Thr Glu Gln Gly Tyr Arg Val Ser Ser Tyr Leu Pro Leu Leu Pro Thr
               180                 185                 190
   Glu Lys Ile Thr Lys Val Phe Gly Asp Glu Ala Ser
           195                 200
<210> 20
   <211> 21
   <212> DNA
   <213> Artificial Sequence
<220>
   <223> PRIMER 1
<400> 20
   ctctaccagc tatgattcac c 21
<210> 21
   <211> 20
   <212> DNA
   <213> Artificial Sequence
<220>
   <223> PRIMER 2
<400> 21
   tgatatcttc tgcatgcacc 20
<210> 22
   <211> 26
   <212> DNA
   <213> Artificial Sequence
<220>
   <223> PRIMER 3
<400> 22
   ggtacctacc agctatgatt cacctc 26
<210> 23
   <211> 29
   <212> DNA
   <213> Artificial Sequence
<220>
   <223> PRIMER 4
<400> 23
   agatctagca ccttagtatt tcaattttg 29
<210> 24
   <211> 21
   <212> DNA
   <213> Artificial Sequence
<220>
   <223> PRIMER 5
<400> 24
   gaatccatgg cggatctgaa g 21
<210> 25
   <211> 20
   <212> DNA
   <213> Artificial Sequence
<220>
   <223> PRIMER 6
<400> 25
   gtccatgtat ctggataccc 20
<210> 26
   <211> 24
   <212> DNA
   <213> Artificial Sequence
<220>
   <223> PRIMER 7
<400> 26
   caagatgagt tcagtgaatt tggg 24
<210> 27
   <211> 22
   <212> DNA
   <213> Artificial Sequence
<220>
   <223> PRIMER 8
<400> 27
   tgcattagtt ttgcctgtga gc 22
<210> 28
   <211> 21
   <212> DNA
   <213> Artificial Sequence
<220>
   <223> PRIMER 9
<400> 28
   atttttacat ggacaccacc g 21
<210> 29
   <211> 21
   <212> DNA
   <213> Artificial Sequence
<220>
   <223> PRIMER 10
<400> 29
   accagattcc cactaagatg c 21
<210> 30
   <211> 21
   <212> DNA
   <213> Artificial Sequence
<220>
   <223> PRIMER 11
<400> 30
   tccatatatg gacgaggttc g 21
<210> 31
   <211> 20
   <212> DNA
   <213> Artificial Sequence
<220>
   <223> PRIMER 12
<400> 31
   gcagctgtgt tacccttcag 20
<210> 32
   <211> 23
   <212> DNA
   <213> Artificial Sequence
<220>
   <223> PRIMER 13
<400> 32
   cagtcgctcc aaaatggatg tgc 23
<210> 33
   <211> 24
   <212> DNA
   <213> Artificial Sequence
<220>
   <223> PRIMER 14
<400> 33
   gattttctta ggaagaaggc ttgg 24
<210> 34
   <211> 23
   <212> DNA
   <213> Artificial Sequence
<220>
   <223> PRIMER 15
<400> 34
   ctagctggtc tataatggat gcc 23
<210> 35
   <211> 25
   <212> DNA
   <213> Artificial Sequence
<220>
   <223> PRIMER 16
<400> 35
   gaatcaattt acctcaatag aaggc 25
<210> 36
   <211> 21
   <212> DNA
   <213> Artificial Sequence
<220>
   <223> PRIMER 17
<400> 36
   ttccaccatg ggtgaggctc c 21
<210> 37
   <211> 22
   <212> DNA
   <213> Artificial Sequence
<220>
   <223> PRIMER 18
<400> 37
   tctcaactca acttgtgaat cg 22
<210> 38
   <211> 24
   <212> DNA
   <213> Artificial Sequence
<220>
   <223> PRIMER 19
<400> 38
   atggaattat acaacggtga gagg 24
<210> 39
   <211> 25
   <212> DNA
   <213> Artificial Sequence
<220>
   <223> PRIMER 20
<400> 39
   ttttaagtat tccttatgtt tctcc 25
<210> 40
   <211> 20
   <212> DNA
   <213> Artificial Sequence
<220>
   <223> PRIMER 21
<400> 40
   tatggctgaa gaggtggagg 20
<210> 41
   <211> 22
   <212> DNA
   <213> Artificial Sequence
<220>
   <223> PRIMER 22
<400> 41
   tgatgcctca tctccaaaca cc 22
<210> 42
   <211> 30
   <212> DNA
   <213> Artificial Sequence
<220>
   <223> PRIMER 23
<400> 42
   ctcgagaaca atggcggatc tgaagtcaac 30
<210> 43
   <211> 32
   <212> DNA
   <213> Artificial Sequence
<220>
   <223> PRIMER 24
<400> 43
   ggtacctgtt tctgtctctt gtaaattttg gc 32
<210> 44
   <211> 30
   <212> DNA
   <213> Artificial Sequence
<220>
   <223> PRIMER 25
<400> 44
   ctcgagaaca atgagttcag tgaatttggg 30
<210> 45
   <211> 35
   <212> DNA
   <213> Artificial Sequence
<220>
   <223> PRIMER 26
<400> 45
   ggatccttgt tttgcctgtg agcgatgtaa ttagc 35
<210> 46
   <211> 32
   <212> DNA
   <213> Artificial Sequence
<220>
   <223> PRIMER 27
<400> 46
   ctcgagacaa atggacacca ccggccggct cc 32
<210> 47
   <211> 34
   <212> DNA
   <213> Artificial Sequence
<220>
   <223> PRIMER 28
<400> 47
   ggtaccacag atgcagcttt agacatatct ttgc 34
<210> 48
   <211> 33
   <212> DNA
   <213> Artificial Sequence
<220>
   <223> PRIMER 29
<400> 48
   ctcgagacaa atggacgagg ttcgccggcg acc 33
<210> 49
   <211> 35
   <212> DNA
   <213> Artificial Sequence
<220>
   <223> PRIMER 30
<400> 49
   ggtaccacga aagttatttt ggatacatct tttgc 35
<210> 50
   <211> 30
   <212> DNA
   <213> Artificial Sequence
<220>
   <223> PRIMER 31
<400> 50
   aagcttacaa atggatgtgc gccggcgacc 30
<210> 51
   <211> 29
   <212> DNA
   <213> Artificial Sequence
<220>
   <223> PRIMER 32
<400> 51
   ggtaccacgg aagaaggctt ggaaacagc 29
<210> 52
   <211> 30
   <212> DNA
   <213> Artificial Sequence
<220>
   <223> PRIMER 33
<400> 52
   aagcttacaa atggatgccc gccggcgacc 30
<210> 53
   <211> 33
   <212> DNA
   <213> Artificial Sequence
<220>
   <223> PRIMER 34
<400> 53
   ggtaccacat ttacctcaat agaaggcatt gtc 33
<210> 54
   <211> 32
   <212> DNA
   <213> Artificial Sequence
<220>
   <223> PRIMER 35
<400> 54
   ctcgagaaca atgggtgagg ctccagatgt cg 32
<210> 55
   <211> 35
   <212> DNA
   <213> Artificial Sequence
<220>
   <223> PRIMER 36
<400> 55
   ggtacctgac tcaacttgtg aatcgttttc atgtc 35
<210> 56
   <211> 28
   <212> DNA
   <213> Artificial Sequence
<220>
   <223> PRIMER 37
<400> 56
   ctcgagccaa caatggaatt atacaacg 28
<210> 57
   <211> 33
   <212> DNA
   <213> Artificial Sequence
<220>
   <223> PRIMER 38
<400> 57
   ggatcctctt ttaagtattc cttatgtttc tcc 33
<210> 58
   <211> 29
   <212> DNA
   <213> Artificial Sequence
<220>
   <223> PRIMER 39
<400> 58
   ctcgagaaca atggctgaag aggtggagg 29
<210> 59
   <211> 30
   <212> DNA
   <213> Artificial Sequence
<220>
   <223> PRIMER 40
<400> 59
   ggatcctgtg atgcctcatc tccaaacacc 30
<210> 60
   <211> 417
   <212> DNA
   <213> Hevea brasiliensis
<400> 60
   atggctgaag acgaagacaa ccaacaaggg caggggaggg ggttaaaata tttgggtttt 60
   gtgcaagacg cggcaactta tgctgtgact accttctcaa acgtctatct ttttgccaaa 120
   gacaaatctg gtccactgca gcctggtgtc gatatcattg agggtccggt gaagaacgtg 180
   gctgtacctc tctataatag gttcagttat attcccaatg gagctctcaa gtttgtagac 240
   agcacggttg ttgcatctgt cactattata gatcgctctc ttcccccaat tgtcaaggac 300
   gcatctatcc aagttgtttc agcaattcga gctgccccag aagctgctcg ttctctggct 360
   tcttctttgc ctgggcagac caagatactt gctaaggtgt tttatggaga gaattga 417
<210> 61
   <211> 138
   <212> PRT
   <213> Hevea brasiliensis
<400> 61
   Met Ala Glu Asp Glu Asp Asn Gln Gln Gly Gln Gly Glu Gly Leu Lys
   1               5                   10                  15
   Tyr Leu Gly Phe Val Gln Asp Ala Ala Thr Tyr Ala Val Thr Thr Phe
               20                  25                  30
   Ser Asn Val Tyr Leu Phe Ala Lys Asp Lys Ser Gly Pro Leu Gln Pro
           35                  40                  45
   Gly Val Asp Ile Ile Glu Gly Pro Val Lys Asn Val Ala Val Pro Leu
       50                  55                  60
   Tyr Asn Arg Phe Ser Tyr Ile Pro Asn Gly Ala Leu Lys Phe Val Asp
   65                  70                  75                  80
   Ser Thr Val Val Ala Ser Val Thr Ile Ile Asp Arg Ser Leu Pro Pro
                   85                  90                  95
   Ile Val Lys Asp Ala Ser Ile Gln Val Val Ser Ala Ile Arg Ala Ala
               100                 105                 110
   Pro Glu Ala Ala Arg Ser Leu Ala Ser Ser Leu Pro Gly Gln Thr Lys
           115                 120                 125
   Ile Leu Ala Lys Val Phe Tyr Gly Glu Asn
       130                 135
<210> 62
   <211> 22
   <212> DNA
   <213> Artificial Sequence
<220>
   <223> PRIMER 41
<400> 62
   atggctgaag acgaagacaa cc 22
<210> 63
   <211> 24
   <212> DNA
   <213> Artificial Sequence
<220>
   <223> PRIMER 42
<400> 63
   attctctcca taaaacacct tagc 24
<210> 64
   <211> 32
   <212> DNA
   <213> Artificial Sequence
<220>
   <223> PRIMER 43
<400> 64
   ctcgagaaca atggctgaag acgaagacaa cc 32
<210> 65
   <211> 32
   <212> DNA
   <213> Artificial Sequence
<220>
   <223> PRIMER 44
<400> 65
   ggatccaaat tctctccata aaacacctta gc 32
<210> 66
   <211> 855
   <212> DNA
   <213> Hevea brasiliensis
<400> 66
   atggaattat acaacggtga gaggccaagt gtgttcagac ttttagggaa gtatatgaga 60
   aaagggttat atagcatcct aacccagggt cccatcccta ctcatattgc cttcatattg 120
   gatggaaacg ggaggtttgc taagaagcat aaactgccag aaggaggtgg tcataaggct 180
   ggatttttag ctcttctgaa cgtactaact tattgctatg agttaggagt gaaatatgcg 240
   actatctatg cctttagcat cgataatttt cgaaggaaac ctcatgaggt tcagtacgta 300
   atgaatctaa tgctggagaa gattgaaggg atgatcatgg aagaaagtat catcaatgca 360
   tatgatattt gcgtgcgttt tgttggtaat ctgaagcttt tagatgagcc actcaagacc 420
   gcagcagata agataatgag ggctactgcc aaaaattcca aatttgtgct tctccttgct 480
   gtatgctaca cttcaactga tgagatcgtg catgctgttg aagaatcctc taaggataaa 540
   ttgaaatccg atgaaatttg caacgatgga aacggagatt gtgtgattaa aattgaggag 600
   atggagccat attctgaaat aaaacttgta gagcttgaga gaaacactta cataaatcct 660
   tatcctgatg tcttgattcg aacttctggg gagacccgtc tgagcaacta cctactttgg 720
   cagactacta attgcatact gtattctcct catgcactgt ggccagagat tggtcttcga 780
   cacgtggtgt gggcagtaat taactgccaa cgtcattatt cttacttgga gaaacataag 840
   gaatacttaa aataa 855
<210> 67
   <211> 284
   <212> PRT
   <213> Hevea brasiliensis
<400> 67
   Met Glu Leu Tyr Asn Gly Glu Arg Pro Ser Val Phe Arg Leu Leu Gly
   1               5                   10                  15
   Lys Tyr Met Arg Lys Gly Leu Tyr Ser Ile Leu Thr Gln Gly Pro Ile
               20                  25                  30
   Pro Thr His Ile Ala Phe Ile Leu Asp Gly Asn Gly Arg Phe Ala Lys
           35                  40                  45
   Lys His Lys Leu Pro Glu Gly Gly Gly His Lys Ala Gly Phe Leu Ala
       50                  55                  60
   Leu Leu Asn Val Leu Thr Tyr Cys Tyr Glu Leu Gly Val Lys Tyr Ala
   65                  70                  75                  80
   Thr Ile Tyr Ala Phe Ser Ile Asp Asn Phe Arg Arg Lys Pro His Glu
                   85                  90                  95
   Val Gln Tyr Val Met Asn Leu Met Leu Glu Lys Ile Glu Gly Met Ile
               100                 105                 110
   Met Glu Glu Ser Ile Ile Asn Ala Tyr Asp Ile Cys Val Arg Phe Val
           115                 120                 125
   Gly Asn Leu Lys Leu Leu Asp Glu Pro Leu Lys Thr Ala Ala Asp Lys
       130                 135                 140
   Ile Met Arg Ala Thr Ala Lys Asn Ser Lys Phe Val Leu Leu Leu Ala
   145                 150                 155                 160
   Val Cys Tyr Thr Ser Thr Asp Glu Ile Val His Ala Val Glu Glu Ser
                   165                 170                 175
   Ser Lys Asp Lys Leu Lys Ser Asp Glu Ile Cys Asn Asp Gly Asn Gly
               180                 185                 190
   Asp Cys Val Ile Lys Ile Glu Glu Met Glu Pro Tyr Ser Glu Ile Lys
           195                 200                 205
   Leu Val Glu Leu Glu Arg Asn Thr Tyr Ile Asn Pro Tyr Pro Asp Val
       210                 215                 220
   Leu Ile Arg Thr Ser Gly Glu Thr Arg Leu Ser Asn Tyr Leu Leu Trp
   225                 230                 235                 240
   Gln Thr Thr Asn Cys Ile Leu Tyr Ser Pro His Ala Leu Trp Pro Glu
                   245                 250                 255
   Ile Gly Leu Arg His Val Val Trp Ala Val Ile Asn Cys Gln Arg His
               260                 265                 270
   Tyr Ser Tyr Leu Glu Lys His Lys Glu Tyr Leu Lys
           275                 280
<210> 68
   <211> 24
   <212> DNA
   <213> Artificial Sequence
<220>
   <223> PRIMER 45
<400> 68
   atggaattat acaacggtga gagg 24
<210> 69
   <211> 25
   <212> DNA
   <213> Artificial Sequence
<220>
   <223> PRIMER 46
<400> 69
   ttttaagtat tccttatgtt tctcc 25
<210> 70
   <211> 28
   <212> DNA
   <213> Artificial Sequence
<220>
   <223> PRIMER 47
<400> 70
   ctcgagccaa caatggaatt atacaacg 28
<210> 71
   <211> 33
   <212> DNA
   <213> Artificial Sequence
<220>
   <223> PRIMER 48
<400> 71
   ggatcctctt ttaagtattc cttatgtttc tcc 33